# EUROPEAN PATENT SPECIFICATION

(11) **EP 0 402 312 B1**
(45) Date of publication and mention of the grant of the patent: **25.01.1995**
(21) Application number: 90810387.2
(22) Date of filing: 25.05.1990
(51) Int. Cl.: C07F 9/30, A61K 31/66

(54) **P-substituted propane-phosphinic acid compounds**
P-substituierte Propanphosphinsäureverbindungen
Composés P-substitués de l'acide propane-phosphinique

(30) Priority: 03.06.1989 GB 8912814
(43) Date of publication of application: 12.12.1990
(73) Proprietor: CIBA-GEIGY AG, 4002 Basel (CH)
(72) Inventor: Mickel, Stuart J., Dr., CH-4415 Lausen (CH); von Sprecher, Georg, Dr., CH-4104 Oberwil (CH)

(56) References cited:
- EP-A- 0 319 479
- EP-A- 0 319 482
- EP-A- 0 356 128
- DE-C- 2 032 712

## Description

The invention relates to compounds of the formula I 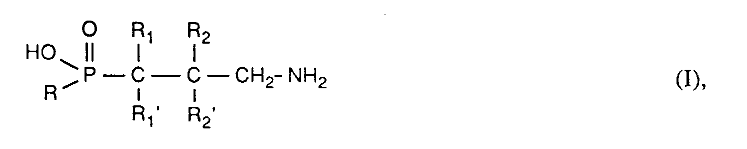
wherein either R₁ is halogen, R₁' is halogen or hydrogen and R₂ and R₂' denote hydrogen or R₁ and R₁' represent hydrogen, R₂ is an aliphatic or aromatic radical and R₂' is hydroxy or R₂ and R₂' together represent oxo, and wherein R denotes an aliphatic, cycloaliphatic, cycloaliphatic-aliphatic or araliphatic radical having 2 or more carbon atoms or, if R₁ and R₁' denote hydrogen, R₂ represents an aromatic radical and R₂' is hydroxy, R represents methyl, and to their salts, to a process for the manufacture of compounds of the formula I, to pharmaceutical compositions containing the same and to their use as a medicament or for the manufacture thereof.

Aliphatic radicals R are, for example, alkyl groups that may be interrupted by one or two mutually spaced atoms selected from oxygen and sulphur and/or substituted by halogen, hydroxy, oxo and/or optionally acylated amino, such as alkyl, alkyl mono-, di- or polysubstituted by halogen and/or hydroxy, alkyl substituted by oxo, alkyl substituted by optionally acylated amino or by hydroxy and optionally acylated amino, alkyl being interrupted by one or two mutually spaced atoms selected from oxygen and sulphur or alkyl being interrupted by one or two mutually spaced atoms selected from oxygen and sulphur and substituted by halogen and/or hydroxy, alkenyl groups that may be mono-, di- or polysubstituted by halogen and/or hydroxy, such as lower alkenyl or lower alkenyl substituted by halogen and/or hydroxy, or alkynyl groups, such as lower alkynyl.

Cycloaliphatic radicals R are, for example, cycloalkyl groups that may be interrupted by one or two mutually spaced atoms selected from oxygen and sulphur and/or substituted by hydroxy, such as cycloalkyl, cycloalkyl being interrupted by one or two mutually spaced atoms selected from oxygen and sulphur or cycloalkyl substituted by hydroxy.

Cycloaliphatic-aliphatic radicals R are, for example, cycloalkyl-lower alkyl groups that may be interrupted by one or two mutually spaced atoms selected from oxygen and sulphur and/or substituted by hydroxy and/or lower alkylthio, such as cycloalkyl-lower alkyl, cycloalkyl-lower alkyl being interrupted by one or two mutually spaced atoms selected from oxygen and sulphur or cycloalkyl-lower alkyl substituted in the cycloalkyl moiety by hydroxy or lower alkylthio and/or in the alkylene moiety by hydroxy.

Araliphatic radicals R are, for example, phenyl-lower alkyl or naphthyl-lower alkyl radicals that may be substituted in the aryl ring by halogen, lower alkyl, lower alkoxy and/or trifluoromethyl and/or in the lower alkylene moiety by hydroxy, such as phenyl-lower alkyl, phenyl-(1-hydroxy)-lower alkyl, naphthyl-lower alkyl or phenyl-lower alkyl substituted in the phenyl moiety by halogen, lower alkyl, lower alkoxy and/or trifluoromethyl.

Aromatic radicals R₂ may be carbocyclic or heterocyclic aromatic radical, such as phenyl, naphthyl, or phenyl substituted by halogen, lower alkyl, lower alkoxy and/or trifluoromethyl or pyridyl.

In compounds of formula I the group R is bonded to the P-atom via a carbon atom.

Optionally acylated amino is, for example, amino, lower alkanoylamino or phthalimido.

Alkyl, alkenyl and alkynyl R may contain up to and including 14, preferably 12 carbon atoms and are represented by lower alkyl, lower alkenyl and lower alkynyl. Alkyl R may also be a C₈-C₁₄-, e.g. a C₈-C₁₂-alkyl, such as an octyl, nonyl, decyl, undecyl or dodecyl group, e.g. a decyl or dodecyl group.

Alkyl or alkenyl mono-, di- or polysubstituted by halogen and/or hydroxy is represented by mono- or dihydroxy-lower alkyl, hydroxy-lower alkenyl, mono-, di- or polyhalogeno-lower alkyl, mono-, di- or polyhalogeno-lower alkenyl, mono-, di- or polyhalogeno-lower hydroxyalkyl and mono-, di- or polyhalogeno-lower hydroxyalkenyl.

Alkyl substituted by oxo is, for example oxo-lower alkyl.

Alkyl substituted by optionally acylated amino is, for example, amino-lower alkyl, N-lower alkanoylamino-lower alkyl or phthalimido-lower alkyl.

Alkyl substituted by optionally acylated amino and by hydroxy is, for example, amino-lower hydroxyalkyl, N-lower alkanoylamino-lower hydroxyalkyl or phthalimido-lower hydroxyalkyl.

Alkyl being interrupted by one or two atoms selected from oxygen and sulphur is represented by lower alkoxy-lower alkyl, lower alkylthio-lower alkyl, lower alkanesulphinyl-lower alkyl, lower alkanesulphonyl-lower alkyl, lower alkoxy-lower alkoxy-lower alkyl, di-lower alkoxy-lower alkyl, di-lower alkylthio-lower alkyl, and lower alkoxy-lower alkylthio-lower alkyl.

Alkyl being interrupted by one or two atoms selected from oxygen and sulphur and substituted by hydroxy and/or halogen is represented by lower alkoxy-(hydroxy)lower alkyl and lower alkoxy-(halogeno)lower alkyl.

Cycloalkyl is represented by C₃-C₈-cycloalkyl.

Cycloalkyl substituted by hydroxy is represented by 1-hydroxy-C₃-C₈-cycloalkyl.

Cycloalkyl and cycloalkyl in cycloalkyl-lower alkyl, in either case, being interrupted by one or two atoms selected from oxygen and sulphur is represented by oxa-C₃-C₈-cycloalkyl, thia-C₃-C₈-cycloalkyl, dioxa-C₃-C₈-cycloalkyl, dithia-C₃-C₈-cycloalkyl and oxathia-C₃-C₈-cycloalkyl.

Cycloalkyl-lower alkyl substituted in the cycloalkyl moiety by hydroxy and/or lower alkylthio and/or in the alkylene moiety by hydroxy is represented by lower alkylthiocycloalkyl-lower alkyl, cycloalkyl-(hydroxy)lower alkyl and lower alkylthiocycloalkyl-(hydroxy)lower alkyl.

The general definitions used herein have the following meaning within the scope of the present invention.

The term "lower" referred to above and hereinafter in connection with organic radicals or compounds respectively, if not defined explicitly otherwise, defines such with up to and including 7, preferably up to and including 4, carbon atoms.

Lower alkyl R is represented by C₂-C₇-alkyl, e.g. ethyl, propyl, isopropyl, butyl, isobutyl, sec.-butyl, tert.-butyl, (2-methyl)butyl, hexyl or heptyl. Lower alkyl other than R denotes, for example, C₁-C₄-alkyl, e.g. methyl, ethyl, propyl, isopropyl, butyl or tert.-butyl.

Lower alkenyl denotes, for example, C₂-C₇-alkenyl, preferably C₃-C₅-alkenyl, carrying the double bond in a higher than the α,β-position, and is e.g. 2-propenyl (allyl), but-3-en-1-yl,(2-methyl)prop-2-en-1-yl(isobutenyl) or (5-methyl)but-2-en-1-yl, but may also carry the double bond in α,β-position and may be, for example, vinyl, prop-1-enyl or but-1-enyl, or may be a C₆- or C₇-alkenyl, such as a hexenyl or heptenyl, group.

Lower alkynyl denotes, for example, C₂-C₇-alkynyl, preferably C₃-C₅-alkynyl, carrying the triple bond in a higher than the α,β-position and is e.g. 2-propynyl (propargyl), but-3-yn-1-yl, but-2-yn-1-yl or pent-3-yn-1-yl.

C₃-C₈-Cycloalkyl preferably has 3 to 6 ring carbon atoms and thus is C₃-C₆-cycloalkyl, e.g. cyclopropyl, cyclobutyl, cyclopentyl or cyclohexyl.

C₃-C₈-Cycloalkyl-lower alkyl preferably has 3 to 6 ring and 1 to 4 chain carbon atoms and is, for example, C₃-C₆-cycloalkyl-C₁-C₄-alkyl, such as cyclopropylmethyl, cyclobutylmethyl or cyclohexylmethyl.

Mono- or dihydroxy-lower alkyl preferably carries one of the hydroxy groups in α-position and is for example, α-hydroxy-C₂-C₇-alkyl, such as α-hydroxy-C₂-C₄-alkyl, e.g. 1-hydroxyethyl, 2-(2-hydroxy)propyl, 1-hydroxybutyl, 2-(2-hydroxy)butyl or 1-(1-hydroxy-2-methyl)propyl, or α,β-dihydroxy-C₂-C₇-alkyl, such as 1,2-dihydroxy-prop-2-yl, but may also carry a single hydroxy group in a higher than the α-position and denote, for example, β-, γ- or δ-hydroxy-C₂-C₇-alkyl, e.g. 3-hydroxypropyl or 2-,3-or 4-hydroxybutyl.

Hydroxy-lower alkenyl preferably carries the hydroxy group in α-position and the double bond in a higher than the α,β-position and is, for example, corresponding α-hydroxy-C₃-C₅-alkenyl, e.g. 1-hydroxybut-2-enyl.

Mono-, di- or polyhalogeno-lower alkyl is for example, mono-, di- or trifluoro-C₂-C₅-alkyl, e.g. 3,3,3-trifluoropropyl, 4,4,4-trifluorobutyl, 1- or 2-fluorobutyl or 1,1-difluorobutyl.

Mono-, di- or polyhalogeno-lower alkenyl is, for example, mono-, di- or trifluoro-C₃-C₅-alkenyl, e.g. 2-fluorobut-2-enyl.

Mono-, di- or polyhalogeno-lower hydroxyalkyl and mono-, di- or polyhalogeno-lower hydroxyalkenyl preferably carries the hydroxy group in α-position and the halogen atom(s) in a higher than the α-position and is, for example, corresponding mono-, di- or trifluoro-α-hydroxy-C₂-C₇-alkyl or mono- di- or trifluoro-C₃-C₇-alkenyl, e.g. 2-fluoro-1-hydroxybutyl, 2-fluoro-1-hydroxy-but-2-en-1-yl or 4,4,4-trifluoro-1-hydroxybutyl.

Lower alkoxy-lower alkyl preferably has up to 10 carbon atoms and is, for example, C₁-C₄-alkoxy-C₁-C₄-alkyl, such as C₁-C₃-alkoxy-C₁-C₃-alkyl, e.g. methoxymethyl, ethoxymethyl, 2-methoxyethyl, 2-ethoxyethyl, 3-methoxypropyl or 1- or 2-methoxybutyl.

Lower alkoxy is, for example, C₁-C₄-alkoxy, e.g. methoxy, ethoxy, isopropyloxy, propyloxy, butyloxy, sec.-butyloxy or tert.-butyloxy.

Lower alkoxy-lower alkoxy-lower alkyl is, for example, C₁-C₄-alkoxy-C₂-C₄-alkoxy-C₁-C₄-alkyl, e.g. 2-methoxyethoxymethyl.

Lower alkylthio-lower alkyl preferably has up to 10 carbon atoms and is, for example, C₁-C₄-alkylthio-C₁-C₄-alkyl, such as C₁-C₃-alkylthio-C₁-C₃-alkyl, e.g. methylthiomethyl, ethylthiomethyl, 2-methylthioethyl, 2-ethylthioethyl or 3-methylthiopropyl.

Lower alkansulphinyl- and lower alkanesulphonyl-lower alkyl preferably has up to 10 carbon atoms and is, for example, C₁-C₄-alkanesulfinyl- or C₁-C₄-alkanesulfonyl-C₁-C₄-alkyl, e.g. ethanesulphinylmethyl or ethanesulphonylmethyl.

Di-lower alkoxy-lower alkyl preferably has up to 15 carbon atoms totally and is, for example, di-C₁-C₄-alkoxy-C₁-C₃-alkyl, such as di-C₁-C₃-alkoxy-C₁-C₃-alkyl, e.g. dimethoxymethyl, diethoxymethyl, dipropyloxymethyl, 1,1- or 2,2-diethoxyethyl, diisopropyloxymethyl, di-n-butyloxymethyl or 3,3-dimethoxypropyl.

Di-lower alkylthio-lower alkyl preferably has up to 15 carbon atoms totally and is, for example, di-C₁-C₄-alkylthio-C₁-C₄-alkyl, such as di-C₁-C₃-alkylthio-C₁-C₃-alkyl, e.g. dimethylthiomethyl, diethylthiomethyl or 1,1- or 2,2-dimethylthioethyl.

Lower alkoxy-(hydroxy)lower alkyl is, for example C₁-C₄-alkoxy-C₁-C₇-(hydroxy)alkyl e.g. 2-(2-hydroxy-3-methoxy)propyl.

Lower alkoxy-(halogeno)lower alkyl is, for example C₁-C₄-alkoxy-C₁-C₇-(halogeno)alkyl e.g. 1-(2-fluoro-1-methoxy)butyl.

Oxo-lower alkyl carries the oxo group preferably in a position higher than the α-position and is, for example, oxo-C₂-C₇alkyl, especially oxo-C₃-C₆alkyl, such as 2-oxopropyl, 2-or 3-oxobutyl or 3-oxopentyl.

Amino-lower alkyl is, for example, amino-C₂-C₇alkyl, especially amino-C₃-C₆alkyl, such as 3-aminopropyl or 3- or 4-aminobutyl. Similarly, N-lower alkanoylamino-lower alkyl and phthalimido-lower alkyl is, for example, N-C₂-C₇alkanoylamino- or phthalimido-C₂-C₇alkyl, especially -C₃-C₆alkyl, such as 3-acetamidopropyl, 3- or 4-acetamido butyl, 3-phthalimidopropyl or 3- or 4-phthalimidobutyl.

Amino-lower hydroxyalkyl is, for example, amino-C₃-C₇(hydroxy)alkyl, such as 3-amino-2-hydroxy-propyl or 4-amino-2-hydroxybutyl. Similarly, N-lower alkanoylamino-lower hydroxyalkyl and phthalimido-lower hydroxyalkyl is, for example, N-C₂-C₇alkanoylamino- or phthalimido-C₂-C₇alkyl, especially -C₃-C₇alkyl, such as 3-acetamido- or 3-phthalimido-2-hydroxy-propyl or 4-phthalimido-2-hydroxybutyl.

Hydroxy-C₃-C₈-cycloalkyl is, for example, 1-hydroxy-C₃-C₆-cycloalkyl, e.g. 1-hydroxycyclobutyl.

Oxa- or thia-C₃-C₈-cycloalkyl preferably has 2 to 6 ring carbon atoms is, for example, 2-oxacyclopropyl (oxiranyl), 2- or 3-oxacyclobutyl (oxetanyl), 2- or 3-thiacyclobutyl (thietanyl), 2- or 3-oxacylcopentyl (tetrahydrofuranyl), 2- or 3-thiacyclopentyl (thiolanyl) or 2-oxacyclohexyl (tetrahydropyranyl).

Dioxa-C₃-C₈-cycloalkyl preferably has 3 to 5 ring carbon atoms and carries those two oxygen atoms in 1,3-position to each other, and represents e.g. 1,3-dioxolan-2-yl or 1,3-dioxan-2-yl.

Dithia-C₃-C₈-cycloalkyl preferably has 3 to 5 ring carbon atoms and carries those two sulphur atoms in 1,3-position to each other and represents e.g. 1,3-dithiolan-2-yl or 1,3-dithioxan-2-yl. Oxathio-C₃-C₈-cycloalkyl is, for example 1,3-oxathiolan-2-yl or 1,3-oxathioxan-2-yl.

C₃-C₈-Cycloalkyl-(hydroxy)lower alkyl preferably has 3 to 6 ring and 1 to 4 chain carbon atoms and is, for example, cyclo-C₃-C₆-alkyl-C₁-C₄-alkyl, e.g. 1-cyclopropyl-1-hydroxymethyl or 1-hydroxy-1-cyclobutylmethyl. Lower alkylthiocycloalkyl-(hydroxy) lower alkyl is, for example, 1-hydroxy-1-(2-methylthiocyclopropyl).

Halogen, R₁, R₁' and/or as a substituent of aromatic radicals R₂, is preferably fluoro, but may also be chloro, bromo or iodo.

A phenyl or naphthyl group may have one or more than one, preferably one or two of the same or different substituents as defined hereinbefore. Phenyl- or naphthyl-lower alkyl is e.g. benzyl, naphth-2-ylmethyl, 1- or 2-phenylethyl or 2- or 3-phenylpropyl, each optionally substituted as described hereinbefore.

Salts of the compounds of the formula I are particularly pharmaceutically acceptable salts thereof, such as the corresponding addition salts with acids, as well as the salts with bases. Suitable acids for the formation of acid addition salts are, for example, mineral acids, such as hydrochloric, hydrobromic, sulphuric or phosphoric acid, or organic acids, such as organic sulphonic acids, for example, benzenesulphonic, 4-toluenesulphonic or methanesulphonic acid, and organic carboxylic acids, such as acetic, lactic, palmitic, stearic, malic, maleic, fumaric, tartaric, ascorbic or citric acid. Salts with bases are, for example, alkali metal or alkaline earth metal salts, such as sodium, potassium, calcium or magnesium salts, or ammonium salts, such as those with ammonia or suitable organic amines, e.g. diethylamine, di-(2-hydroxyethyl)-amine or tri-(2-hydroxyethyl)-amine. The compounds of the formula I may also form inner salts.

Depending on the presence of asymmetric carbon atoms, the compounds of this invention may be in the form of mixtures of isomers, particularly racemates, or in the form of pure isomers, particularly optical antipodes.

It has been found that the compounds of the formula I and their pharmaceutically acceptable salts possess valuable pharmacological properties. They show an effective binding at the GABA_{B}-receptor and are found to act as antagonists on said receptor. Mechanistically, antagonism at GABA_{B} receptors may increase the release of fast excitatory amino acid transmitters, i.e. glutamate and aspartate, thus improving information processing in the brain. In line with this is the finding that the late inhibitory postsynaptic potential in hippocampus, attributed to a GABA_{B} mechanism, is shortened by the antagonists thus allowing a faster sequence of nerve impulse transfer.

On the other hand, chronic treatment with antidepressants and repetitive electroshock have been found to increase the number of GABA_{B} receptors in rat cerebral cortex. In line with receptor theories, chronic treatment with GABA_{B} antagonists should result in the same effect. For these and other reasons, GABA_{B} antagonists may therefore act as antidepressants.

The GABA_{B} antagonists of the present invention interact at the GABA_{B} receptor with IC₅₀ values starting from about 10⁻⁷ M (moles/litre) in rat brain cortex membranes. In contrast to GABA_{B} agonists, such as baclofen, they do not potentiate the stimulation of adenylate cyclase in rat cerebral cortex slices by noradrenaline, but antagonize the effects of baclofen. The antagonism against baclofen has also been shown in in vitro electrophysiological models, such as the penicilline-induced "epileptic" hippocampal slice preparation, where baclofen, at a concentration of 6 µM inhibits "epileptic"-like discharges of pyramidal cells. The compounds of the invention antagonise the effects of baclofen at concentrations from approximately 10 to approximately 100 µM. In vivo, antagonism has been shown by ionophoresis of baclofen on rat cerebral cortex, and systemic application of antagonists in doses of 10 - 100 mg/kg. The muscle relaxant effects of baclofen measured in the rotarod model are also antagonized at doses of about 30 mg/kg i.p.

The antagonists do not only show antagonistic effects against baclofen, but have, as theoretically expected (see above), also effects on their own as antagonists of endogenous GABA. Thus the antagonists are active in behavioural models which are established in the art to be indicative of antidepressant, anxiolytic and/or nootropic properties. Compounds of the formula I have been found to be active, after peroral application, in the swim test according to Porsolt, in the Geller test, the one trial, step-down passive avoidance test (one-trial modification) in pretrial and posttrial situations, in the two compartment test and in the complex labyrinth. In addition, in studies on Rhesus monkeys, an increase in playfulness, exploration, social grooming and a reduction of signs of anxiety were observed. Accordingly, the compounds of formula I may be used as nootropic, antidepressant and anxiolytic agents. Of course, they may also be used as baclofen-antidotes.

The invention relates, for example, to compounds of the formula I, wherein either R₁ is halogen, R₁' is halogen or hydrogen and R₂ and R₂' denote hydrogen or R₁ and R₁' represent hydrogen, R₂ is an aromatic radical and R₂' is hydroxy or R₂ and R₂' together represent oxo, or wherein R denotes an aliphatic, cycloaliphatic, cycloaliphatic-aliphatic or araliphatic radical having 2 or more carbon atoms, and to their salts, to a process for the manufacture of compounds of the formula I, to pharmaceutical containing the same and to their use as a medicament or for the manufacture thereof.

The invention relates in particular to compounds of the formula I, wherein R₁ is halogen, R₁' is halogen or hydrogen and R₂ and R₂' are hydrogen or R₁ and R₁' are hydrogen, R₂ denotes lower alkyl, phenyl, phenyl mono- or disubstituted by halogen, lower alkyl, lower alkoxy and/or trifluoromethyl or pyridyl and R₂' is hydroxy or R₂ and R₂' together represent oxo, and wherein R denotes lower alkyl having 2 or more carbon atoms, lower alkenyl, lower alkynyl, a cycloalkyl, hydroxycycloalkyl, cycloalkyl-lower alkyl, cycloalkyl-(hydroxy)lower alkyl or lower alkylthiocycloalkyl(hydroxy)-lower alkyl group having 3 to 6 ring carbon atoms, oxo-lower alkyl, amino-lower alkyl, lower alkanoylamino-lower alkyl, phthalimido-lower alkyl, mono- or dihydroxy-lower alkyl, hydroxy-lower alkenyl, amino-(hydroxy)lower alkyl, lower alkanoylamino-(hydroxy)lower alkyl, phthalimido-(hydroxy)lower alkyl, mono-, di- or polyhalogeno-lower alkyl, mono-, di- or polyhalogeno-lower alkenyl, mono-, di- or polyhalogeno-(hydroxy)lower alkyl, mono-, di- or polyhalogeno-(hydroxy)lower alkenyl, amino lower alkoxy-lower alkyl, lower alkylthio-lower alkyl, lower alkanesulphinyl-lower alkyl, lower alkanesulphonyl-lower alkyl, di-lower alkoxy-lower alkyl, di-lower alkylthio-lower alkyl, lower alkoxy-(hydroxy)lower alkyl, lower alkoxy-(halogeno)lower alkyl, phenyl-lower alkyl, phenyl-lower alkyl mono- or disubstituted, in the phenyl moiety, by halogen, lower alkyl, lower alkoxy and/or trifluoromethyl, naphthyl-lower alkyl, oxa- or thiacycloalkyl having 2 to 6 ring carbon atoms, or dioxa-, oxathia- or dithiacycloalkyl having 3 to 5 ring carbon atoms or, if R₁ and R₁' denote hydrogen, R₂ represents phenyl, phenyl mono- or disubstituted by halogen, lower alkyl, lower alkoxy and/or trifluoromethyl or pyridyl and R₂' is hydroxy, R represents methyl, and to their salts, especially pharmaceutically acceptable salts.

The invention relates in particular,for example, to compounds of the formula I, wherein R₁ is halogen, R₁' is halogen or hydrogen and R₂ and R₂' are hydrogen or R₁ and R₁' are hydrogen, R₂ denotes phenyl or phenyl mono- or disubstituted by halogen, lower alkyl, lower alkoxy and/or trifluoromethyl and R₂' is hydroxy or R₂ and R₂' together represent oxo, and wherein R denotes lower alkyl having 2 or more carbon atoms, lower alkenyl, lower alkynyl, a cycloalkyl, hydroxycycloalkyl, cycloalkyl-lower alkyl, cycloalkyl-(hydroxy)lower alkyl or lower alkylthiocycloalkyl-(hydroxy)-lower alkyl group having 3 to 6 ring carbon atoms, oxo-lower alkyl, amino-lower alkyl, lower alkanoylamino-lower alkyl, phthalimido-lower alkyl, mono- or dihydroxy-lower alkyl, hydroxy-lower alkenyl, amino-(hydroxy)lower alkyl, lower alkanoylamino-(hydroxy)lower alkyl, phthalimido-(hydroxy)lower alkyl, mono-, di- or polyhalogeno-lower alkyl, mono-, di- or polyhalogeno-lower alkenyl, mono-, di- or polyhalogeno-(hydroxy)lower alkyl, mono-, di- or polyhalogeno-(hydroxy)lower alkenyl, amino lower alkoxy-lower alkyl, lower alkylthio-lower alkyl, lower alkanesulphinyl-lower alkyl, lower alkanesulphonyl-lower alkyl, di-lower alkoxy-lower alkyl, di-lower alkylthio-lower alkyl, lower alkoxy-(hydroxy)lower alkyl, lower alkoxy-(halogeno)lower alkyl, phenyl-lower alkyl, phenyl-lower alkyl mono- or disubstituted, in the phenyl moiety, by halogen, lower alkyl, lower alkoxy and/or trifluoromethyl, naphthyl-lower alkyl, oxa- or thiacycloalkyl having 2 to 6 ring carbon atoms, or dioxa-, oxathia- or dithiacycloalkyl having 3 to 5 ring carbon atoms, and to their salts, especially pharmaceutically acceptable salts.

The invention relates especially to compounds of the formula I, wherein either R₁ and R₁' are fluoro and R₂ and R₂' represent hydrogen or R₁ and R₁' are hydrogen, R₂ is phenyl, phenyl substituted by halogen such as fluoro, C₁-C₄alkyl, such as methyl, C₁-C₄alkoxy such as methoxy, and/or trifluoromethyl and R₂' is hydroxy or R₁ and R₁' are hydrogen and R₂ and R₂' together represent oxo, and wherein R is C₂-C₁₂-alkyl, such as ethyl, butyl, isobutyl, pentyl or isopentyl, C₂-C₇-alkenyl, such as but-3-enyl, C₂-C₇-alkynyl, such as pent-3-ynyl, mono- or dihydroxy-C₂-C₇-alkyl, such as 2-(2-hydroxy)propyl, 2-(1,2-di-hydroxy)propyl, 2-(2-hydroxy)butyl or 1-hydroxybutyl, oxo-C₃-C₇alkyl, such as 3-oxobutyl, amino-C₃-C₆alkyl, such as 3-aminopropyl or 4-aminobutyl, phthalimido-C₃-C₆alkyl, such as 3-phthalimidopropyl or 4-phthalimidobutyl, or phthalimido-C₃-C₇(hydroxy)alkyl, such as 3-phthaloimido-2-hydroxypropyl or 4-phthalimido-2-hydroxy-butyl, and their salts, especially pharmaceutically acceptable salts.

Especially preferred are compounds of the formula I, wherein R₁ and R₁' are fluoro and R₂ and R₂' are hydrogen, or R₁ and R₁' are hydrogen and R₂ and R₂' together represent oxo, and wherein R denotes C₂-C₇-alkyl, such as ethyl, butyl or isobutyl, α-saturated C₃-C₇-alkenyl, such as but-3-enyl, α-saturated C₃-C₇-alkynyl, such as pent-3-ynyl, α-, β-, γ- or δ-hydroxy-C₂-C₇-alkyl, such as 2-(2-hydroxy)propyl or 1-hydroxybutyl, α,α-difluoro-C₂-C₄-alkyl, such as 1,1-difluorobutyl, mono-, di- or trifluoro-α-hydroxy-C₃-C₇-alkyl, such as 1-hydroxy-4,4,4-trifluorobutyl, α-saturated mono-, di- or trihalogeno-α-hydroxy-C₃-C₇-alkenyl, such as 1-hydroxy-2-fluoro-but-2-enyl, C₁-C₄-alkoxy-C₁-C₄-alkyl, such as 2-ethoxyethyl or 3-methoxypropyl, di-C₁-C₄-alkoxy-C₁-C₄-alkyl, C₃-C₆-cycloalkyl-C₁-C₄-alkyl, such as cyclopropylmethyl, α-hydroxy-C₃-C₆-cycloalkyl, such as 1-hydroxycylobutyl, or C₃-C₆-cycloalkyl-α-hydroxy-C₁-C₄-alkyl, such as 1-cyclopropyl-1-hydroxymethyl, and to their salts, especially pharmaceutically acceptable salts.

Also preferred are compounds of the formula I, wherein R₁ and R₁' are hydrogen, R₂ is phenyl, phenyl substituted by halogen such as fluoro, C₁-C₄alkyl, such as methyl, C₁-C₄alkoxy such as methoxy, and/or trifluoromethyl and R₂' is hydroxy, and wherein R represents C₁-C₇alkyl, such as methyl or n-, sec.- or iso-butyl, and their salts, especially pharmaceutically acceptable salts.

In the preferred subgroups of compounds of the formula I specified hereinbefore, R most preferably denotes C₃-C₄alkyl, such as propyl, isopropyl or n-, sec.- or iso-butyl, or, if applicable, hydrogen or methyl.

Very especially preferred are the compounds of the formula I, wherein R is C₂-C₇-alkyl, such as n-, sec.- or iso-butyl, and wherein R₁ and R₁' are fluoro and R₂ and R₂' are hydrogen, or R₁ and R₁' are hydrogen and R₂ and R₂' together represent oxo, and pharmaceutically acceptable salts thereof.

Most preferred are the compounds of the formula I, wherein R is C₃-C₇-alkyl and wherein R₁ and R₁' are fluoro and R₂ and R₂' are hydrogen, or R₁ and R₁' are hydrogen and R₂ and R₂' together represent oxo, and pharmaceutically acceptable salts thereof.

The invention specifically relates to the compounds of the formula I described in the Examples herein, and to their salts, especially pharmaceutically acceptable salts.

The process for the manufacture of compounds of the formula I, is characterized in that
a) in a compound of formula II 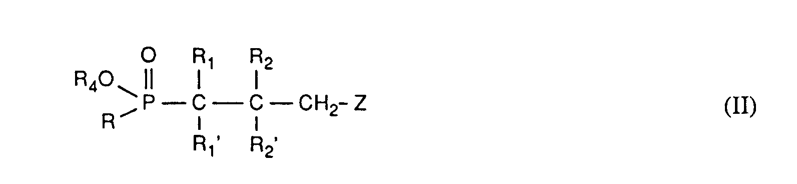 in which R, R₁, R₁', R₂ and R₂' have their previous significances, Z represents a protected or latent amino group Z₀ and R₄ denotes hydrogen or a hydroxy-protective group R₅, and wherein amino as a constituent of R and/or hydroxy R₂' or oxo R₂ + R₂' may be present in a temporarily protected form, any group R₅ or R₆ is replaced by hydrogen and/or any group Z₀ is converted into -NH₂; or
b) in a compound of the formula III 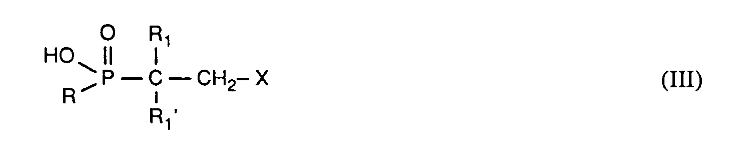 in which R₁ and R₁' have their previous significances and X is a group capable of being converted into a group of formula -CH₂NH₂ (Ia), the group X is converted into a group of formula Ia; or
c) a compound of formula IV 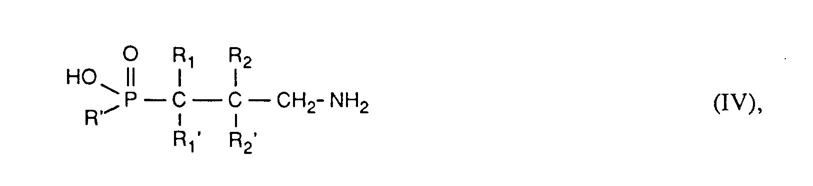 wherein R' may be selected from lower alkenyl, lower alkadienyl or lower alkynyl, to produce a compound of formula I, wherein R is lower alkyl, or from phenyl to produce a compound of formula I, wherein R is cyclohexyl, is reduced, and, if desired, a resulting salt obtained in this process may be converted into the free compound or into another salt and/or, if desired, a resulting free compound is converted into a salt to suit the above definition and/or, if desired, a resulting mixture of isomers is separated into the individual isomers.

Protected hydroxy groups such as groups -OR₅ present in a protected form in starting materials of the formula II are, for example, etherified hydroxy groups, such as hydroxy groups etherified with aliphatic, cycloaliphatic or araliphatic alcohol, e.g. with a lower alkanol, a cycloalkanol, or a phenyl- or diphenyl-lower alkanol, or hydroxy groups etherified with an aliphatic silanol, e.g. with a tri-lower alkyl silanol. As groups R₅O-, lower alkoxy, e.g. C₁-C₄-alkoxy, mono- or diphenyl-lower alkoxy, e.g. 1-phenyl- or 1,1-diphenyl-C₁-C₄-alkoxy, and tri-lower alkylsilyloxy, e.g. tri-C₁-C₄-alkyl-, such as trimethylsilyloxy, are especially preferred. Intermediarily protected hydroxy groups R'₂ are preferably hydroxy group etherified with an aliphatic silanol as specified hereinbefore.

Protected amino groups Z₀ as well as intermediarily protected amino groups as constituent of R in starting materials of the formula II are, for example, acylamino groups such as lower alkanoylamino, e.g. acetylamino, or phthalimido, lower alkoxycarbonylamino unsubstituted or substituted by phenyl, e.g. benzyloxycarbonylamino or tert.-butoxycarbonylamino groups, or 1-aryl-methylamino groups e.g. benzylamino, or 1-phenyl-lower alkylamino, silylated amino groups, such as tri-lower alkylsilylamino or especially bis-(tri-lower alkylsilyl)amino, e.g. bis-trimethylsilylamino. A latent amino group Z₀ may be e.g. nitro or azido.

Intermediarily protected oxo group R₂ + R₂' are preferably ketalised or thioketalised oxo group such as specified hereinafter.

Preferred compounds of formula II are those having the formula IIa 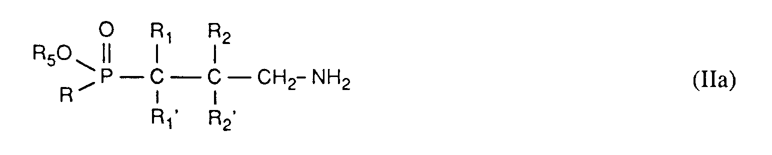
wherein R₅ represents a hydroxy-protective group, for example, C₁-C₄-alkyl or C₁-C₄-alkyl substituted by lower alkanoyloxy or by one or two optionally substituted phenyl groups, such as 1-C₂-C₇-alkanoyloxy-C₁-C₄-alkyl, e.g. pivaloyloxymethyl, or 1-phenyl- or 1,1-diphenyl-C₁-C₄-alkyl, e.g. benzyl, or having the formula IIb 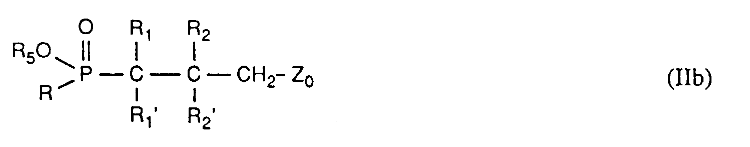
wherein R₅ represents a hydroxy-protective group, for example, C₁-C₄-alkyl, C₁-C₄-alkyl substituted by one or two optionally substituted phenyl groups, such as 1-phenyl- or 1,1-diphenyl-C₁-C₄-alkyl, e.g. benzyl, or a silyl group, such as tri-C₁-C₄-alkylsilyl, e.g. trimethylsilyl, and Z₀ has its previous significance and denotes, for example, C₁-C₇-alkanoylamino, e.g. acetylamido, phthalimido or bis-silylamino, such as bis(tri-C₁-C₄-alkylsilyl)amino, e.g. bis(trimethylsilyl)amino, or having the formula IIc 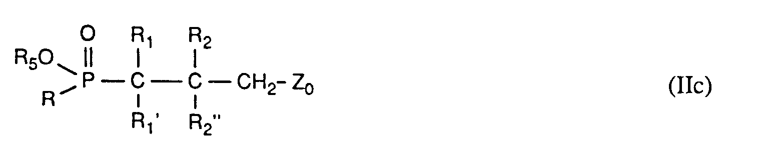
wherein R₅ represents a hydroxy-protective group, for example, C₁-C₄-alkyl, C₁-C₄-alkyl substituted by one or two optionally substituted phenyl groups, such as 1-phenyl- or 1,1-diphenyl-C₁-C₄-alkyl, e.g. benzyl, or a silyl group, such as tri-C₁-C₄-alkylsilyl, e.g. trimethylsilyl, and R₂'' is a silylated hydroxy group, such as trimethylsilyloxy, and wherein in formulae IIa, IIb and IIc R, R₁, R₁', R₂ and R₂' have their previous significances if not indicated otherwise.

The replacement of the protective group R₅ in compounds of formula II, IIa, IIb or IIc by hydrogen may be effected by treatment with a suitable basic or acidic agent such as an alkali metal hydroxide, e.g. sodium hydroxide, or lithium hydroxide, an alkali metal halide particularly bromide or iodide such as lithium bromide or sodium iodide, thiourea, an alkali metal thiophenolate such as sodium thiophenolate, or a protonic or Lewis acid, such as a mineral acid, e.g. hydrochloric acid or a tri-lower alkyl halosilane, e.g. trimethylchlorosilane. The replacement reaction may be carried out in the absence or presence of a solvent and, if necessary, while cooling or heating, in a closed vessel and/or under an atmosphere of an inert gas.

When R₅ denotes C₁-C₄-alkyl substituted in 1-position by one or two phenyl groups, e.g. when R₅ is benzyl, the replacement of such a group in compounds of formulae II, IIa or IIb by hydrogen may be effected by hydrogenolysis in the presence of a metallic hydrogenation catalyst, or any other suitable procedure.

Alternatively, the replacement of the protective group, e.g. of a silyl or, for example of an alkyl group, R₅ in compounds of formulae II, IIa, IIb or IIc by hydrogen may be effected by treatment with an acid under hydrolytic conditions, especially with a mineral acid such as a hydrohalic acid e.g. hydrochloric acid which is used in dilute or concentrated aqueous form, or by treatment with an organic silyl halide such as trimethylsilyl iodide or bromide, followed by hydrolysis, if necessary. The reaction is preferably conducted at elevated temperature e.g. while refluxing the reaction mixture and, if necessary using an organic diluent, in a closed vessel and/or under an atmosphere of an inert gas. The kind of replacement of the protective group R₅ depends e.g. on the substituent R present in a compound of formula II which must be retained in converting a compound of formula II to a compound of formula I. Said replacement may be effected e.g. according to the illustrating examples.

Protected amino group or latent amino groups Z₀ in compounds of formula II, IIb or IIc may be converted into free amino according to known methods, which are selected according to the characteristics of the protected or latent amino group to be converted into amino, such as solvolytic or hydrogenolytic procedures, for example, hydrolysis in the presence of an acid or a base, acidolysis, e.g. treatment with trifluoroacetic acid, treatment with hydrazine, or hydrogenolysis in the presence of a metallic hydrogenation catalyst, or any other suitable procedure.

Depending on the groups involved, the replacement and conversion operations may be carried out in any sequence or simultaneously by methods which are well known *per se*.

It is preferred that all protecting groups are converted, R₅ or hydroxy-protective groups of R₂' being converted hydrogen and Z₀ being converted to NH₂, in a single step, by treatment with an acid, preferably a hydrohalic acid, especially hydrochloric acid, under hydrolytic conditions.

The compounds of formula II may be prepared, for example, by various methods according to the nature of the group X in the formula V defined hereinafter, e.g. by reacting, in the presence of a basic catalyst or in the presence of agents forming free radicals, a compound of the formula V 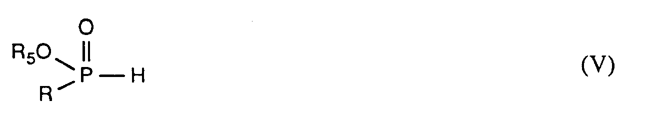
in which R and R₅ have their previous significance which can be prepared by reaction of a compound of the formula R-PHal₂ (Va; Hal = halogen) with an alcohol R₅OH in the presence of a tri-lower alkylamine or, more advantageously, by reaction of aqueous hypophosphonous acid with an orthoester of the formula C(C₁-C₄-alkyl)(OR₅)₃(Vb) yielding, in the latter case, a compound V, wherein R denotes C(C₁-C₄-alkyl)(OR₅)₂, with a compound of formula VI 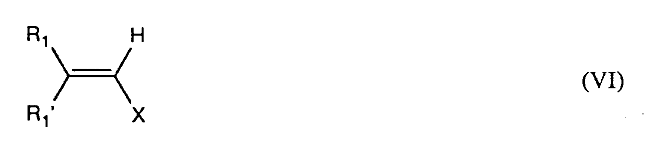
in which R₁ and R₁' have their previous significance and X is a group capable of being converted into a group of formula -CH₂Z, wherein Z has its previous significance, in order to produce a compound of formula VII 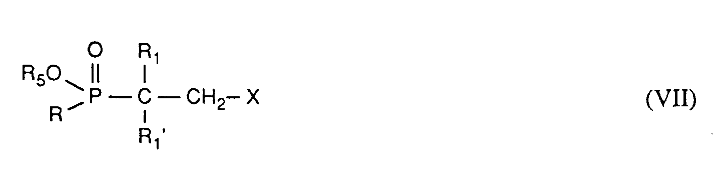
wherein R₁, R₁', R₅, R and X have their previous significances; and then converting the group X into a group of formula -CH₂Z.

A group X is primarily cyano but may also represent carbamoyl, a group of formula -CH₂Z₀ (VIIa) in which Z₀ has the previous significance; or X is a group of formula -CH=Y in which -C=Y is an optionally functionally modified carbonyl group such as a corresponding ketal or thioketal group, including a corresponding cyclic group.

The conversion of the group X into the group of the formula -CH₂-NH₂ is preferably effected in an analogous manner as described hereinafter for process variant b). If desired, said group may subsequently be re-protected by methods known *per se* into a group of the formula -CH₂-Z₀, wherein Z₀ has the meaning indicated.

The compounds of formula V are either known or they may be prepared by methods as described hereinbefore. Specific examples of compounds of formula V include: isopropyl (ethyl)phosphonite, isopropyl(n-propyl)-phosphonite, isobutyl(n-butyl)phosphonite, isobutyl(isopropyl)-phosphonite, isobutyl(isobutyl)phosphonite and isobutyl (sec.-butyl)-phosphonite.

Likewise, compounds of formula VI are either known or can be obtained by methods which are well known.

Alternatively, a compound of the formula VIII 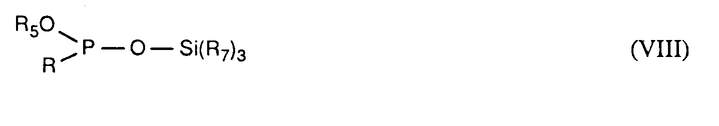
in which R₅ is C₁-C₄-alkyl or C₁-C₄-alkyl substituted by one or two phenyl residues or an additional group -Si(R₇)₃, each R₇, independently, is C₁-C₆-alkyl, preferably C₁-C₂-alkyl, particularly methyl, the groups R₅ and R₇ being the same or different, can be reacted with a compound of the formulae IXa, IXb or VI 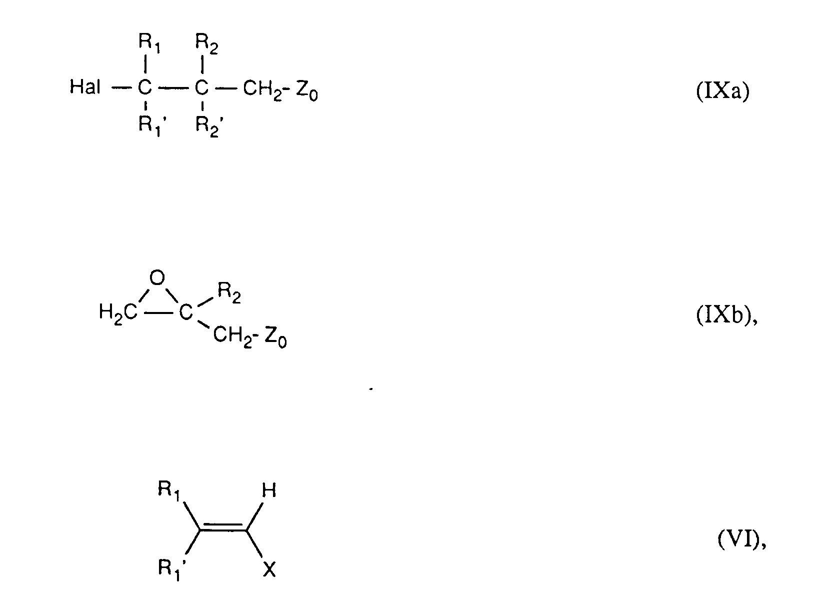
in which R₁, R₁', R₂, R₂', Z₀ and X have their previous significances, X being primarily cyano or a group of the formula -CH=Y and Hal stands for halogen, such as iodo, bromo or chloro. The reaction with an epoxide of formula IXb is advantageously carried out in the presence of a mild Lewis acid, such as anhydrous zinc chloride, whilst the reactions with halides of formulae IXa or VI are preferably carried out under the conditions of the Arbusov method, e.g. at a reaction temperature ranging from room temperature to an elevated temperature, e.g. 160°C, while removing the trialkyl silyl halide formed in the reaction.

The compounds of formula IIb may also be prepared by reacting a compound of the formula a compound of formula X 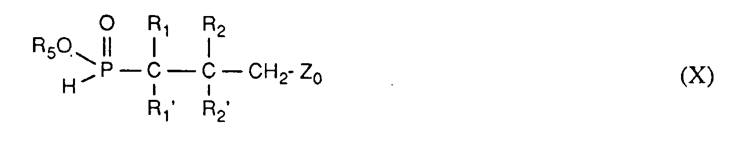
wherein R₁, R₁', R₂, R₂' and Z₀ have their previous significances and R₅O denotes protected, e.g. esterified, hydroxy, with a silylating agent, such as a hexa-lower alkyl silazane or a tri-lower alkyl halosilane, e.g. with hexamethyldisilazane, or with trimethylchlorosilane. in the presence of triethylamine, to produce a compound of formula XI 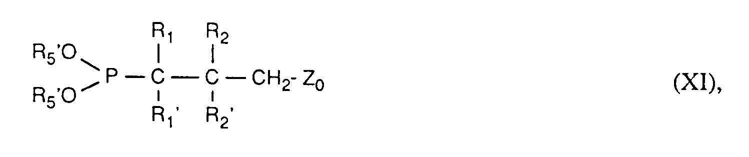
wherein R₅' denotes a group R₅ being tri-lower alkylsilyl, e.g. trimethylsilyl, and Z₀ denotes bis(tri-lower alkylsilyl)amino, such as bis(trimethylsilylamino).

The intermediate of the formula X or XI is then reacted with a compound capable of converting the 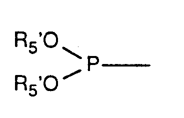
group into a 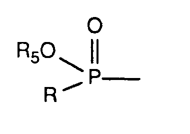
group
wherein R has its previous significance to produce a compound of formula IIb, in which R₅ has its previous significance. Thus, the intermediate of the formula X may be reacted, in the presence of a basic condensation agent, such as a tri-lower alkyl amine, e.g. of N-ethyl-N,N-diisopropyl-amine, with a corresponding halide, e.g. a lower alkyl halide of the formula R-Hal (XII, Hal=halogen), preferably under basic conditions, or may be reacted, for the manufacture of compounds IIb, in which R is an aliphatic, cycloaliphatic-aliphatic or araliphatic hydrocarbon radical having at least 2 carbon atoms in each of the aliphatic moieties, with a terminally unsaturated aliphatic, cycloaliphatic-aliphatic or araliphatic hydrocarbon having an terminal double bond between the carbon atom via which R is to be bonded to the P-atom and the adjacent carbon atom, or may be reacted, for the manufacture of compounds IIb, in which the carbon atom via which R is bonded to the P-atom is α-substituted by one hydroxy group, with a corresponding aliphatic, cycloaliphatic, cycloaliphatic-aliphatic or araliphatic aldehyde or ketone which corresponds, if the aldehyde or ketone functional group is replaced by one free valence and one hydroxy group, to the group R in the desired intermediate IIb.

Starting materials of formula X can be obtained starting from a compound of formula XIII or XIV 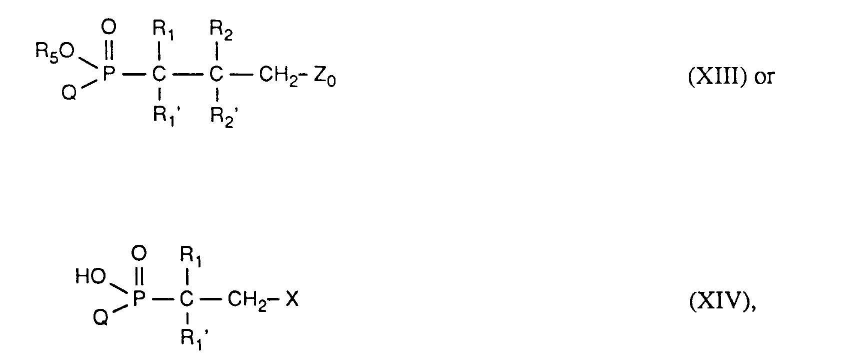
wherein Q is an α,α-di-lower alkoxy-lower alkyl group and R₁, R₁', R₂ R₂', X and Z₀ have their previous significance, the respective protecting groups R₅ and Z₀, or R₅ and X, respectively, being retained, when the compound of formula XIII or XIV is treated with a protic anhydrous medium.

Examples of such protic anhydrous media include: anhydrous hydrogen chloride gas, or an anhydrous medium may be generated from an organic compound having one or more Si-Cl bonds together with an agent e.g. an alkanol capable of cleaving the Si-Cl bond, to produce an anhydrous protic medium *in situ*.

Preferred anhydrous protic media include therefore trimethyl silyl chloride in dichloromethane/ethanol or in technical chloroform which contains ethanol.

The group Q preferably has the formula -C(R₈)-C(OR₉)(OR₁₀)(XV) in which R₈ denotes lower alkyl and R₉ and R₁₀, independently of one another, represent lower alkyl or together represent lower alkylene.

This process for the manufacture of compounds of the formula X is preferably carried out at a temperature ranging from -80°C to 100°C, preferably from 0°C-50°C.

While the relative molar ratios of the reactants i.e. of reactant XII or XIV to the organic silyl chloride, used may vary within a wide range, it is preferred to use molar ratios ranging from 1 to 2 molar equivalents of the latter, per molar equivalent of XII or XIV.

Compounds of the formula IIb, wherein R₁ and R₁' denote hydrogen, R₂ denotes hydroxy and R₂' represents hydrogen or R₂ and R₂' together denote oxo, may also be produced by reacting a compound having the formula XV 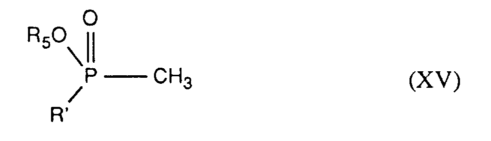
in the form of the salt of the formula XV' 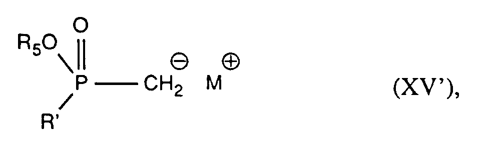
wherein R₅ has its previous significance and R' is an aliphatic, cycloaliphatic or cycloaliphatic-aliphatic hydrocarbon radical, and M is an alkali metal, alkaline earth metal or transition metal, preferably lithium, sodium or potassium, calcium, zinc or tin, with a compound having the formula XVI 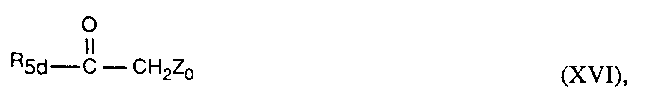
wherein R_{5d} denotes a hydroxy protective group such as specified in formula IIb for R₅, halogeno, such as chloro or bromo, or hydrogen and Z₀ has its previous significance, to produce a compound having the formula IIb wherein R₅ is R_{5d}, R is R', R₁ and R'₁denote hydrogen, R₂ is hydroxy and R₂' denotes hydrogen or R₂ and R₂' together denote oxo.

Intermediates of the formula IIc in which R₁ and R₁' represent hydrogen, R₂ denotes an aromatic radical and R₂' is a silylated hydroxy group, are preferably prepared by a novel process reacting hypophosphonous acid of formula XVII with propargyl alcohol of formula XVIII 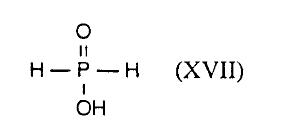
and

HO-CH₂C≡CH (XVIII)

in an araliphatic hydrocarbon solvent, such as toluene, to give a compound of the formula XIX 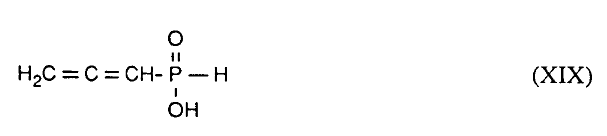
which, on reaction first with a haloformic acid lower alkyl ester of the formula Hal-COOR₅ (XX), wherein Hal denotes halo and R denotes lower alkyl, such as ethyl chloroformate, in the presence of a tri-lower alkylamine, such as triethylamine, and subsequently with an orthoacetic acid tri-lower alkylester, such as triethyl orthoacetate, in the presence of a Lewis acid, such as boron trifluoride, at 0° to 30° C, yields a compound of the formula XXI

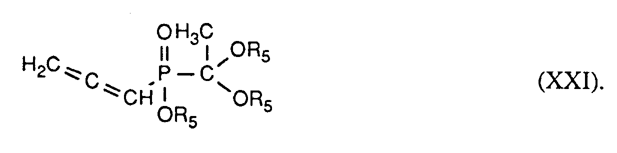

The compound of the formula XXI is then reacted with compound of the formula R₂-M (XXII), wherein M denotes a metallic group, such as a halomagnesium group, for example, the iodomagnesium group and copper-I-bromide/dimethylsulphide complex, preferably in dimethylsulphide/diethyl ether at -10° to -30° C, giving a compound of the formula XXIII

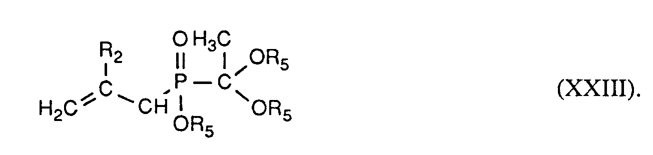

The P-protecting group is removed as described hereinbefore, preferably by action of trimethylsilyl chloride in an approximately 9:1 mixture of dichloromethane and ethanol, and, without isolation reacted subsequently with butyl lithium in tetrahydrofuran at -60° to -80° and a compound of the formula R-Hal (XXIV), wherein R has the meanings indicated and Hal denotes a halogen atom to give a compound of the formula XXV

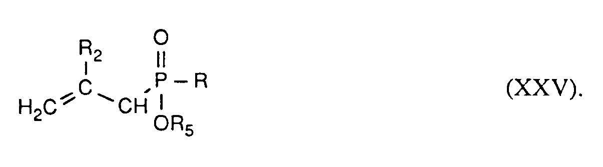

The product obtained from the reaction of tertiary butyl urethane and tertiary butyloxychloride in the presence of sodium hydroxide in a lower alkanol is then reacted with the intermediate of formula XXV in the presence of osmium tetroxide and silver nitrate in a mixture of acetonitrile, water and tertiary butanol, to form a compound of the formula IIc in which R₅, R₁, R₁', R₂ and R₂'' have the meanings indicated. This intermediate can be converted into the corresponding compound of the formula I, for example, by reaction with a trimethylsilyl halide, such as trimethylsilyl bromide, in dichloromethane, and then treatment with aqueous methanol and subsequently with propylene oxide in ethanol.

In a preferred embodiment of process variant a) for the manufacture of compounds of formula I a compound of the formula IIa 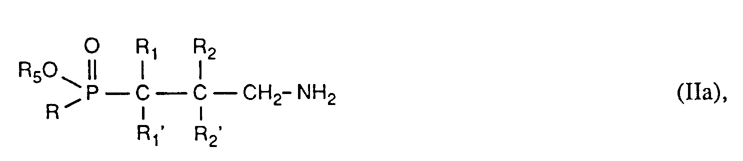
wherein R₅ denotes lower alkyl and R, R₁, R₁', R₂ and R₂' have their previous significances, is subjected to basic or acidic hydrolysis or is treated with a tri-lower alkyl halosilane

In starting materials of the formula III for process variant b) X is primarily cyano but may also represent carbamoyl, a group of formula -CH₂Z₀ (VIIa) in which Z₀ has the previous significance; or X is a group of formula -CH=Y in which -C=Y is an optionally functionally modified carbonyl group such as a corresponding ketal or thioketal group, including a corresponding cyclic group.

When, in a compound of formula III X is an activating group Xₐ such as cyano or -CH=O, then either a basic catalyst or a free radical catalyst may be employed. When, however, X is e.g. a residue of formula -CH₂Z₀, then free radical catalysts are required.

A basic catalyst used in the first step may be e.g. an alkali metal C₁-C₄-alkoxide, for example, a sodium or potassium C₁-C₄-alkoxide, in particular sodium methoxide, sodium ethoxide or potassium tert.-butoxide, an alkaline or alkaline earth metal fluoride, such as potassium fluoride or caesium fluoride, or an alkali metal hydride, such as sodium hydride. The reaction may be effected with or without the use of an added solvent. If a solvent is added, this is preferably an alcohol, in particular a C₁-C₄-alkanol corresponding to the alkoxide used as basic catalyst. The reaction temperature may vary from 0° C to the boiling point of any added solvent.

Agents forming free radicals are, for example, compound convertible into free radicals by ionizing or ultra-violet radiation, preferably peroxy compounds, such as inorganic peroxy compounds, e.g. hydrogen peroxide or ammonium persulphate, or organic peroxides, e.g. benzoyl peroxide or tert.-butyl peroxide, or organic azo compounds, e.g. azo-bis-isobutyronitrile. Reactions involving free radical-forming agents may be conducted in the optional presence of a solvent and, if necessary, while cooling or heating, in a closed vessel and/or in an atmosphere of an inert gas.

The conversion of a group X into the group -CH₂-NH₂ is carried out according to known methods. Cyano and carbamoyl are converted into aminomethyl by reduction, cyano, for example, by hydrogenation in the presence of a suitable catalyst, e.g. Raney nickel and of a solvent, such as ethanol, which may preferably contain ammonia, and carbamoyl, for example, by treatment with a suitable hydride reducing agent, such as borane in tetrahydrofuran.

The conversion of a group -CH=Y, in which Y is oxygen, into the group of the formula -CH₂-NH₂ is carried out by known reductive amination procedures, e.g. treatment with sodium cyanoborohydride in the presence of ammonium acetate in a suitable solvent, such as dioxane, and while cooling, e.g. at about 0°C.

These reactions are carried out according to known methods, in the absence or presence of a solvent, which may also serve as a reagent, if necessary, while cooling or heating, in a closed vessel and/or in the atmosphere of an inert gas.

The compounds of formula III may be prepared in an analogous manner as described hereinbefore for the preparation of intermediates of the formula VII. For example, compounds of the formula III, wherein R₂ and R₂' are hydrogen and R, R₁, R₁' and X have the meanings indicated, can be obtained e.g. by reacting, in the presence of a basic catalyst or in the presence of agents forming free radicals, a compound of the formula V 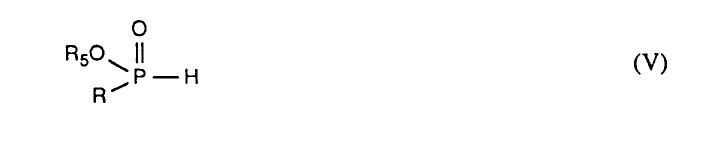
in which R and R₅ have their previous significance, with a compound of formula VI 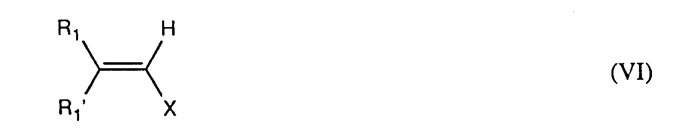
in which R₁, R₁' and X have their previous significance, and subsequently removing the O-protecting group R₅, for example, as described hereinbefore under process variant a) in order to produce a compound of formula III.

In a variation of this process, suitable for the preparation of compounds of the formula III, wherein R has the meaning indicated, R₁ and R₁' denote hydrogen, R₂ₐ denotes a aliphatic, cycloaliphatic, cycloaliphatic-aliphatic or aromatic hydrocarbon radical and X denotes carbamoyl, the compound of the formula V is O-protected, for example, by means of trimethylsilyl chloride and then reacted with a corresponding epoxide of the formula XXVI 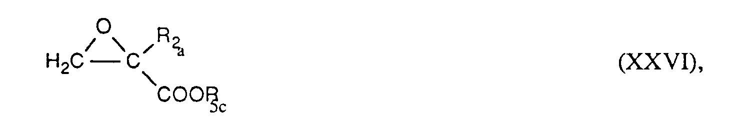
wherein R_{5c} represents lower alkyl, forming a compound of the formula XXVII 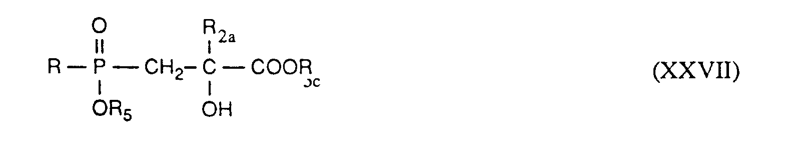
which is then reacted with ammonia in a lower alkanol, such as ethanol, in the presence of an alkali metal cyanide, such as sodium cyanide. Subsequently, the the group R₅ is split off to give the corresponding compound of the formula III, wherein R₁ and R₁' denote hydrogen, R₂ is a group R₂ₐ, R₂' is hydroxy and X denotes carbamoyl.

Alternatively, a compound of the formula VIII 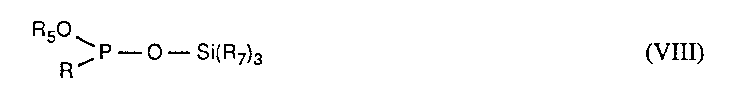
in which R₅ is C₁-C₄-alkyl or C₁-C₄-alkyl substituted by one or two phenyl residues or an additional group -Si(R₇)₃, each R₇, independently, is C₁-C₆-alkyl, preferably C₁-C₂-alkyl, particularly methyl, the groups R₅ and R₇ being the same or different, can be reacted with a compound of the formulae XXVIIIa, XXVIIIb 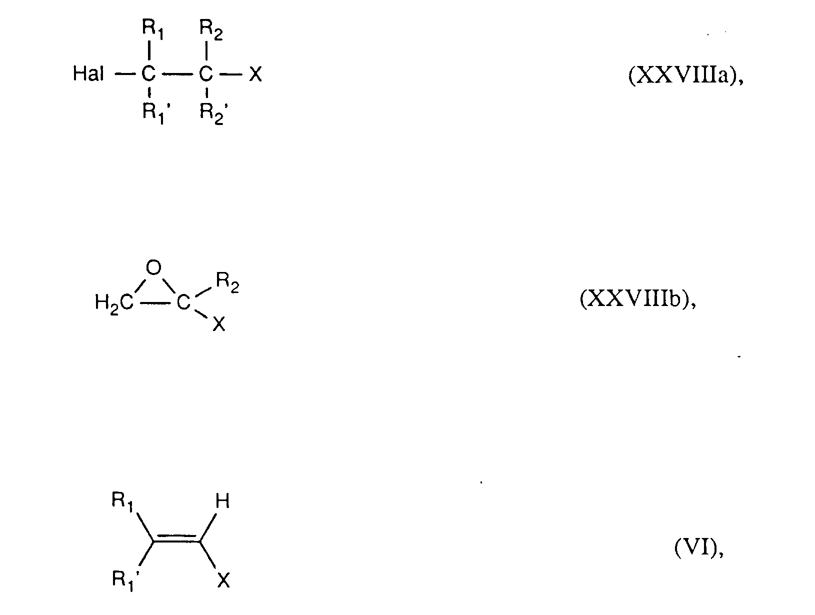
in which R₁, R₁', R₂, R₂' and X have their previous significances, X being primarily cyano or a group of the formula -CH=Y, and Hal stands for halogen, such as iodo, bromo or chloro. The reaction with an epoxide of formula XXVIIIb is advantageously carried out in the presence of a mild Lewis acid, such as anhydrous zinc chloride, whilst the reaction with halides of formulae XXVIIIa is preferably carried out under the conditions of the Arbusov method, e.g. at a reaction temperature ranging from room temperature to an elevated temperature, e.g. 160°C, while removing the trialkyl silyl halide formed in the reaction.

The reduction of compounds of the formula IV according to process variant c) may be effected by any suitable reducing agent, such as hydrogen in the presence of a catalyst, for the reduction of aryl e.g. Raney nickel or Nishimura catalyst and for the reduction of aliphatic multiple bonds e.g. Palladium on charcoal, in the presence or absence of a solvent and at room temperature or elevated temperature.

The compounds of formula IV may be produced according to any of the methods described herein for the manufacture of compounds of formula I starting from starting materials having the respective unsaturated substituent R' instead of R. Furthermore, compounds of formula IV may also be obtained starting from the corresponding R'-dichlorophosphine by reaction with a lower alkanol, such as ethanol, and a tri-lower alkylamine, such as triethylamine, reacting the resulting R'-phosphonous acid ester with a compound of formula VI 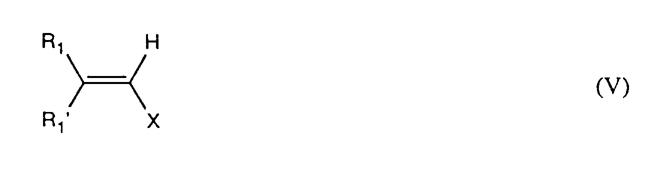
and converting the group X into the corresponding group -CH₂-NH₂.

Alternatively, a compound of the formula XXIX 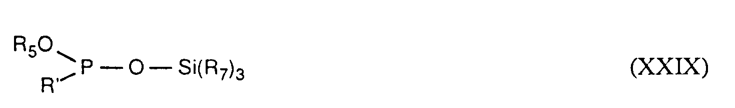
in which R₅ is C₁-C₄-alkyl or C₁-C₄-alkyl substituted by one or two phenyl residues or an additional group -Si(R₇)₃, each R₇, independently, is C₁-C₆-alkyl, preferably C₁-C₂-alkyl, particularly methyl, the groups R₅ and R₇ being the same or different, can be reacted with a compound of the formulae IXa, IXb or VI 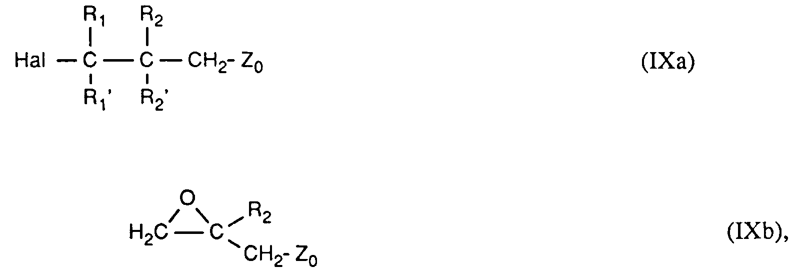 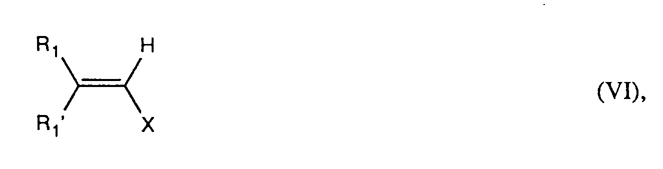
in which R₁, R₁', R₂, R₂', Z₀ and X have their previous significances, X being primarily cyano or a group of the formula -CH=Y and Hal stands for halogen, such as iodo, bromo or chloro. The reaction with an epoxide of formula IXb is advantageously carried out in the presence of a mild Lewis acid, such as anhydrous zinc chloride, whilst the reactions with halides of formulae IXa or VI are preferably carried out under the conditions of the Arbusov method, e.g. at a reaction temperature ranging from room temperature to an elevated temperature, e.g. 160°C, while removing the trialkyl silyl halide formed in the reaction.

In either process, the amino group is set free from a protected amino group Z₀ and, if present, the hydroxy group is set free from a protected hydroxy group in order to obtain the corresponding compound of the formula IV.

All above-mentioned reactions are carried out according to standard methods, in the presence or absence of diluents, preferably such as are inert to the reagents and are solvents thereof, of catalysts, condensing or said other agents respectively and/or inert atmospheres, at low temperatures, room temperature or elevated temperatures preferably near the boiling point of the solvents used, at atmospheric or super-atmospheric pressure.

Compounds of the formula I obtainable according to the process of the invention may be interconverted into another.

Thus, compounds of formula I, wherein R is substituted by hydroxy, and/or R₂' denotes hydroxy, can be converted into the corresponding hydroxy-free compounds, for example, by reacting with thiocarbonyldiimidazole and treating the resulting imidazolylthiourethane in the presence of a radical-initiator, such as azoisobutyronitrile, with a tri-lower alkyl-stannane, e.g. with (C₄H₉)₃SnH, for example in benzene at 60 to 80°C.

Also double and/or triple bonds present in the group R may be reduced to single bonds, triple bonds also to double bonds to yield the corresponding less unsaturated compound of formula I.

The invention further includes any variant of the present processes, in which an intermediate product obtainable at any stage thereof is used as starting material and the remaining steps are carried out, or in which the starting materials are formed under the reaction conditions, or in which the reaction components are used in the form of their salts or optically pure antipodes. Whenever desirable, the above processes are carried out after first suitably protecting any potentially interfering reactive functional groups, e.g. as illustrated herein.

Advantageously, those starting materials should be used in said reactions that lead to the formation of those compounds indicated above as being preferred.

The invention also relates to novel starting materials, especially to those leading to the preferred compounds of the formula I, and to processes for their manufacture.

Depending on the choice of starting materials and methods, the new compounds may be in the form of one of the possible isomers, for example, as diastereomers, as optical isomers (antipodes), as racemates, or as mixtures thereof.

In case diastereomeric mixtures of the above compounds or intermediates are obtained, these can be separated into the single racemic or optically active isomers by methods in themselves known, e.g. by fractional distillation, crystallization or chromatography.

The racemic products of formula I or basic intermediates can be resolved into the optical antipodes, for example, by separation of diastereomeric salts thereof, e.g., by the fractional crystallization of d- or l-(tartrate, dibenzoyltartrate, mandelate or camphorsulphonate) salts.

Advantageously, the more active of the antipodes of the compounds of this invention is isolated.

Furthermore, the compounds of the invention are either obtained in the free (Zwitterion-) form, or as a salt thereof. For example, any resulting free compound can be converted into a corresponding acid addition salt, preferably with the use of a pharmaceutically acceptable acid or anion exchange preparation, salts with bases by treatment of the free compounds with bases or suitable cation exchange techniques, or resulting salts can be converted into the corresponding free compounds, for example the acid addition salts, with the use of a stronger base, such as a metal or ammonium hydroxide, or any basic salt, e.g., an alkali metal hydroxide or carbonate, or a cation exchange preparation and the salts with bases by treatment with suitable acidic reagents. These or other salts, for example, the picrates, can also be used for purification of the compounds obtained; the compounds are then first converted into salts. In view of the close relationship between the free compounds and the compounds in the form of their salts, whenever a compound is referred to in this context, a corresponding salt is also intended, provided such is possible or appropriate under the circumstances and the term "salts" shall, if desired also include the free compounds, where appropriate according to meaning and purpose.

The compounds, including their salts, may also be obtained in the form of their hydrates, or include other solvents used for the crystallization.

The present invention also relates to the use of the compounds of the invention for the preparation of pharmaceutical compositions, especially pharmaceutical compositions having selective GABA_{B}-antagonistic activity which can be used for the treatment of e.g. cognitive and memory disorders, depressive states of mind and anxieties.

The pharmaceutical compositions according to the invention are those suitable for enteral, such as oral or rectal, and parenteral administration to mammals, including man, for the treatment of diseases responsive to GABA_{B}-receptor blocking as given above, comprising an effective GABA_{B}-receptor blocking amount of a compound of the invention, alone or in combination with one or more pharmaceutically acceptable carriers.

The pharmacologically active compounds of the invention are incorporated into pharmaceutical compositions comprising an effective amount thereof in conjunction or admixture with excipients or carriers suitable for either enteral or parenteral application.

Preferred are tablets and gelatin capsules comprising the active ingredient together with a) diluents, e.g. lactose, dextrose, sucrose, mannitol, sorbitol, cellulose and/or glycine; b) lubricants, e.g. silica, talcum, stearic acid, its magnesium or calcium salts and/or polyethylene glycol; for tablets also c) binders, e.g. magnesium aluminium silicate, starch paste, gelatin, tragacanth, methylcellulose, sodium carboxymethylcellulose and/or polyvinylpyrrolidone; if desired, d) disintegrants, e.g. starches, agar, alginic acid or its sodium salt, or effervescent mixtures; and/or e) absorbents, colourants, flavours and sweeteners. Injectable compositions are preferably aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, the compositions may also contain other therapeutically valuable substances. Said compositions are prepared according to conventional mixing, granulating or coating methods, respectively, and contain about 0.1 to 75 %, preferably about 1 to 50 %, of the active ingredient.

The present invention also relates to the use of compounds of the invention having GABA_{B}-antagonistic properties and pharmaceutical compositions comprising said compounds for the treatment in mammals of disorders responsive to selective GABA_{B}-receptor blocking, particularly cognitive and memory disorders, and also of depressions and anxieties.

One aspect relates advantageously to the method of treatment of nootropic disorders in mammals, using an effective amount of a compound of the invention, preferably in the form of above-cited pharmaceutical compositions.

The dosage of active compound administered is dependent on the species of warm-blooded animal (mammal), the body weight, age and individual condition, and on the form of administration.

A unit dosage for a mammal of about 50 to 70 kg may contain between about 10 and 500 mg of the active ingredient.

The following examples are intended to illustrate the invention and are not to be construed as being limitations thereon. Temperatures are given in degrees Centigrade. If not mentioned otherwise, all evaporations are performed under reduced pressure, preferably between about 2 and 13 kPa. The structure of final products, intermediates and starting materials is confirmed by analytical methods, e.g. microanalysis and spectroscopic characteristics (e.g. MS, IR, NMR). The compounds of formula I are hereinafter referred to as 3-amino-1-R₁-1-R₁'-2-R₂-2-R₂'-propyl(R)phosphinic acids.

Example 1: A solution of 0.642 g of ethyl 3-(N-tert.-butoxycarbonylamino)-2-oxo-propyl(n-butyl)phosphinate in 10 ml of anhydrous dichloromethane under an inert atmosphere is treated with 1.53 g of trimethylbromosilane. After stirring for 7 hours at room temperature the volatile material is removed *in vacuo* to afford a pale yellow oil. This oil is dissolved in methanol containing 1 % of water, and the clear pale yellow solution is stirred overnight at room temperature. The solvent is removed *in vacuo* to afford 3-amino-2-oxo-propyl(n-butyl)phosphinic acid hydrobromide, m.p. 135-138°. Suspension in ethanol and treatment with propylene oxide affords 3-amino-2-oxo-propyl-(n-butyl)phosphinic acid of m.p. 128-130°.

The starting material may be prepared as follows.

A suspension of 8.0 g of sodium hydride (55 % dispersion in oil) in 35 ml of anhydrous tetrahydrofuran under an inert atmosphere is treated with 35 ml of an anhydrous tetrahydrofuran solution of 25 g of ethyl n-butylphosphinate. The resulting suspension is stirred at room temperature for 1 hour before dropwise addition of 32 ml of methyl iodide. After 3 hours stirring at room temperature 10 ml of water are carefully added followed by 200 ml of dichloromethane. Separation of the organic layer, drying over anhydrous magnesium sulphate and removal of the solvent *in vacuo* affords an oil. Kugelrohrdistillation at 90° and 10⁻¹ mbar affords ethyl n-butyl-(methyl)phosphinate.

A solution of 5.82 g of lithium diisopropylamide in 30 ml of anhydrous tetrahydrofuran is cooled to -78°C under an inert atmosphere. Under constant stirring at -78° a solution of 9.84 g of ethyl n-butyl(methyl)phosphinate in 20 ml of anhydrous tetrahydrofuran is added over 15 minutes under a positive pressure of an inert atmosphere via a canula. The resulting pale yellow solution is stirred for 1 hour at -78° and a pre-cooled (-78°C) solution of 1.89 g of N-tert.-butoxycarbonylglycine methyl ester in 10 ml of anhydrous tetrahydrofuran is added over 5 minutes via a canula. Thin layer chromatography indicates complete reaction after circa 10 minutes. At -78° 4.0 g of of glacial acetic acid is added and the mixture is allowed to warm to room temperature. The reaction mixture is diluted with dichloromethane and washed with water. Drying over magnesium sulphate and removal of the solvent *in vacuo* affords a pale yellow oil. Removal of the excess starting material by distillation and chromatography of the resulting yellow oil on silica gel yields ethyl 3-(N-tert.-butoxycarbonylamino)-2-oxo-propyl(n-butyl)phosphinate as a colourless viscous oil.

Example 2: A solution of 1.2 g of ethyl 3-amino-2-(4-chlorophenyl)-2-hydroxy-propyl-(n-butyl)phosphinate hydrochloride in 5 ml of ethanol is treated with 0.24 g of sodium hydroxide dissolved in 3 ml of water. The solution heated to 60° for 20 hours. After this time the solution is cooled to room temperature and washed 2-times with 50 ml each of dichloromethane and once with 50 ml of diethyl ether. The aqueous phase is evaporated to dryness. The residue is suspended in n-propanol, heated to 80°C for 10 minutes and filtered. The n-propanol is removed *in vacuo* to afford a white solid. Chromatography on reverse-phase silica gel followed by drying in vacuo at 80° yields sodium 3-amino-2-(4-chlorophenyl)-2-hydroxy-propyl(n-butyl)phosphinate; m.p. 215-215.5°; 1H-NMR spectrum (D₂O): δ = 7.4 ppm (4H, s), 2.91 ppm (2H, ABq), 2.22 ppm (2H, ABq,d), 1.37-1.0 ppm (611, m), 0.75 ppm (3H, t).

The starting material may be obtained as follows:
To a solution of 19.6 g of methyl 2-(4-chlorophenyl)acrylate in 200 ml of dry chloroform, under an inert atmosphere are added 47.06 g of 55 % m-chloroperbenzoic acid and the resulting solution is refluxed for 18 hours. The mixture is then cooled and the solid removed by filtration. Evaporation *in vacuo* of the filtrate and chromatography of the residue on silica-gel affords methyl 2-(4-chlorophenyl)-2,3-epoxy-propionate as a colourless oil.

A solution of 4.5 g of ethyl n-butylphosphinate in anhydrous tetrahydrofuran containing 3.79 g of triethylamine is treated under an inert gas with an anhydrous tetrahydrofuran solution of 4.07 g of trimethylchlorosilane. A white precipitate forms immediately and the resulting suspension is stirred at room temperature under an inert gas for 24 hours. The reaction mixture is then filtered under an inert gas and the filtrate is concentrated *in vacuo* to afford a cloudy oil which is then treated with 2.58 g of methyl 2-(4-chlorophenyl)-2,3-epoxy-propionate and 0.5 g of anhydrous zinc chloride. An exothermic reaction results. After the exothermia has subsided, the clear solution is heated slowly to 70° and stirred at this temperature for 6 more hours after which time chromatographic analysis indicates completeness of the reaction. The reaction mixture is cooled to room temperature, diluted with dichloromethane and washed with water. Drying of the organic layer over anhydrous magnesium sulphate and removal of the solvent *in vacuo* affords an oil. The excess starting material is removed by Kugelrohr-distillation in high vacuum and the pale yellow residue is chromatographed on silica-gel to yield methyl 2-(4-chlorophenyl)-2-trimethylsilyloxy-3-(0-ethyl-P-n-butyl-phospinyl)-propionate as a viscous, colourless oil.

To a solution of 9.16 g of methyl 2-(4-chlorophenyl)-2-trimethylsilyloxy-3-(0-ethyl-P-n-butyl-phosphinyl)-propionate in 70 ml of absolute ethanol are added 0.103 g of sodium cyanide followed by 20 g of liquid ammonia. The resulting mixture is heated in an autoclave for 20 hours at 50° and 12 bar. The ammonia is removed under water pump vacuum and the ethanol is moved by rotary evaporation to afford a light brown-coloured oil. Chromatography on silica-gel affords 2-(4-chlorophenyl)-2-hydroxy-3-(0-ethyl-P-n-butylphosphinyl)-propionamide as an oily solid with an ill defined melting point of circa 100°.

A solution of 1.04 g of 2-(4-chlorophenyl)-2-hydroxy-3-(0-ethyl-P-n-butylphosphinyl)-propionamide in 10 ml of anhydrous tetrahydrofuran is heated to reflux and treated with 0.69 g of borane/dimethyl sulphide complex over 15 minutes while collecting the liberated dimethyl sulphide by distillation. Reflux is continued for 4 hours and the reaction mixture cooled to room temperature and treated with 0.85 g of absolute methanol. After addition is complete the reaction is stirred for a further 30 minutes before cooling to 0° and addition of 1.19 ml of a 2.0 m solution of hydrogen chloride in absolute ether. The solvent is then removed to afford ethyl 3-amino-2-(4-chlorophenyl)-2-hydroxy-propyl(n-butyl)-phosphinate hydrochloride as a hygroscopic, glassy white solid.

Example 3: A solution of 5.0 g of 2-cyano-1-fluoro-ethenyl(n-butyl)phosphinic acid and 10 ml of liquid ammonia in 150 ml of absolute ethanol is treated with 0.75 g of 5 % rhodium on charcoal. The suspension is shaken under hydrogen (20 bars) at 20°C until thin-layer chromatography indicates complete reaction. The catalyst is removed by filtration and the filtrate is evaporated *in vacuo* to give a solid. Crystallisation from ethanol/acetone affords 3-amino-1-fluoro-propyl(n-butyl)phosphinic acid.

The starting material may be obtained as follows:
24 g of sodium hydride (55 % dispersion in oil) is washed with hexane and suspended in 100 ml of absolute tetrahydrofuran under an inert atmosphere. A solution of 104.4 g of ethyl diethoxymethylphosphinate in 100 ml of anhydrous tetrahydrofuran is added dropwise maintaining the temperature at 20°. The reaction is exothermic and gas evolution results. After the addition is complete, stirring is continued for 1.5 hours before adding 209.7 g of n-butylbromide at 20°. Finally, the suspension is stirred for 2.5 hours, cooled in an ice/water bath and water is added carefully. The mixture is concentrated *in vacuo* and the residue partitioned between dichloromethane and water. Drying of the organic layer over sodium sulphate and removal of the solvent affords a colourless oil. Distillation in high vacuo affords ethyl diethoxymethyl(n-butyl)-phosphinate; b.p. 71.5-74° (10⁻³ mbar).

109 g of ethyl diethoxymethyl(n-butyl)phosphinate are dissolved in 160 ml of 4.0 m aqueous hydrochloric acid. The clear solution is warmed to reflux for 24 hours, then cooled to room temperature and washed with diethyl ether. Evaporation of the aqueous layer affords n-butylphosphonous acid as an oil. This oil is dried in high vacuo. A former sample is obtained by washing the ether extracts with hexane followed by water and evaporation of the aqueous layer.

A suspension of 12.2 g of n-butylphosphonous acid in 50 ml of hexamethyldisilazane is heated to reflux under an inert atmosphere for 24 hours. The excess hexamethyldisilazane is removed by distillation at atmospheric pressure. To the residue are added 4.45 g of 3,3-difluoro-acrylonitrile. The reaction mixture is stirred at room temperature for 20 hours followed by heating 50°C for 2 hours. After cooling to room temperature, the mixture is diluted with dichloromethane and washed with water. The organic layer is dried over sodium sulphate and evaporated *in vacuo* to afford 2-cyano-1-fluoro-ethenyl(n-butyl)phosphinic acid as an oil which can be used without further purification.

Example 4: A solution of 1.0 g of ethyl-3-(N-tert.butoxycarbonylamino)-2-oxo-propyl(cyclohexylmethyl)phosphinate in 15 ml of anhydrous dichloromethane under an inert atmosphere at room temperature is treated with 2.1 g of trimethylbromosilane. After stirring for 7 hours the volatile components are removed under reduced pressure to give a pale yellow oil. This oil is dissolved in methanol containing 1 % of water and the clear pale yellow solution is stirred overnight at room temperature. The solvent is removed under reduced pressure to afford 3-amino-2-oxo-propyl(cyclohexylmethyl)phosphinic acid hydrobromide, m.p. 180-182°C. Suspension in ethanol and treatment with propylene oxide affords 3-amino-2-oxo-propyl(cyclohexylmethyl)phosphinic acid, ¹H-NMR (D₂O): δ(ppm) = 4.15 (2H, s, CH₂N), 3.10 (2H, d, J = 15 Hz, CH₂-P), 1.80 (2H, m, P-CH₂), 1.73-1.52 (5H, m, 2 x CH₂ + CH), 1.34-0.95 (6H, m, 3 x CH₂).

The starting material may be prepared as follows:
A suspension of 16.5 g of sodium hydride (80 % in oil) in 100 ml of anhydrous tetrahydrofuran under an inert atmosphere at 20° is treated with 100 ml of an anhydrous tetrahydrofuran solution of 104.4 g of ethyl (diethoxymethyl)phosphinate at such a rate so that the temperature does not exceed 25°. After the addition is complete, the resulting suspension is stirred for 1 hour at room temperature. This suspension is then treated with 85.5 g of benzylbromide and the reaction mixture stirred overnight at room temperature. The reaction mixture is then cooled in an ice/water bath and 200 ml of water are carefully added. The clear solution is then partitioned between dichloromethane and water. Separation of the dichloromethane layer followed by drying and removal of the solvent affords a pale yellow oil. Distillation in high vacuum affords ethyl-P-benzyl-P-diethoxymethylphosphinate of b.p. 105-125° (10⁻² mbar); ¹H-NMR (CDCl₃): δ(ppm) = 7.29 (5H, m, Ph), 4.58 (1H, d, J = 9 Hz, CHP), 4.08 (2H, q, CH₂OP), 3.83 and 3.66 (4H, m, 2 x CH₂OC), 3.21 (2H, AB_{q} J = 15 x 6.0 Hz, CH₂Ph), 1.23 (9H, m, 3 x CH₃).

A solution of 4.29 g of ethyl P-benzyl-P-diethoxymethyl phosphinate in 42 ml of absolute ethanol is treated with 10 g of Raney nickel and the suspension hydrogenated at 100-110°C and 120 bar for 34 hours. The catalyst is removed by filtration and the residue washed with absolute ethanol. The combined filtrate and washings are concentrated in value to afford an oil. Distillation in high vacuum affords O-ethyl-P-cyclohexylmethyl-P-diethoxymethylphosphinate; b.p. 103-105° (10⁻² mbar); ¹H-NMR (CDCl₃): δ(ppm) = 4.62 (1H, d, J = 9 Hz, CHP), 4.17 (2H, m, CH₂OP), 3.84 and 3.69 (4H, m, 2 x CH₂OC), 1.88 (3H, m, CH₂ + CH), 1.68 (6H, m, 3 x CH₂), 1.30 (9H, m, 3 x CH₃).

A solution of 38.3 g of ethyl P-cyclohexylmethyl-P-diethoxymethylphosphinate in 27 ml of water is treated with 27 ml of 37 % aqueous hydrochloric acid and the mixture heated to reflux for 5 hours. The mixture is cooled to room temperature and washed with ether/hexane 1:1. Three phases are obtained. The lower 2 phases are removed and the water is removed under reduced pressure to afford cyclohexylmethylphosphonous acid as a white solid; ¹H-NMR (CDCl₃): δ(ppm) = 7.40 (1H, broad singlet, exchanges D₂O, P-OH), 7.12 (1H, d, J = 526Hz, H-P), 2.10-1.75 (7H, m, 3 x CH₂ + CH), 1.48-1.05 (6H, m, 3 x CH₃).

A tetrahydrofuran solution of 19.3 g of cyclohexylmethylphosphonous acid is cooled to 5°, at which temperature a suspension forms, and treated with 12.14 g of triethylamine. An exothermia ensues and the suspension is re-cooled to 5° before slow addition of 13.02 g ethyl chloroformate in 20 ml of anhydrous tetrahydrofuran. An exothermic reaction occurs with gas evolution. The white suspension is stirred overnight at room temperature and subsequently filtered. The filtrate is concentrated under reduced pressure and the residue partitioned between dichloromethane and water. The organic phase is removed, dried and concentrated *in vacuo* to give a yellow oil. Distillation in high vacuum affords ethyl (cyclohexylmethyl)phosphinate; b.p. 120-130° (2 x 10⁻² mbar); ¹H-NMR (CDCl₃): δ(ppm) = 7.17 (1H, d, J = 527 Hz, P-H), 4.13 (2H, m, CH₂OP), 1.95-1.58 (7H, m, 3 x CH₂ + CH), 1.38-0.75 (9H, m, 3 x CH₂ + CH₃).

A suspension of 2.25 g of sodium hydride (55 % dispersion in oil) in 10 ml of anhydrous tetrahydrofuran is cooled to 0° under an inert atmosphere. A solution of 8.9 g of ethyl (cyclohexylmethyl)phosphinate in 10 ml of anhydrous tetrahydrofuran is added dropwise maintaining the temperature at between 0-5°. After the addition is complete the suspension is warmed to room temperature and stirred for 30 minutes before re-cooling to 0°C. A solution of 19.87 g of methyl iodide in 10 ml of anhydrous tetrahydrofuran is added slowly - very exothermic ! After warming to room temperature, the reaction mixture is stirred for 45 minutes, re-cooled to 0° and carefully treated with water. Removal of the organic phase, drying and concentration affords a yellow oil. Chromatography on silica gel gives ethyl P-cyclohexylmethyl-P-methylphosphinate as a colourless oil; ¹H-NMR (CDCl₃): δ(ppm) = 4.04 (2H, m, CH₂OP), 1.94-1.57 (7H, m, 3 x CH₂ + CH), 1.45 (3H, d, J = 15 Hz, P-CH₃), 1.36-0.95 (9H, m, 3 x CH₂ + CH₃).

A solution of 4.14 g lithium diisopropylamide in 60 ml of anhydrous tetrahydrofuran is cooled to -73°C under an inert atmosphere. With constant stirring a -78° solution of 7.9 g of ethyl P-cyclohexylmethyl-P-methylphosphinate in 20 ml of anhydrous tetrahydrofuran is added over 15 minutes under a positive pressure of an inert atmosphere via a canula. The resulting pale yellow solution is stirred for 1 hour at -78° and a pre-cooled (-78°) solution of 1.22 g N-tert.-butoxycarbonylglycine methyl ester in 30 ml of anhydrous tetrahydrofuran is added over 5 minutes via a canula. Chromatographic analysis after 15 minutes stirring at -78° indicates the reaction to be complete. At -78° 2.21 g glacial acetic acid is added and the mixture allowed to warm to room temperature. The reaction is diluted with dichloromethane and washed with water. Drying of the solvent and removal under reduced pressure affords a pale yellow oil.

Removal of the starting material by distillation and chromatography of the residue on silica-gel gives ethyl 3-(N-tert.-butoxycarbonylamino)-2-oxo-propyl(cyclohexylmethyl)phosphinate as a pale yellow oil; ¹H-NMR (CDCl₃): δ(ppm) = 5.5 (1H, br s, exch. D₂O, NH), 4.10 (4H, m, CH₂OP + CH₂N), 3.10 (2H, AB_{q}, d, CH₂C=O), 1.95-1.58 (7H, m, 3 x CH₂ + CH), 1.44 (9H, s, tBu), 1.38-0.95 (9H, m, 3 x CH₂ + CH₃).

Example 5: A solution of 0.73 g of sodium 3-(N-tert.-butoxycarbonylamino)-2-hydroxy-2-methyl-propyl(n-butyl)phosphinate is dissolved in 10 ml of 1.0 aqueous hydrochloric acid and the solution stirred at 20° for 16-20 hours. After this time the solution is washed with dichloromethane followed by ether and the water removed under reduced pressure at 40° to afford an oily solid. This solid is treated with n-propanol and a few grams of charcoal. Subsequent heating to reflux and filtration gives a colourless solution. Removal of the solvent *in vacuo* and drying of the solid in vacuo at 80° gives a pale yellow solid. Dissolution of this solid in absolute ethanol followed by treatment with propylene oxide affords a white solid. Recrystallisation from an ethanol/acetone mixture gives 3-amino-2-hydroxy-2-methyl-propyl(n-butyl)phosphinic acid of m.p. 187-189°.

The starting materials may be obtained as follows:
A solution of 13.44 g of methallylamine hydrochloride and 27.3 g of di-tert.-butyl carbonate in 250 ml of dichloromethane at 20° is treated with 25.2 g of triethylamine. The solution is stirred for 1 hour at 20° washed with water and the organic phase is dried and the solvent is removed to give a colourless oil. Flash-chromatography on silica-gel affords pure 3-(N-tert.-butoxycarbonylamino)-2-methyl-prop-2-ene as a colourless oil; ¹H-NMR (CDCl₃): δ(ppm) = 4.83 (2H, m, 2 x CH = C), 4.70-4.60 (1H, broad s, exch. D₂O, NH), 3.67 (2H, s, CH₂N), 1.73 (3H, s, CH₃), 1.45 (9H, s, tBu).

A solution of 27.18 g of m-chloroperbenzoic acid in chloroform is cooled to circa 10° under argon and treated, by dropwise addition, with a chloroform solution of 17.1 g of 3-(N-tert.-butoxycarbonylamino)-2-methyl-prop-2-ene over a period of 1 hour maintaining the temperature below 15° with external cooling. About 30 minutes after the addition is complete and a white precipitate begins to form. Completeness of the reaction can be judged by chromatographic analysis. When the reaction is complete, the suspension is diluted with chloroform and washed with 3 x 300 ml of a 10 % aqueous solution of sodium bisulphite followed by 3 x 300 ml of of a 10 % aqueous solution of sodium bicarbonate. Drying of the organic layer and removal of the solvent *in vacuo* afforded 3-(N-tert.-butoxycarbonylamino)-2-methyl-2,3-epoxypropane as a colourless oil; ¹H-NMR (CDCl₃): δ(ppm) = 3.3 (2H, t, CH₂N), 2.65 (2H, AB_{q}, CH₂) 1.43 (9H, s, tBu), 1.33 (3H, s, CH₃).

A solution of 15.0 g of ethyl n-butylphosphinate in anhydrous tetrahydrofuran containing 11.11 g of triethylamine is treated under an inert gas with an anhydrous tetrahydrofuran solution of 11.95 g of trimethylchlorosilane. A white precipitate forms immediately and the resulting suspension is stirred for 24 hours at room temperature under an inert gas. The reaction mixture is then filtered under an inert gas and the filtrate concentrated *in vacuo* to afford a cloudy oil which is then treated with 3.76 g of 3-(N-tert.-butoxycarbonylamino)-2-methyl-2,3-epoxypropane and 1 g of anhydrous zinc chloride. An exothermic reaction results. After the exotherm has subsided the clear solution is heated slowly to 70° and stirred at this temperature for 4 hours, after which time chromatographic analysis indicates the reaction to be complete. The reaction mixture is cooled to room temperature, diluted with dichloromethane and washed with water. Drying of the solvent and removal *in vacuo* affords an oil. The excess starting material is removed by Kugelrohr-distillation in high vacuum and the pale yellow residue chromatographed on silica-gel to give ethyl 3-(N-tert.-butoxycarbonylamino)-2-hydroxy-2-methyl-propyl-(n-butyl)phosphinate as a colourless oil; ¹H-NMR (CDCl₃): δ(ppm) = 5.17 + 4.68 (1H, Exch. D₂O, NH), 4.07 (2H, m, CH₂OP), 3.18 (2H, m, CH₂N), 1.93-1.22 (24H, m, becomes 23H on D₂O exchange), 0.92 (3H, t, CH₃). ³¹P-NMR (CDCl₃); 59.6, 59.3.

A solution of 0.86 g of ethyl 3-(N-tert-butoxycarbonylamino)-2-hydroxy-2-methylpropyl-(n-butyl)phosphinate in 10 ml of ethanol is treated with a solution of 0.4 g of sodium hydroxide in 3 ml of water and the resulting solution is heated to 60° for 24 hours. After this time the reaction is cooled to 20° and the solvent removed. The residue is partitioned between dichloromethane and water and the aqueous phase is removed and concentrated *in vacuo*. This residue is dissolved in hot n-propanol and filtered through celite. After removal of the n-propanol sodium 3-(N-tert.-butoxycarbonylamino)-2-hydroxy-2-methyl-propyl(n-butyl)phosphinate is obtained as a pale yellow hygroscopic solid which can be used without any further purification.

Example 6: A solution of 0.64 g of ethyl 3-(N-tert-butoxycarbonylamino)-2-oxopropyl(cyclopropylmethyl)phosphinate in 10 ml of anhydrous dichloromethane under an inert atmosphere at room temperature is treated with 1.53 g of trimethylbromosilane. The pale yellow solution is stirred for 4 hours at room temperature and the volatile materials are removed under reduced pressure to give a pale yellow oil. This oil is redissolved in methanol containing 1 % water and the clear pale yellow solution is stirred for 30 minutes at room temperature. The solvent is removed and the residue is dried in high vacuum at 50°C for 24 hours to afford 3-amino-2-oxopropyl(cyclopropylmethyl)phosphinic acid hydrobromide salt as a yellow gum. This gum is dissolved in ethanol and treated with propylene oxide to afford, after filtration and drying 3-amino-2-oxo-propyl(cyclopropylmethyl)phosphinic acid as a pale yellow solid of m.p. 109-110°C; ¹H-NMR (D₂O): δ(ppm) = 4.15 (2H, s, CH₂N), 3.18 (2H, d, J = 15 Hz, CH₂P), 1.58 (2H, d, d, J = 15 + 6 Hz, PCH₂), 0.83 (1H, m, CH), 0.55 (2H, m, CH₂), 0.17 (2H, m, CH₂).

The starting material may be obtained as follows:
A suspension of 2.22 g of sodium hydride (80 % in oil) in 50 ml of anhydrous tetrahydrofuran under an inert atmosphere at 20° is treated with 50 ml of an anhydrous tetrahydrofuran solution of 7.24 g of ethyl (methyl)phosphinate at such a rate so that the temperature does not exceed 25°. After the addition is complete the resulting suspension is stirred for 1 hour at room temperature before slow addition of 10.0 g of bromomethylcyclopropane. The reaction mixture is stirred for a further 3 hours at 20° cooled in an ice/water bath and 100 ml of water added carefully. The clear solution is then partitioned between dichloromethane and water. Separation of the organic layer followed by drying and removal of the solvent affords a pale yellow oil. Distillation under high vacuum affords ethyl cyclopropylmethyl(methyl)phosphinate of b.p. 100°C (10⁻¹ mbar); ¹H-NMR (CDCl₃): δ(ppm) = 4.04 (2H, m, CH₂OP) 1.66 (2H, d, CH₂P), 1.47 (3H, d, CH₃), 1.30 (3H, t, CH₃) 0.88 (1H, m, CH), 0.55 (2H, m, CH₂), 0.15 (2H, m, CH₂).

A solution of 6.97 g of lithium diisopropylamide in 100 ml of anhydrous tetrahydrofuran is cooled to -78° under an inert atmosphere. With constant stirring, a -78° solution of 10.5 g of ethyl cyclopropylmethyl(methyl)phosphinate in 30 ml of absolute tetrahydrofuran is added over 15-20 minutes via a canula under a positive pressure of inert gas. The resulting pale yellow solution is stirred for 1 hour at -78° and a pre-cooled (-78°) solution of 2.06 g of N-tert.-butoxycarbonyl glycine methyl ester in 20 ml absolute tetrahydrofuran is added over 5-10 minutes via a canula. Chromatographic analysis after 15 minutes stirring at 78°C indicated the reaction to be complete. At -78° 3.75 ml of glacial acetic acid is added and the mixture allowed to warm to room temperature. The reaction mixture is diluted with dichloromethane and washed with water. Drying of the solvent and removal under reduced pressure affords a pale yellow oil. Removal of the excess starting material by distillation and chromatography of the residue on silica gel gives ethyl 3-(N-tert.-butoxycarbonylamino)-2-oxo-propyl(cyclopropylmethyl)phosphinate as a pale yellow oil; ¹H-NMR (CDCl₃): δ(ppm) = 5.40 (1H, Br t, Exch. D₂O, NH), 4.14 (4H, m, CH₂OP + CH₂N) 3.17 (2H, d, J = 17.5 Hz, CH₂C=O), 1.93-1.68 (2H, m, PCH₂), 1.45 (9H, s, tBu), 1.35 (3H, t, CH₃), 0.93 (1H, m, CH), 0.62 (2H, m, CH₂), 0.27 (2H, m CH₂).

Example 7: A solution of 0.3 g ethyl 3-(N-tert.butoxycarbonylamino)-2-(4-chlorophenyl)-2-hydroxy-propyl(n-butyl)phospinate in 5 ml of anhydrous dichloromethane is treated with 0.53 g of trimethylbromosilane and the resulting colourless solution is stirred for 24 hours at room temperature under an inert atmosphere. The volatile materials are removed under reduced pressure to give an off-white foam, which is redissolved in 5 ml of methanol containing 1 % of water and the solution is stirred for 20 hours at room temperature. After this time, evaporation of the solvent in vacuo followed by drying of the resulting solid in high vacuum gives 3-amino-3-(4-chlorophenyl)-2-hydroxypropyl-n-butyl phosphinic acid hydrobromide. Dissolution of this salt in ethanol and treatment with propylene oxide gives 3-amino-2-(4-chlorophenyl)-2-hydroxy-propyl-(n-butyl)phosphinic acid of m.p. 215-216.5°; ¹H-NMR (D₂O): δ(ppm) = 7.32 (4H, m, Ph), 3.26 (2H, s, CH₂N), 2.40 (2H, m, CH₂P), 1.07 (6H, m, 3 x CH₂), 0.88 (3H, t, CH₃).

The starting material may be prepared as follows:
A solution of 218 g of propa-1,2-dienylphosphinic acid in 900 ml of anhydrous dichloromethane is cooled to 10° under an inert atmosphere and treated with 167.5 g of triethylamine. A slight exothermia results, and the mixture is re-cooled to 10° before dropwise addition of 180 g of ethyl chloroformate dissolved in 200 ml of dichloromethane over 130 minutes maintaining the temperature at between 10 and 15°. Gas evolution results and a white precipitate is formed. The suspension is stirred overnight and filtered. The solid obtained is washed with tetrahydrofuran and the combined washings and filtrate are washed with water. The organic phase are combined, and dried, and the solvent is removed under reduced pressure. Distillation in high vacuum affords ethyl propa-1,2-dienyl phosphinate of b.p. 47-50° (6 x 10⁻³ mbar); ¹H-NMR (CDCl₃): δ(ppm) = 7.21 (1H, d, d, J = 576 + 477 P-H), 5.43 (1H, t, d, d, CH), 5.10 (2H, d, CH₂), 4.14 (2H, m, CH₂OP), 1.36 (3H, t, CH₃).

A solution of 41.25 g of ethyl propa-1,2-dienyl phosphinate in 100 ml of triethyl orthoacetate is treated with 1 g of boron trifluoride diethyl etherate. After 3 hours at room temperature the solution is diluted with dichloromethane and washed with a 10 % aqueous sodium bicarbonate solution. The organic phase is dried and the volatile materials are removed under reduced pressure. Distillation of the residue in high vacuum affords ethyl 1,1-diethoxyethyl-(propa-1,2-dienyl)phosphinate of b.p. 89-125° (10⁻³ mbar) as a colourless oil. ¹H-NMR (CDCl₃): δ(ppm) = 5.44 (1H, d, d, CH), 5.02 (2H, d, d, CH₂), 4.22 (2H, m, CH₂OP), 3.65 (4H, m, 2 x CH₂OC), 1.53 (3H, d, J = 16 Hz, P-C-CH₃), 1.33 (3H, t, CH₃), 1.20 (6H, t, 2 x CH₃).

A solution of 4.78 g 4-chloroiodobenzene in 20 ml of dry diethyl ether is added to 0.486 g of magnesium turnings under argon so that the metal is just covered with the solvent. Reaction is initiated by gently warming and the remainder of the chloroiodobenzene ether solution is added at such a rate so as to maintain a gentle reflux. After the addition is complete the mixture is refluxed for a further 1 hour. The brown cloudy solution is then cooled to 0° and added slowly to a suspension of 4.1 g of copper(1)bromide dimethyl sulphide complex in dry ether pre-cooled to -45°. The resulting orange/yellow suspension is stirred at -45° for 1-1¹/₂ hours.

Then a chilled ether solution of 4.979 g of ethyl 1,1-dimethoxyethyl-(propa-1,2-dienyl)phosphinate is added over 30 minutes maintaining the temperature at less than or equal to -40°. The mustard coloured suspension is stirred for 2¹/₂ hours at -40° followed by 1¹/₂ hours at -20°. To the light red suspension a saturated ammonium chloride solution is added and warmed slowly to room temperature. The reaction mixture is partitioned between dichloromethane and water. The organic phase is dried and the solvent is removed in vacuo to give a semi-solid residue which is suspended in ether and filtered. Removal of the ether and chromatography of the residue on silica gel affords ethyl 1,1-diethoxyethyl-2-(4-chlorophenyl)-prop-1-enyl phosphinate as a pale yellow oil. ¹H-NMR (CDCl₃): δ(ppm) = 7.40 (4H, m, Ph), 5.53 (1H, d, CH), 5.35 (1H, d, CH), 4.06 (2H, q, CH₂OP), 3.90-3.60 (4H, m, 2 x CH₂OC), 3.06 (2H, d, J = 15 Hz, P-CH₂), 1.55 (3H, d, J = 15 Hz, P-CH₃), 1.30-1.05 (9H, t, 3 x CH₃).

A solution of 14.42 g of ethyl 1,1-diethoxyethyl-2-(4-chlorophenyl)-prop-1-enyl phosphinate in 50 ml of anhydrous dichloromethane containing 10 % absolute ethanol is treated with 6.518 g of trimethylchlorosilane. After stirring at room temperature for 24 hours the volatile material is removed under reduced pressure. Chromatography of the resulting oil on silica-gel gives ethyl 2-(4-chlorophenyl)-prop-1-enyl phosphinate as a colourless oil; ¹H-NMR (CDCl₃): δ(ppm) = 7.35 (4H, m, Ph), 7.05 (1H, d, t, J = 549 and 1.5 Hz, P-H), 5.56 (1H, d, CH), 5.30 (1H, d, CH), 4.20-3.97 (2H, q, CH₂OC), 3.07 (2H, d, J = 1.5 Hz, P-CH₂), 1.27 (3H, t, CH₃).

A tetrahydrofuran solution of 2.5 g of ethyl 2-(4-chlorophenyl)prop-1-enylphosphinate is cooled to -78° under an inert atmosphere and one equivalent of n-butyl lithium in hexane is added over 15-20 minutes so that the internal temperature remains below -70°. The pale yellow solution is stirred for additional 15 minutes at -78° and then treated with 1.95 g of n-butyl iodide maintaining the temperature at -78°. After additional 10 minutes at -78°, the reaction is quenched with a saturated aqueous solution of ammonium chloride and warmed to 0°. Dilution with dichloromethane and washing with saturated aqueous ammonium chloride solution followed by drying and removal of the solvent affords an oil. Careful chromatography on silica-gel gives ethyl (n-butyl) 2-(4-chlorophenyl)prop-1-enylphosphinate as a colourless oil; ¹H-NMR (CDCl₃): δ(ppm) = 7.38 (4H, m, Ph), 5.48 (1H, d, CH), 5.32 (1H, d, CH), 4.11-3.80 (2H, m, CH₂OP), 3.02 (2H, d, J = 16 Hz, CH₂P), 1.65 (2H, m, PCH₂), 1.51 (2H, m, CH₂), 1.33 (2H, m, CH₂), 1.20 (3H, t CH₃), 0.57 (3H, t, CH₃).

An absolute methanol solution of 2.34 g of tert.-butyl carbamate is cooled to 0° under an inert atmosphere and treated carefully with 2.17 g of tert.-butyl hypochlorite. The resulting pale yellow solution is stirred for 15 minutes at 0° and a solution of 0.84 g of sodium hydroxide in 10 ml of absolute methanol added dropwise. The cooling bath is removed and the solution stirred for 10 minutes before removal of the methanol. Trituration of the remaining slurry with ether and collection of the solid by filtration followed by drying in high vacuo affords sodium N-chloro-tert.-butylcarbamate.

An acetonitrile suspension of 0.69 g sodium N-chloro-tert.-butylcarbamate is treated with 0.675 g of silver nitrate and the resulting brown suspension treated with 0.6 g of ethyl (n-butyl) 2-(4-chlorophenyl) prop-1-enylphosphinate followed by 10.18 mg of osmium tetroxide and 180 g of water. The black suspension is stirred at room temperature for 24 hours and filtered through celite. The filtrate is treated with 20 ml of 5 % aqueous sodium sulphite and the two phase mixture heated to reflux for 2 hours. After cooling to room temperature the acetonitrile is removed in vacuo and the aqueous layer extracted with chloroform. Drying of the organic layer and removal of the solvent affords an oil. Chromatography on silica gel gives ethyl 3-N-(tert.-butyloxycarbonyl) amino-2-(4-chlorophenyl)-2-hydroxy-propyl(n-butyl)phosphinate as a white waxy solid, m.p. 85-90°; (diastereomeric mixture). ¹H-NMR (CDCl₃): δ(ppm) = 7.39 (Ph) 6.05-5.90 (exch D₂O, OH), 5.09 (exch D₂O, NH), 4.17-3.95 (CH₂OP + CHN), 4.67-4.44 (CHOP + CHN), 4.25-4.07 (CH₂N), 2.40-2.12 (CH₂P), 1.73-1.0 (tBu, 3 x CH₃ + CH₃), 0.89 (CH₃), 0.75 (CH₃).

Example 8: Analogously to the method described in Example 7 3-amino-2-(4-chlorophenyl)2-hydroxy-propyl(methyl)phosphinic acid of m.p. 219.5-220° can be obtained; ¹H-NMR (D₂O): δ(ppm) = 7.43 (4H, m, Ph), 3.36 (2H, AB₂, CH₂N), 2.66-2.35 (2H, m, CH₂P), 1.09 (3H, d, J = 14.54 Hz P-Me).

Example 9: In an analogous manner as described in Example 3, 3-amino-1,1-difluoro-propyl(n-butyl)phosphinic acid and its hydrochloride can be obtained starting from ethyl butylphosphinate by deprotonation with sodium hydride and reaction with bromodifluoromethane in tetrahydrofuran at 0° followed by reaction with n-butyllithium in tetrahydrofuran under an Argon atmosphere at -70° and subsequently with N-(p-nitrobenzoyl)aziridine and treatment of the resulting ethyl 3-(p-nitrobenzoylamino)-1,1-difluoro-propyl(n-butyl)phosphinate with boiling hydrochloric acid.

Example 10: A solution of 0.48 g of ethyl 3-(N-tert.-butoxycarbonylamino)-2-(4-chlorophenyl)-2-hydroxy-propyl(diethoxymethyl)phosphinate in 10 ml of anhydrous dichloromethane is treated with 0.76 g of trimethylbromosilane and the resulting colourless solution stirred for 24 hours at room temperature under an inert atmosphere. The volatile materials are removed under reduced pressure to give a foam which is redissolued in 5 ml of methanol containing 1 % of water and the solution stirred for 20 hours at room temperature. After this time evaporation of the solvent in vacuo followed by chromatography of the residue on reverse-phase silicagel and drying of the off white solid thus obtained in high vacuum gives 3-amino-2-(4-chlorophenyl)-2-hydroxypropyl(diethoxymethyl)phosphinic acid hydrobromide salt. Dissolution of the salt in the methanol and treatment with propylene oxide gives 3-amino-2-(4-chlorophenyl)-2-hydroxy-propyl(diethoxymethyl)phosphinic acid; ¹H-NMR (D₂O): δ(ppm) = 7.39 (4H, m, Ph), 4.75 (1H, d, CH-P), 3.67 - 3.23 (6H, m, 2x CH₂CO + CH₂N), 2.38 - 1.97 (2H, AB_{q}, CH₂P),1.23 - 1.04 (6H, t, 2x CH₃).

The starting material may be obtained as follows:
A solution of 218 g of propa-1,2-dienyl phosphinic acid in 900 ml of anhydrous dichloromethane is cooled to 10° under an inert atmosphere and treated with 167.5 g of triethylamine. A slight exotherm results and the mixture is re-cooled to 10° before dropwise addition of 180 ml of a dichloromethane solution of 180 g ethyl chloroformate over 130 minutes maintaining the temperature at between 10 and 15°, gas evolution results and a white precipitate is formed. The suspension is stirred overnight and filtered. The solid is washed with carbon tetrachloride and the combined washings and filtrate washed with water. The organic phase is dried and the solvent removed under reduced pressure. Distillation in high vacuum affords ethyl propa-1,2-dienyl phosphinate of b.p. 47-50° (6x10⁻³ mbar); ¹H-NMR (CDCl₃): δ(ppm) = 7.21 (1H, d, d, J = 576 + 4 Hz, P-H), 5.43 (1H, t, d, d, CH), 5.10 (2H, d, CH₂), 4.14 (2H, m, CH₂OC), 1.36 (3H, t, CH₃).

A solution of 41.25 g of ethyl propa-1,2-dienyl phosphinate in 100 ml of triethyl orthoacetate is treated with 1 g of boron trifluoride diethyl etherate. After 3 hours at room temperature the solution is diluted with dichloromethane and washed with 10 % aqueous sodium bicarbonate solution. The organic phase is dried and the volatile material removed under reduced pressure. Distillation of the residue in high vacuum affords ethyl (1,1-diethoxyethyl)propa-1,2-dienyl phosphinate of b.p. 80-125° (10⁻³ mbar) as a colourless oil. ¹H-NMR (CDCl₃): δ(ppm) = 5.44 (1H, d, d, CH), 5.02 (2H, d, d, CH₂) 4.22 (2H, m, CH₂OP), 3.65 (4H, m, 2 x CH₂OC), 1.53 (3H, d, J = 16Hz, P-CH₂), 1.33 (3H, t, CH₃), 1.20 (6H, t, 2 x CH₃), 1.30-1.05 (9H, t, 3 x CH₃).

A solution of 4.78 g 4-chloroiodobenzene in 20 ml of dry diethyl ether is added to 0.486 g of magnesium turnings under argon so that the metal is just covered with the solvent. Reaction is initiated by gently warming and the remainder of the chloroiodobenzene ether solution is added at such a rate so as to maintain a gentle reflux. After the addition is complete the mixture is refluxed for a further 1 hour. The brown cloudy solution is then cooled to 0° and added slowly to a suspension of 4.1 g of copper(1)bromide dimethyl sulphide complex in dry ether pre-cooled to -45°. The resulting orange/yellow suspension is stirred at -45° for 1-1¹/₂ hours before addition of a chilled ether solution of 4.97 g of ethyl (1,1-diethoxyethyl)propa-1,2-dienyl phosphinate over 30 minutes maintaining the temperature at less than as equal to -40°. The mustard coloured suspension is stirred for 2¹/₂ hours at -40° followed by 1¹/₂ hours at -20°. To the light red suspension is added saturated ammonium chloride solution and warmed slowly to room temperature. The reaction is partitioned between dichloromethane and water. The organic phase is dried and the solvent removed in vacuo to give a semi-solid residue which is suspended in ether and filtered. Removal of the ether and chromatography of the residue on silicagel affords ethyl 2-(4-chlorophenyl)(1,1-diethoxyethyl)prop-1-eny l phosphinate as a pale yellow oil. ¹H-NMR (CDCl₃): δ(ppm) = 7.40 (4H, m, PH), 5.53 (1H, d, CH) 5.35 (1H, d, CH), 4.06 (2H, q, CH₂OP), 3.90-3.60 (4H, m, 2 x CH₂OC), 3.06 (2H, d, J = 15 Hz, P-CH₂), 1.55 (3H, d, J= 15 Hz, P-CH₃).

A solution of 14.42 g of ethyl (1,1-diethoxyethyl)-2-(4-chlorophenyl)prop-1-enylphosphinate in 50 ml of anhydrous dichloromethane containing 10 % of absolute ethanol is treated with 6.518 g of trimethylchlorosilane. After stirring at room temperature for 24 hours the volatile material is removed under reduced pressure. Chromatography of the resulting oil on silica-gel gives ethyl 2-(4-chlorophenyl)prop-1-enylphosphinate as a colourless oil; ¹H-NMR (CDCl₃): δ(ppm) = 7.35 (4H, m, Ph), 7.05 (1H, d, t, J = 549 and 1.5 Hz, P-H), 5.56 (1H, d, CH), 5.30 (1H, d, CH), 4.20-3.97 (2H, q, CH₂OP), 3.07 (2H, d, t, J = 17 + 1.5 Hz, P-CH₂), 1.27 (3H, t, CH₃).

A solution of 1.68 g of ethyl 2-(4-chlorophenyl)prop-1-enylphosphinate in 20 ml of triethyl orthoformate is treated with 0.1 g of boron trifluoride diethyl etherate and the resulting solution stirred for 7 days at room temperature. The reaction mixture is then treated with 20 ml 10 % aqueous sodium hydrogen carbonate solution and extracted twice with dichloromethane. The organic layer is removed, dried and concentrated to give an oil. Chromatography on silica gel gives ethyl 2-(4-chlorophenyl)prop-1-enyl-(diethoxymethyl)phosphinate as a colourless oil. ¹H-NMR (CDCl₃): δ(ppm) = 7.38 (4H, m; Ph), 5.51 (1H, d, CH), 4.62 (1H, d, CH-P), 4.05 (2H, q, CH₂OP), 3.88 - 3.56 (CH, m, 2x CH₂OC), 2.07 (2H, d,d, CH₂P), 1.28 - 1.14 (9H, m, 3x CH₃).

Reaction of ethyl 2-(4-chlorophenyl)prop-1-enyl(diethoxymethyl)phosphinate with tertiary butyl N-chloro-N-iodo-carbamate in the presence of osmium tetroxide in an analogous manner as described in Example 7 gives ethyl 3-(N-tert.-butoxycarbonylamino)-2-(4-chlorophenyl)-2-hydroxy-propyl(diethoxymethyl)phosphinate as a colourless oil; ¹H-NMR (CDCl₃): δ(ppm) = 7.46 - 7.26 (4H, m, Ph), 5.66 + 5.22 (1H, exchange with D₂O, OH), 5.07 (1H, exchange with D₂O, NH), 4.52 (1H, d, CH-P), 4.18 (2H, m, CH₂OP, diastereomer A), 3.9 - 3.05 (8H, m, 2x CH₂OC, CH₂N, CH₂OP), 2.59 - 2.20 (2H, AB_{q}, CH₂P), 1.37 (9H, s, tert.-butyl), 1.33 - 0.90 (9H, m, 3x CH₃).

Example 11: In a manner analogous to the method described in Example 7, 3-amino-2-(4-chlorophenyl)-2-hydroxy-propyl(cyclohexylmethyl)phosphinic acid can be manufactured.

Example 12: In a manner analogous to the method described in Example 7, 3-amino-2-(4-chlorophenyl)-2-hydroxy-propyl(benzyl)phosphinic acid can be manufactured.

Example 13: In a manner analogous to the method described in Example 7, 3-amino-2-(4-chlorophenyl)-2-hydroxy-propyl(cyclopropylmethyl)phosphinic acid can be manufactured.

Example 14: In a manner analogous to the method described in Example 6, 3-amino-2-(4-chlorophenyl)-2-oxo-propyl(benzyl)phosphinic acid can be manufactured.

Example 15: In a manner analogous to the method described in Example 6, 3-amino-2-(4-chlorophenyl)-2-oxo-propyl(diethoxymethyl)phosphinic acid can be manufactured.

Example 16: Preparation of 10,000 tablets each containing 100 mg of the active ingredient, for example, 3-amino-2-oxo-propyl(n-butyl)phosphinic acid, can be manufactured as follows:

| Composition | |
|---|---|
| Active ingredient | 1,000.00 g |
| Lactose | 257.00 g |
| Corn starch | 75.00 g |
| Polyethylene glycol 6,000 | 75.00 g |
| Magnesium stearate | 18.00 g |
| Purified water | q.s. |

Procedure: All the powders are passed through a screen with openings of 0.6 mm. Then the drug substance, lactose, magnesium stearate and half of the starch are mixed in a suitable mixer. The other half of the starch is suspended in 40 ml of water and the suspension added to the boiling solution of the polyethylene glycol in 150 ml of water. The paste formed is added to the powders which are granulated, if necessary, with an additional amount of water. The granulate is dried overnight at 35°, broken on a screen with 1,2 mm openings and compressed into tablets with 12.8 mm diameter, uppers bisected.

Example 17: Preparation of 10,000 capsules each containing 25 mg of the active ingredient, for example, 3-amino-2-oxo-propyl(n-butyl)phosphinic acid, can be manufactured as follows:

| Composition | |
|---|---|
| Active ingredient | 250.0 g |
| Lactose | 1,750.0 g |

Procedure: All the powders are passed through a screen with openings of 0.6 mm. Then the drug substance is placed in a suitable mixer and mixed with the lactose until homogenous. No. 3 capsules are filled with 200 mg using a capsule filling machine.

Example 18: In a manner analogous to that described in Examples 16 and 17 tablets and capsules comprising as the active ingredients 10 - 100 mg of another compounds of the invention, e.g. as described in the Examples 1 to 15.

## Claims (Claims for the following Contracting State(s): AT, BE, CH, DE, DK, FR, GB, IT, LI, LU, NL, SE)

1. A compound of the formula I 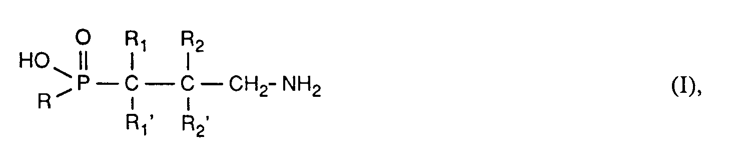 wherein either R₁ is halogen, R₁' is halogen or hydrogen and R₂ and R₂' denote hydrogen or R₁ and R₁' represent hydrogen, R₂ is an aliphatic or aromatic radical and R₂' is hydroxy or R₂ and R₂' together represent oxo, and wherein R denotes an aliphatic, cycloaliphatic, cycloaliphatic-aliphatic or araliphatic radical having 2 or more carbon atoms or, if R₁ and R₁' denote hydrogen, R₂ represents an aromatic radical and R₂' is hydroxy, R represents methyl, or a salt thereof.

2. A compound according to claim 1, of the formula I, wherein either R₁ is halogen, R₁' is halogen or hydrogen and R₂ and R₂' denote hydrogen or R₁ and R₁' represent hydrogen, R₂ is an aromatic radical and R₂' is hydroxy or R₂ and R₂' together represent oxo, or wherein R denotes an aliphatic, cycloaliphatic, cycloaliphatic-aliphatic or araliphatic radical having 2 or more carbon atoms, or a salt thereof.

3. A compound as claimed in claim 1, of the formula I, wherein R₁ is halogen, R₁' is halogen or hydrogen and R₂ and R₂' are hydrogen or R₁ and R₁' are hydrogen, R₂ denotes lower alkyl, phenyl, phenyl mono- or disubstituted by halogen, lower alkyl, lower alkoxy and/or trifluoromethyl or pyridyl and R₂' is hydroxy or R₂ and R₂' together represent oxo, and wherein R denotes lower alkyl having 2 or more carbon atoms, lower alkenyl, lower alkynyl, a cycloalkyl, hydroxycycloalkyl, cycloalkyl-lower alkyl, cycloalkyl-(hydroxy)lower alkyl or lower alkylthiocycloalkyl-(hydroxy)-lower alkyl group having 3 to 6 ring carbon atoms, oxo-lower alkyl, amino-lower alkyl, lower alkanoylamino-lower alkyl, phthalimido-lower alkyl, mono- or dihydroxy-lower alkyl, hydroxy-lower alkenyl, amino-(hydroxy)lower alkyl, lower alkanoylamino-(hydroxy)lower alkyl, phthalimido-(hydroxy)lower alkyl, mono-, di- or polyhalogeno-lower alkyl, mono-, di- or polyhalogeno-lower alkenyl, mono-, di- or polyhalogeno-(hydroxy)lower alkyl, mono-, di- or polyhalogeno-(hydroxy)lower alkenyl, amino lower alkoxy-lower alkyl, lower alkylthio-lower alkyl, lower alkanesulphinyl-lower alkyl, lower alkanesulphonyl-lower alkyl, di-lower alkoxy-lower alkyl, di-lower alkylthio-lower alkyl, lower alkoxy-(hydroxy)lower alkyl, lower alkoxy-(halogeno)lower alkyl, phenyl-lower alkyl, phenyl-lower alkyl mono- or disubstituted, in the phenyl moiety, by halogen, lower alkyl, lower alkoxy and/or trifluoromethyl, naphthyl-lower alkyl, oxa- or thiacycloalkyl having 2 to 6 ring carbon atoms, or dioxa-, oxathia- or dithiacycloalkyl having 3 to 5 ring carbon atoms or, if R₁ and R₁' denote hydrogen, R₂ represents phenyl, phenyl mono- or disubstituted by halogen, lower alkyl, lower alkoxy and/or trifluoromethyl or pyridyl and R₂' is hydroxy, R represents methyl, or a salt thereof.

4. A compound as claimed in claim 2, of the formula I, wherein R, is halogen, R₁' is halogen or hydrogen and R₂ and R₂' are hydrogen or R₁ and R₁' are hydrogen, R₂ denotes phenyl or phenyl mono- or disubstituted by halogen, lower alkyl, lower alkoxy and/or trifluoromethyl and R₂' is hydroxy or R₂ and R₂' together represent oxo, and wherein R denotes lower alkyl having 2 or more carbon atoms, lower alkenyl, lower alkynyl, a cycloalkyl, hydroxycycloalkyl, cycloalkyl-lower alkyl, cycloalkyl-(hydroxy)lower alkyl or lower alkylthiocycloalkyl-(hydroxy)-lower alkyl group having 3 to 6 ring carbon atoms, oxo-lower alkyl, amino-lower alkyl, lower alkanoylamino-lower alkyl, phthalimido-lower alkyl, mono- or dihydroxy-lower alkyl, hydroxy-lower alkenyl, amino-(hydroxy)lower alkyl, lower alkanoylamino-(hydroxy)lower alkyl, phthalimido-(hydroxy)lower alkyl, mono-, di- or polyhalogeno-lower alkyl, mono-, di- or polyhalogeno-lower alkenyl, mono-, di- or polyhalogeno-(hydroxy)lower alkyl, mono-, di- or polyhalogeno-(hydroxy)lower alkenyl, amino lower alkoxy-lower alkyl, lower alkylthio-lower alkyl, lower alkanesulphinyl-lower alkyl, lower alkanesulphonyl-lower alkyl, di-lower alkoxy-lower alkyl, di-lower alkylthio-lower alkyl, lower alkoxy-(hydroxy)lower alkyl, lower alkoxy-(halogeno)lower alkyl, phenyl-lower alkyl, phenyl-lower alkyl mono- or disubstituted, in the phenyl moiety, by halogen, lower alkyl, lower alkoxy and/or trifluoromethyl, naphthyl-lower alkyl, oxa- or thiacycloalkyl having 2 to 6 ring carbon atoms, or dioxa-, oxathia- or dithiacycloalkyl having 3 to 5 ring carbon atoms, or a salt thereof.

5. A compound as claimed in claim 2, of the formula I, wherein either R₁ and R₁' are fluoro and R₂ and R₂' represent hydrogen or R₁ and R₁' are hydrogen, R₂ is phenyl, phenyl substituted by halogen, C₁-C₄alkyl, C₁-C₄alkoxy and/or trifluoromethyl and R₂' is hydroxy or R₁ and R₁' are hydrogen and R₂ and R₂' together represent oxo, and wherein R is C₂-C₁₂-alkyl, C₂-C₇-alkenyl, C₂-C₇-alkynyl, mono- or dihydroxy-C₂-C₇-alkyl, oxo-C₃-C₇alkyl, amino-C₃-C₆alkyl, phthalimido-C₃-C₆alkyl or phthalimido-C₃-C₇(hydroxy)alkyl, or a salt thereof.

6. A compound as claimed in claim 2, of the formula I, wherein R₁ and R₁' are fluoro and R₂ and R₂' are hydrogen, or R₁ and R₁' are hydrogen and R₂ and R₂' together represent oxo, and wherein R denotes C₂-C₇-alkyl, α-saturated C₃-C₇-alkenyl, α-saturated C₃-C₇-alkynyl, α-, β-, γ- or δ-hydroxy-C₂-C₇-alkyl, α,α-difluoro-C₂-C₄-alkyl, mono-, di-or trifluoro-α-hydroxy-C₃-C₇-alkyl, mono-, di- or trihalogeno-α-hydroxy-C₃-C₇-alkenyl, C₁-C₄-alkoxy-C₁-C₄-alkyl, di-C₁-C₄-alkoxy-C₁-C₄-alkyl, C₃-C₆-cycloalkyl-C₁-C₄-alkyl, α-hydroxy-C₃-C₆-cycloalkyl or C₃-C₆-cycloalkyl-α-hydroxy-C₁-C₄-alkyl, or a salt thereof.

7. A compound as claimed in claim 1, of the formula I, wherein R₁ and R₁' are hydrogen, R₂ is phenyl, phenyl substituted by halogen, C₁-C₄alkyl, C₁-C₄alkoxy and/or trifluoromethyl and R₂' is hydroxy, and wherein R represents C₁-C₇alkyl, or a salt thereof.

8. A compound as claimed in any one of claims 1 to 7, wherein R denotes C₃-C₄alkyl or, if applicable, methyl, or a salt thereof.

9. A compound as claimed in claim 2, wherein R is C₂-C₇-alkyl, and wherein R₁ and R₁' are fluoro and R₂ and R₂' are hydrogen, or R₁ and R₁' are hydrogen and R₂ and R₂' together represent oxo, or a salt thereof.

10. 3-Amino-2-oxo-propyl(n-butyl)phosphinic acid or a salt thereof.

11. 3-Amino-2-(4-chlorophenyl)-2-hydroxy-propyl(n-butyl)phosphinic acid or a salt thereof.

12. 3-Amino-1,1-difluoro-propyl(n-butyl)phosphinic acid or a salt thereof.

13. 3-Amino-2-oxo-propyl(cyclopropylmethyl)phosphinic acid or a salt thereof.

14. 3-Amino-2-oxo-propyl(cyclohexylmethyl)phosphinic acid or a salt thereof.

15. 3-Amino-2-hydroxy-2-methyl-propyl(n-butyl)phosphinic acid or a salt thereof.

16. 3-Amino-1-fluoro-propyl(n-butyl)phosphinic acid or a salt thereof.

17. 3-Amino-2-(4-chlorophenyl)-2-hydroxy-propyl(cyclohexylmethyl)phosphinic acid or a salt thereof.

18. 3-Amino-2-(4-chlorophenyl)-2-hydroxy-propyl(benzyl)phosphinic acid or a salt thereof.

19. 3-Amino-2-(4-chlorophenyl)-2-hydroxy-propyl(cyclopropylmethyl)phosphinic acid or a salt thereof.

20. 3-Amino-2-(4-chlorophenyl)-2-hydroxy-propyl(cyclopropylmethyl)phosphinic acid or a salt thereof.

21. 3-Amino-2-(4-chlorophenyl)-2-oxo-propyl(diethoxymethyl)phosphinic acid or a salt thereof.

22. 3-Amino-2-(4-chlorophenyl)-2-hydroxy-propyl(diethoxymethyl)phosphinic acid or a salt thereof.

23. 3-Amino-2-(4-chlorophenyl)-2-hydroxy-propyl(methyl)phosphinic acid or a salt thereof.

24. A compound as claimed in any one of claims 2, 4 to 6 and 9 to 13 for use in a method for the therapeutic treatment of the human or animal body.

25. A compound as claimed in any one of claims 1, 3, 7, 8 and 14 to 23 for use in a method for the therapeutic treatment of the human or animal body.

26. A pharmaceutical composition which contains at least one compound as claimed in any one of claims 2, 4 to 6, 9 to 13 and 24 in admixture to conventional pharmaceutical excipients.

27. A pharmaceutical composition which contains at least one compound as claimed in any one of claims 1, 3, 7, 8, 14 to 23 and 25 in admixture to conventional pharmaceutical excipients.

28. A process for the manufacture of compounds of the formula I 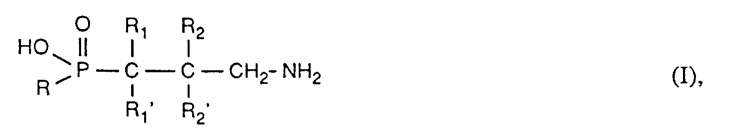 wherein either R₁ is halogen, R₁' is halogen or hydrogen and R₂ and R₂' denote hydrogen or R₁ and R₁' represent hydrogen, R₂ is an aliphatic or aromatic radical and R₂' is hydroxy or R₂ and R₂' together represent oxo, and wherein R denotes an aliphatic, cycloaliphatic, cycloaliphatic-aliphatic or araliphatic radical having 2 or more carbon atoms or, if R₁ and R₁' denote hydrogen, R₂ represents an aromatic radical and R₂' is hydroxy, R represents methyl, and of their salts, characterized in that
a) in a compound of formula II 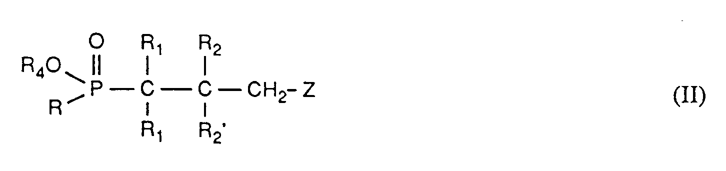 in which R, R₁, R₁', R₂ and R₂' have their previous significances, Z represents a protected or latent amino group Z₀ and R₄ denotes hydrogen or a hydroxy-protective group R₅, and wherein amino as a constituent of R and/or hydroxy R₂' or oxo R₂ + R₂' may be present in a temporarily protected form, any group R₅ or R₆ is replaced by hydrogen and/or any group Z₀ is converted into -NH₂; or
b) in a compound of the formula III 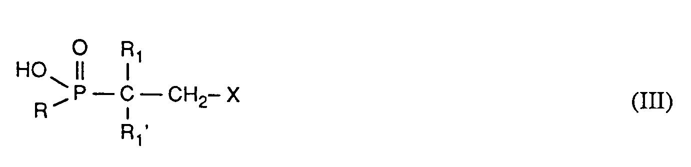 in which R₁ and R₁' have their previous significances and X is a group capable of being converted into a group of formula -CH₂NH₂ (Ia), the group X is converted into a group of formula Ia; or
c) a compound of formula IV 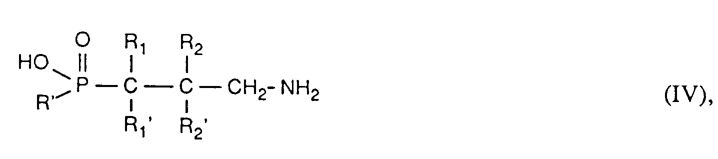 wherein R' may be selected from lower alkenyl, lower alkadienyl or lower alkynyl, to produce a compound of formula I, wherein R is lower alkyl, or from phenyl to produce a compound of formula I, wherein R is cyclohexyl, is reduced, and, if desired, a resulting salt obtained in this process may be converted into the free compound or into another salt and/or, if desired, a resulting free compound is converted into a salt to suit the above definition and/or, if desired, a resulting mixture of isomers is separated into the individual isomers.

29. Use of a compound as claimed in any one of claims 1 to 25 for the manufacture of a nootropic, antidepressant or anxiolytic medicament.

## Claims (Claims for the following Contracting State(s): ES, GR)

1. A process for the manufacture of compounds of the formula I 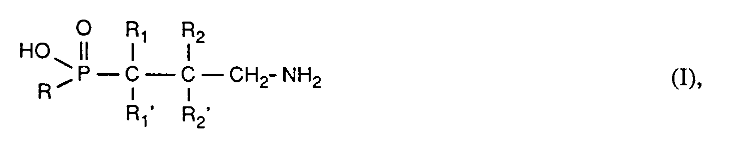 wherein either R₁ is halogen, R₁' is halogen or hydrogen and R₂ and R₂' denote hydrogen or R₁ and R₁' represent hydrogen, R₂ is an aliphatic or aromatic radical and R₂' is hydroxy or R₂ and R₂' together represent oxo, and wherein R denotes an aliphatic, cycloaliphatic, cycloaliphatic-aliphatic or araliphatic radical having 2 or more carbon atoms or, if R₁ and R₁' denote hydrogen, R₂ represents an aromatic radical and R₂' is hydroxy, R represents methyl, and of their salts, characterized in that
a) in a compound of formula II 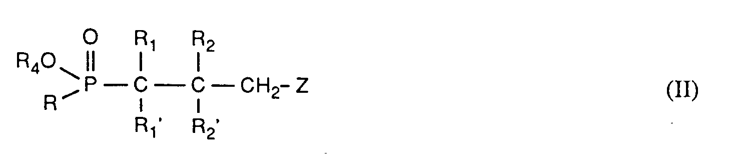 in which R, R₁, R₁', R₂ and R₂' have their previous significances, Z represents a protected or latent amino group Z₀ and R₄ denotes hydrogen or a hydroxy-protective group R₅, and wherein amino as a constituent of R and/or hydroxy R₂' or oxo R₂ + R₂' may be present in a temporarily protected form, any group R₅ or R₆ is replaced by hydrogen and/or any group Z₀ is converted into -NH₂; or
b) in a compound of the formula III 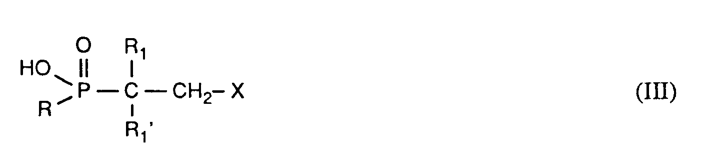 in which R₁ and R₁' have their previous significances and X is a group capable of being converted into a group of formula -CH₂NH₂ (Ia), the group X is converted into a group of formula Ia; or
c) a compound of formula IV 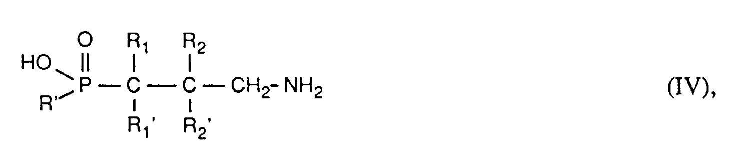 wherein R' may be selected from lower alkenyl, lower alkadienyl or lower alkynyl, to produce a compound of formula I, wherein R is lower alkyl, or from phenyl to produce a compound of formula I, wherein R is cyclohexyl, is reduced, and, if desired, a resulting salt obtained in this process may be converted into the free compound or into another salt and/or, if desired, a resulting free compound is converted into a salt to suit the above definition and/or, if desired, a resulting mixture of isomers is separated into the individual isomers.

2. A process as claimed in claim 1 for the manufacture of compounds of the formula I 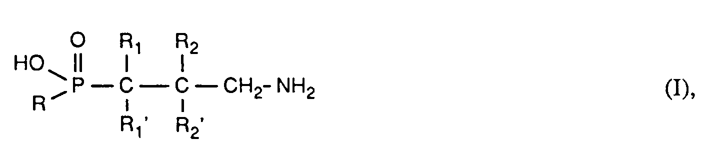 wherein either R₁ is halogen, R₁' is halogen or hydrogen and R₂ and R₂' denote hydrogen or R₁ and R₁' represent hydrogen, R₂ is an aromatic radical and R₂' is hydroxy or R₂ and R₂' together represent oxo, or wherein R denotes an aliphatic, cycloaliphatic, cycloaliphatic-aliphatic or araliphatic radical having 2 or more carbon atoms, and of their salts, characterized in that
a) in a compound of formula II 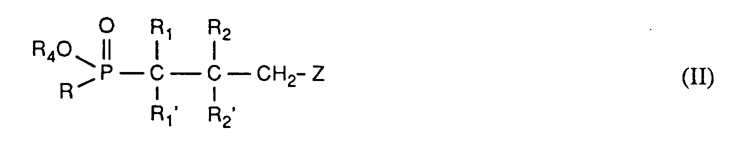 in which R, R₁, R₁', R₂ and R₂' have their previous significances, Z represents a protected or latent amino group Z₀ and R₄ denotes hydrogen or a hydroxy-protective group R₅, and wherein amino as a constituent of R and/or hydroxy R₂' or oxo R₂ + R₂' may be present in a temporarily protected form, any group R₅ is replaced by hydrogen and/or any group Z₀ is converted into -NH₂; or
b) in a compound of the formula III 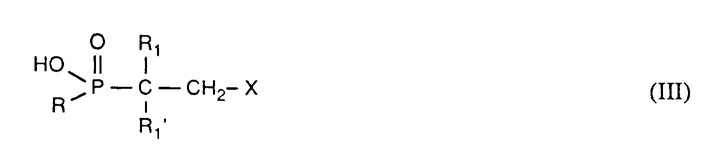 in which R₁ and R₁' have their previous significances and X is a group capable of being converted into a group of formula -CH₂NH₂ (Ia), the group X is converted into a group of formula Ia; or
c) a compound of formula IV 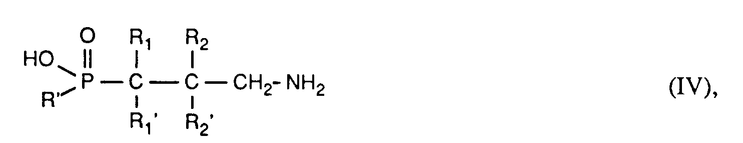 wherein R' may be selected from lower alkenyl, lower alkadienyl or lower alkynyl, to produce a compound of formula I, wherein R is lower alkyl, or from phenyl to produce a compound of formula I, wherein R is cyclohexyl, is reduced, and, if desired, a resulting salt obtained in this process may be converted into the free compound or into another salt and/or, if desired, a resulting free compound is converted into a salt to suit the above definition and/or, if desired, a resulting mixture of isomers is separated into the individual isomers.

3. A process as claimed in claim 1 for the manufacture of compounds of the formula I, wherein R₁ is halogen, R₁' is halogen or hydrogen and R₂ and R₂' are hydrogen or R₁ and R₁' are hydrogen, R₂ denotes lower alkyl, phenyl, phenyl mono- or disubstituted by halogen, lower alkyl, lower alkoxy and/or trifluoromethyl or pyridyl and R₂' is hydroxy or R₂ and R₂' together represent oxo, and wherein R denotes lower alkyl having 2 or more carbon atoms, lower alkenyl, lower alkynyl, a cycloalkyl, hydroxycycloalkyl, cycloalkyl-lower alkyl, cycloalkyl-(hydroxy)lower alkyl or lower alkylthiocycloalkyl-(hydroxy)-lower alkyl group having 3 to 6 ring carbon atoms, oxo-lower alkyl, amino-lower alkyl, lower alkanoylamino-lower alkyl, phthalimido-lower alkyl, mono- or dihydroxy-lower alkyl, hydroxy-lower alkenyl, amino-(hydroxy)lower alkyl, lower alkanoylamino-(hydroxy)lower alkyl, phthalimido-(hydroxy)lower alkyl, mono-, di- or polyhalogeno-lower alkyl, mono-, di- or polyhalogeno-lower alkenyl, mono-, di- or polyhalogeno-(hydroxy)lower alkyl, mono-, di- or polyhalogeno-(hydroxy)lower alkenyl, amino lower alkoxy-lower alkyl, lower alkylthio-lower alkyl, lower alkanesulphinyl-lower alkyl, lower alkanesulphonyl-lower alkyl, di-lower alkoxy-lower alkyl, di-lower alkylthio-lower alkyl, lower alkoxy-(hydroxy)lower alkyl, lower alkoxy-(halogeno)lower alkyl, phenyl-lower alkyl, phenyl-lower alkyl mono- or disubstituted, in the phenyl moiety, by halogen, lower alkyl, lower alkoxy and/or trifluoromethyl, naphthyl-lower alkyl, oxa- or thiacycloalkyl having 2 to 6 ring carbon atoms, or dioxa-, oxathia- or dithiacycloalkyl having 3 to 5 ring carbon atoms or, if R₁ and R₁' denote hydrogen, R₂ represents phenyl, phenyl mono- or disubstituted by halogen, lower alkyl, lower alkoxy and/or trifluoromethyl or pyridyl and R₂' is hydroxy, R represents methyl, or of a salt thereof.

4. A process as claimed in claim 2 for the manufacture of compounds of the formula I, wherein R₁ is halogen, R₁' is halogen or hydrogen and R₂ and R₂' are hydrogen or R₁ and R₁' are hydrogen, R₂ denotes phenyl or phenyl mono- or disubstituted by halogen, lower alkyl, lower alkoxy and/or trifluoromethyl and R₂' is hydroxy or R₂ and R₂' together represent oxo, and wherein R denotes lower alkyl having 2 or more carbon atoms, lower alkenyl, lower alkynyl, a cycloalkyl, hydroxycycloalkyl, cycloalkyl-lower alkyl, cycloalkyl-(hydroxy)lower alkyl or lower alkylthiocycloalkyl-(hydroxy)-lower alkyl group having 3 to 6 ring carbon atoms, oxo-lower alkyl, amino-lower alkyl, lower alkanoylamino-lower alkyl, phthalimido-lower alkyl, mono- or dihydroxy-lower alkyl, hydroxy-lower alkenyl, amino-(hydroxy)lower alkyl, lower alkanoylamino-(hydroxy)lower alkyl, phthalimido-(hydroxy)lower alkyl, mono-, di- or polyhalogeno-lower alkyl, mono-, di- or polyhalogeno-lower alkenyl, mono-, di- or polyhalogeno-(hydroxy)lower alkyl, mono-, di- or polyhalogeno-(hydroxy)lower alkenyl, amino lower alkoxy-lower alkyl, lower alkylthio-lower alkyl, lower alkanesulphinyl-lower alkyl, lower alkanesulphonyl-lower alkyl, di-lower alkoxy-lower alkyl, di-lower alkylthio-lower alkyl, lower alkoxy-(hydroxy)lower alkyl, lower alkoxy-(halogeno)lower alkyl, phenyl-lower alkyl, phenyl-lower alkyl mono- or disubstituted, in the phenyl moiety, by halogen, lower alkyl, lower alkoxy and/or trifluoromethyl, naphthyl-lower alkyl, oxa- or thiacycloalkyl having 2 to 6 ring carbon atoms, or dioxa-, oxathia- or dithiacycloalkyl having 3 to 5 ring carbon atoms, or of a salt thereof.

5. A process as claimed in claim 2 for the manufacture of compounds of the formula I, wherein either R₁ and R₁' are fluoro and R₂ and R₂' represent hydrogen or R₁ and R₁' are hydrogen, R₂ is phenyl, phenyl substituted by halogen, C₁-C₄alkyl, C₁-C₄alkoxy and/or trifluoromethyl and R₂' is hydroxy or R₁ and R₁' are hydrogen and R₂ and R₂' together represent oxo, and wherein R is C₂-C₁₂-alkyl, C₂-C₇-alkenyl, C₂-C₇-alkynyl, mono- or dihydroxy-C₂-C₇-alkyl, oxo-C₃-C₇alkyl, amino-C₃-C₆alkyl, phthalimido-C₃-C₆alkyl or phthalimido-C₃-C₇(hydroxy)alkyl, or of a salt thereof.

6. A process as claimed in claim 1 for the manufacture of compounds of the formula I, wherein R₁ and R₁' are fluoro and R₂ and R₂' are hydrogen, or R₁ and R₁' are hydrogen and R₂ and R₂' together represent oxo, and wherein R denotes C₂-C₇-alkyl, α-saturated C₃-C₇-alkenyl, α-saturated C₃-C₇-alkynyl, α-, β-, γ- or δ-hydroxy-C₂-C₇-alkyl, α,α-difluoro-C₂-C₄-alkyl, mono-, di- or trifluoro-α-hydroxy-C₃-C₇-alkyl, mono-, di- or trihalogeno-α-hydroxy-C₃-C₇-alkenyl, C₁-C₄-alkoxy-C₁-C₄-alkyl, di-C₁-C₄-alkoxy-C₁-C₄-alkyl, C₃-C₆-cycloalkyl-C₁-C₄-alkyl, α-hydroxy-C₃-C₆-cycloalkyl or C₃-C₆-cycloalkyl-α-hydroxy-C₁-C₄-alkyl, or of a salt thereof.

7. A process as claimed in claim 1 for the manufacture of compounds of the formula I, wherein R₁ and R₁' are hydrogen, R₂ is phenyl, phenyl substituted by halogen, C₁-C₄alkyl, C₁-C₄alkoxy and/or trifluoromethyl and R₂' is hydroxy, and wherein R represents C₁-C₇alkyl, or of a salt thereof.

8. A process as claimed in claim any one of claims 1 to 7 for the manufacture of compounds of the formula I, wherein R denotes C₃-C₄alkyl or, if applicable, methyl, or of a salt thereof.

9. A process as claimed in claim 2 for the manufacture of compounds of the formula I, wherein R is C₂-C₇-alkyl, and wherein R₁ and R₁' are fluoro and R₂ and R₂' are hydrogen, or R₁ and R₁' are hydrogen and R₂ and R₂' together represent oxo, or of a salt thereof.

10. A process as claimed in claim 2 for the manufacture of 3-amino-2-oxo-propyl(n-butyl)phosphinic acid or of a salt thereof.

11. A process as claimed in claim 2 for the manufacture of 3-amino-2-(4-chlorophenyl)-2-hydroxy-propyl(n-butyl)phosphinic acid or of a salt thereof.

12. A process as claimed in claim 2 for the manufacture of 3-amino-1,1-difluoropropyl(n-butyl)phosphinic acid or of a salt thereof.

13. A process as claimed in claim 2 for the manufacture of 3-amino-2-oxo-propyl(cyclopropylmethyl)phosphinic acid or of a salt thereof.

14. A process as claimed in claim 2 for the manufacture of 3-amino-2-oxo-propyl(cyclohexylmethyl)phosphinic acid or of a salt thereof.

15. A process as claimed in claim 2 for the manufacture of 3-amino-2-hydroxy-2-methyl-propyl(n-butyl)phosphinic acid or of a salt thereof.

16. A process as claimed in claim 2 for the manufacture of 3-amino-1-fluoro-propyl(n-butyl)phosphinic acid or of a salt thereof.

17. A process as claimed in claim 2 for the manufacture of 3-amino-2-(4-chlorophenyl)-2-hydroxy-propyl(cyclohexylmethyl)phosphinic acid or of a salt thereof.

18. A process as claimed in claim 2 for the manufacture of 3-amino-2-(4-chlorophenyl)-2-hydroxy-propyl(benzyl)phosphinic acid or of a salt thereof.

19. A process as claimed in claim 2 for the manufacture of 3-amino-2-(4-chlorophenyl)-2-hydroxy-propyl(cyclopropylmethyl)phosphinic acid or of a salt thereof.

20. A process as claimed in claim 2 for the manufacture of 3-amino-2-(4-chlorophenyl)-2-hydroxy-propyl(cyclopropylmethyl)phosphinic acid or of a salt thereof.

21. A process as claimed in claim 2 for the manufacture of 3-amino-2-(4-chlorophenyl)-2-oxo-propyl(diethoxymethyl)phosphinic acid or of a salt thereof.

22. A process as claimed in claim 2 for the manufacture of 3-amino-2-(4-chlorophenyl)-2-hydroxy-propyl(diethoxymethyl)phosphinic acid or of a salt thereof.

23. A process as claimed in claim 2 for the manufacture of 3-amino-2-(4-chlorophenyl)2-hydroxypropyl(methyl)phosphinic acid or of a salt thereof.

## Patentansprüche (Patentansprüche für folgende(n) Vertragsstaat(en): AT, BE, CH, DE, DK, FR, GB, IT, LI, LU, NL, SE)

1. Verbindung der Formel I 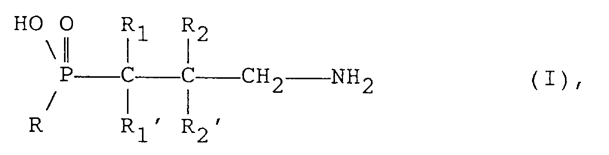 worin entweder R₁ Halogen bedeutet, R₁' Halogen oder Wasserstoff darstellt und R₂ und R₂' Wasserstoff bedeuten oder R₁ und R₁' Wasserstoff darstellen, R₂ einen aliphatischen oder aromatischen Rest bedeutet und R₂' Hydroxy darstellt oder R₂ und R₂' zusammen Oxo bedeuten, und worin R einen aliphatischen, cycloaliphatischen, cycloaliphatisch-aliphatischen oder araliphatischen Rest mit 2 oder mehr Kohlenstoffatomen bedeutet oder wenn R₁ und R₁' Wasserstoff bedeuten, R₂ einen aromatischen Rest darstellt und R₂' Hydroxy bedeutet, R Methyl darstellt oder ein Salz davon.

2. Verbindung nach Anspruch 1 der Formel I, worin entweder R₁ Wasserstoff darstellt, R₁' Halogen oder Wasserstoff bedeutet und R₂ und R₂' Wasserstoff bedeuten oder R₁ und R₁' Wasserstoff darstellen, R₂ einen aromatischen Rest darstellt und R₂' Hydroxy bedeutet oder R₂ und R₂' zusammen Oxo darstellen, oder worin R einen aliphatischen, cycloaliphatischen, cycloaliphatisch-aliphatischen oder araliphatischen Rest mit 2 oder mehr Kohlenstoffatomen bedeutet oder ein Salz davon.

3. Verbindung nach Anspruch 1 der Formel I, worin R₁ Halogen bedeutet, R₁' Halogen oder Wasserstoff darstellt und R₂ und R₂' Wasserstoff sind oder R₁ und R₁' Wasserstoff sind, R₂ Niederalkyl, Phenyl, Phenyl, mono- oder disubstituiert mit Halogen, Niederalkyl, Niederalkoxy und/oder Trifluormethyl, oder Pyridyl bedeutet und R₂' Hydroxy darstellt oder R₂ und R₂' zusammen Oxo wiedergeben und worin R Niederalkyl mit 2 oder mehreren Kohlenstoffatomen, Niederalkenyl, Niederalkinyl, eine Cycloalkyl-, Hydroxycycloalkyl-, Cycloalkylniederalkyl-, Cycloalkyl-(hydroxy)niederalkyl- oder Niederalkylthiocycloalkyl-(hydroxy)niederalkylgruppe mit 3 bis 6 Ringkohlenstoffatomen, Oxoniederalkyl, Aminoniederalkyl, Niederalkanoylaminoniederalkyl, Phthalimidoniederalkyl, Mono- oder Dihydroxyniederalkyl, Hydroxyniederalkenyl, Amino-(hydroxy)niederalkyl, Niederalkanoylamino-(hydroxy)niederalkyl, Phthalimido-(hydroxy)niederalkyl, Mono-, Di- oder Polyhalogenniederalkyl, Mono-, Di- oder Polyhalogenniederalkenyl, Mono-, Di- oder Polyhalogen-(hydroxy)niederalkyl, Mono-, Di- oder Polyhalogen-(hydroxy)niederalkenyl, Aminoniederalkoxyniederalkyl, Niederalkylthioniederalkyl, Niederalkansulfinylniederalkyl, Niederalkansulfonylniederalkyl, Diniederalkoxyniederalkyl, Diniederalkylthioniederalkyl, Niederalkoxy(hydroxy)niederalkyl, Niederalkoxy-(halogen)niederalkyl, Phenylniederalkyl, Phenylniederalkyl, mono- oder disubstituiert in dem Phenylteil mit Halogen, Niederalkyl, Niederalkoxy und/oder Trifluormethyl, Naphthylniederalkyl, Oxa- oder Thiacycloalkyl mit 2 bis 6 Ringkohlenstoffatomen, oder Dioxa-, Oxathia- oder Dithiacycloalkyl mit 3 bis 5 Ringkohlenstoffatomen bedeutet oder, wenn R₁ und R₁' Wasserstoff bedeuten, R₂ Phenyl, Phenyl, mono- oder disubstituiert mit Halogen, Niederalkyl, Niederalkoxy und/oder Trifluormethyl oder Pyridyl bedeutet und R₂' Hydroxy bedeutet, R Methyl wiedergibt, oder ein Salz davon.

4. Verbindung nach Anspruch 2 der Formel I, worin R₁ Halogen bedeutet, R₁' Halogen oder Wasserstoff darstellt und R₂ und R₂' Wasserstoff bedeuten oder R₁ und R₁' Wasserstoff darstellen, R₂ Phenyl oder Phenyl, mono- oder disubstituiert mit Halogen, Niederalkyl, Niederalkoxy und/oder Trifluormethyl bedeutet und R₂' Hydroxy darstellt oder R₂ und R₂' zusammen Oxo wiedergeben und worin R Niederalkyl mit 2 oder mehreren Kohlenstoffatomen, Niederalkenyl, Niederalkinyl, eine Cycloalkyl-, Hydroxycycloalkyl-, Cycloalkylniederalkyl-, Cycloalkyl-(hydroxy)niederalkyl- oder Niederalkylthiocycloalkyl-(hydroxy)niederalkylgruppe mit 3 bis 6 Ringkohlenstoffatomen, Oxoniederalkyl, Aminoniederalkyl, Niederalkanoylaminoniederalkyl, Phthalimidoniederalkyl, Mono- oder Dihydroxyniederalkyl, Hydroxyniederalkenyl, Amino-(hydroxy)niederalkyl, Niederalkanoylamino-(hydroxy)niederalkyl, Phthalimido(hydroxy)niederalkyl, Mono-, Di- oder Polyhalogenniederalkyl, Mono-, Di- oder Polyhalogenniederalkenyl, Mono-, Di- oder Polyhalogen-(hydroxy)niederalkyl, Mono-, Di- oder Polyhalogen(hydroxy)niederalkenyl, Aminoniederalkoxyniederalkyl, Niederalkylthioniederalkyl, Niederalkansulfinylniederalkyl, Niederalkansulfonylniederalkyl, Diniederalkoxyniederalkyl, Diniederalkylthioniederalkyl, Niederalkoxy-(hydroxy)niederalkyl, Niederalkoxy-(halogen)niederalkyl, Phenylniederalkyl, Phenylniederalkyl, mono- oder disubstituiert in dem Phenylteil mit Halogen, Niederalkyl, Niederalkoxy und/oder Trifluormethyl, Naphthylniederalkyl, Oxa- oder Thiacycloalkyl mit 2 bis 6 Ringkohlenstoffatomen oder Dioxa-, Oxathia- oder Dithiacycloalkyl mit 3 bis 5 Ringkohlenstoffatomen wiedergibt, oder ein Salz davon.

5. Verbindung nach Anspruch 2 der Formel I, worin entweder R₁ und R₁' Fluor bedeuten und R₂ und R₂' Wasserstoff wiedergeben oder R₁ und R₁' Wasserstoff darstellen, R₂ Phenyl, Phenyl, substituiert mit Halogen, C₁-C₄-Alkyl, C₁-C₄-Alkoxy und/oder Trifluormethyl bedeutet und R₂' Hydroxy bedeutet oder R₁ und R₁' Wasserstoff darstellen und R₂ und R₂' zusammen Oxo wiedergeben, und worin R C₂-C₁₂-Alkyl, C₂-C₇-Alkenyl, C₂-C₇-Alkinyl, Mono- oder Dihydroxy-C₂-C₇-alkyl, Oxo-C₃-C₇-alkyl, Amino-C₃-C₆-alkyl, Phthalimido-C₃-C₆-alkyl oder Phthalimido-C₃-C₇(hydroxy)alkyl bedeutet, oder ein Salz davon.

6. Verbindung nach Anspruch 2 der Formel I, worin R₁ und R₁' Fluor bedeuten und R₂ und R₂' Wasserstoff darstellen, oder R₁ und R₁' Wasserstoff darstellen und R₂ und R₂' zusammen Oxo wiedergeben, und worin R C₂-C₇-Alkyl, α-gesättigtes C₃-C₇-Alkenyl, α-gesättigtes C₃-C₇-Alkinyl, α-, β-, γ- oder δ-Hydroxy-C₂-C₇-alkyl, α,α-Difluor-C₂-C₄-alkyl, Mono-, Di- oder Trifluor-α-hydroxy-C₃-C₇-alkyl, Mono-, Di- oder Trihalogen-α-hydroxy-C₃-C₇-alkenyl, C₁-C₄-Alkoxy-C₁-C₄-alkyl, Di-C₁-C₄-alkoxy-C₁-C₄-alkyl, C₃-C₆-Cycloalkyl-C₁-C₄-alkyl, α-Hydroxy-C₃-C₆-cycloalkyl oder C₃-C₆-Cycloalkyl-α-hydroxy-C₁-C₄-alkyl bedeutet, oder ein Salz davon.

7. Verbindung nach Anspruch 1 der Formel I, worin R₁ und R₁' Wasserstoff bedeuten, R₂ Phenyl, Phenyl, substituiert mit Halogen, C₁-C₄-Alkyl, C₁-C₄-Alkoxy und/oder Trifluormethyl bedeutet und R₂' Hydroxy darstellt, und worin R C₁-C₇-Alkyl wiedergibt oder ein Salz davon.

8. Verbindung nach einem der Ansprüche 1 bis 7, worin R C₃-C₄-Alkyl oder, falls anwendbar, Methyl wiedergibt oder ein Salz davon.

9. Verbindung nach Anspruch 2, worin R C₂-C₇-Alkyl bedeutet und worin R₁ und R₁' Fluor darstellen und R₂ und R₂' Wasserstoff bedeuten oder R₁ und R₁' Wasserstoff darstellen und R₂ und R₂' zusammen Oxo wiedergeben oder ein Salz davon.

10. 3-Amino-2-oxo-propyl(n-butyl)phosphinsäure oder ein Salz davon.

11. 3-Amino-2-(4-chlorphenyl)-2-hydroxypropyl(n-butyl)phosphinsäure oder ein Salz davon.

12. 3-Amino-1,1-difluor-propyl(n-butyl)phosphinsäure oder ein Salz davon.

13. 3-Amino-2-oxo-propyl(cyclopropylmethyl)phosphinsäure oder ein Salz davon.

14. 3-Amino-2-oxo-propyl(cyclohexylmethyl)phosphinsäure oder ein Salz davon.

15. 3-Amino-2-hydroxy-2-methyl-propyl(n-butyl)phosphinsäure oder ein Salz davon.

16. 3-Amino-1-fluor-propyl(n-butyl)phosphinsäure oder ein Salz davon.

17. 3-Amino-2-(4-chlorphenyl)-2-hydroxy-propyl(cyclohexylmethyl)phosphinsäure oder ein Salz davon.

18. 3-Amino-2-(4-chlorphenyl)-2-hydroxy-propyl(benzyl)phosphinsäure oder ein Salz davon.

19. 3-Amino-2-(4-chlorphenyl)-2-hydroxy-propyl(cyclopropylmethyl)phosphinsäure oder ein Salz davon.

20. 3-Amino-2-(4-chlorphenyl)-2-hydroxy-propyl(cyclopropylmethyl)phosphinsäure oder ein Salz davon.

21. 3-Amino-2-(4-chlorphenyl)-2-oxo-propyl(diethoxymethyl)phosphinsäure oder ein Salz davon.

22. 3-Amino-2-(4-chlorphenyl)-2-hydroxy-propyl(diethoxymethyl)phosphinsäure oder ein Salz davon.

23. 3-Amino-2-(4-chlorphenyl)-2-hydroxy-propyl(methyl)phosphinsäure oder ein Salz davon.

24. Verbindung nach einem der Ansprüche 2, 4 bis 6 und 9 bis 13 zur Verwendung in einem Verfahren zur therapeutischen Behandlung des menschlichen oder tierischen Körpers.

25. Verbindung nach einem der Ansprüche 1, 3, 7, 8 und 14 bis 23 zur Verwendung in einem Verfahren zur therapeutischen Behandlung des menschlichen oder tierischen Körpers.

26. Pharmazeutische Zusammensetzung, die mindestens eine Verbindung nach einem der Ansprüche 2, 4 bis 6, 9 bis 13 und 24 in Anmischung mit einem üblichen pharmazeutischen Exzipienten enthält.

27. Pharmazeutische Zusammensetzung, die mindestens eine Verbindung nach einem der Ansprüche 1, 3, 7, 8, 14 bis 23 und 25 in Anmischung mit einem üblichen pharmazeutischen Exzipienten enthält.

28. Verfahren zur Herstellung einer Verbindung der Formel I 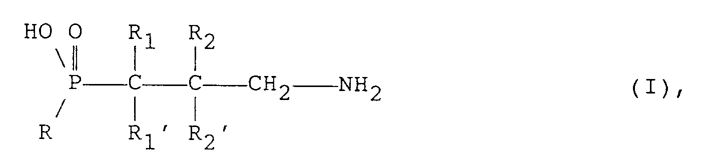 worin entweder R₁ Halogen darstellt, R₁' Halogen oder Wasserstoff bedeutet und R₂ und R₂' Wasserstoff bedeuten oder R₁ und R₁' Wasserstoff wiedergeben, R₂ einen aliphatischen oder aromatischen Rest bedeutet und R₂' Hydroxy darstellt oder R₂ und R₂' zusammen Oxo wiedergeben und worin R einen aliphatischen, cycloaliphatischen, cycloaliphatisch-aliphatischen oder araliphatischen Rest mit 2 oder mehreren Kohlenstoffatomen darstellt oder, wenn R₁ und R₁' Wasserstoff bedeuten, R₂ einen aromatischen Rest wiedergibt und R₂' Hydroxy bedeutet, R Methyl darstellt und deren Salze, dadurch gekennzeichnet, daß
a) in einer Verbindung der Formel II 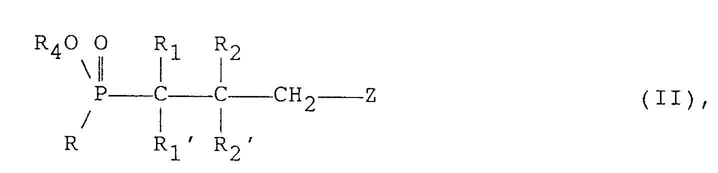 worin R, R₁, R₁', R₂ und R₂' ihre vorstehende Bedeutung aufweisen, Z eine geschützte oder latente Aminogruppe Zₒ wiedergibt und R₄ Wasserstoff oder eine Hydroxyschutzgruppe R₅ bedeutet, und worin Amino als ein Bestandteil von R und/oder Hydroxy R₂' oder Oxo R₂ + R₂' in einer zeitweilig geschützten Form vorliegen können, Gruppen R₅ oder R₆ durch Wasserstoff ersetzt werden und/oder eine Gruppe Zₒ in -NH₂ umgewandelt wird; oder
b) in einer Verbindung der Formel III 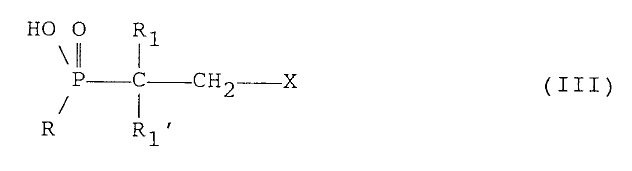 worin R₁ und R₁' ihre vorstehende Bedeutung aufweisen und X eine Gruppe ist, die in eine Gruppe der Formel -CH₂NH₂ (Ia) umgewandelt werden kann, die Gruppe X in eine Gruppe der Formel Ia umgewandelt wird; oder
c) eine Verbindung der Formel IV 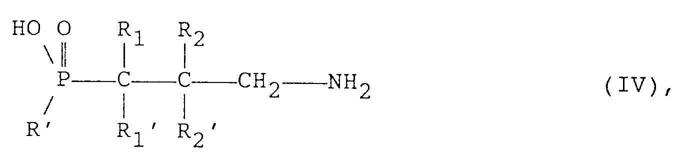 worin R' ausgewählt sein kann aus Niederalkenyl, Niederalkadienyl oder Niederalkinyl zur Herstellung einer Verbindung der Formel I, worin R Niederalkyl bedeutet, oder aus Phenyl zur Herstellung einer Verbindung der Formel I, worin R Cyclohexyl darstellt, reduziert wird und, falls gewünscht, ein sich ergebendes Salz, erhalten in diesem Verfahren, in die freie Verbindung oder in ein anderes Salz umgewandelt werden kann und/oder, falls gewünscht, eine sich ergebende freie Verbindung in ein Salz, das der vorstehenden Definition genügt, umgewandelt wird und/oder, falls gewünscht, ein sich ergebendes Isomerengemisch in die einzelnen Isomeren aufgetrennt wird.

29. Verwendung einer Verbindung nach einem der Ansprüche 1 bis 25 zur Herstellung eines nootropen, antidepressiv wirkenden oder angstlösenden Arzneimittels.

## Patentansprüche (Patentansprüche für folgende(n) Vertragsstaat(en): ES, GR)

1. Verfahren zur Herstellung einer Verbindung der Formel I 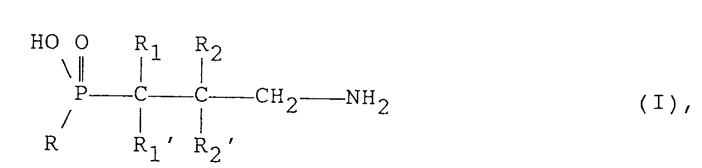 worin entweder R₁ Halogen darstellt, R₁' Halogen oder Wasserstoff bedeutet und R₂ und R₂' Wasserstoff bedeuten oder R₁ und R₁' Wasserstoff wiedergeben, R₂ einen aliphatischen oder aromatischen Rest bedeutet und R₂' Hydroxy darstellt oder R₂ und R₂' zusammen Oxo wiedergeben und worin R einen aliphatischen, cycloaliphatischen, cycloaliphatisch-aliphatischen oder araliphatischen Rest mit 2 oder mehreren Kohlenstoffatomen darstellt oder, wenn R₁ und R₁' Wasserstoff bedeuten, R₂ einen aromatischen Rest wiedergibt und R₂' Hydroxy bedeutet, R Methyl darstellt und deren Salze, dadurch gekennzeichnet, daß
a) in einer Verbindung der Formel II 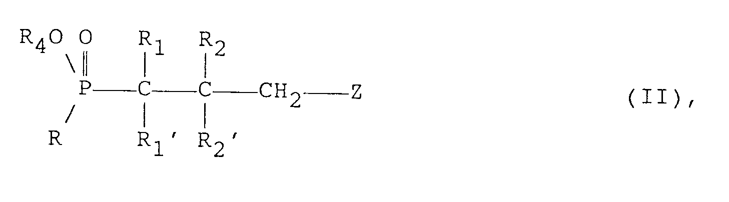 worin R, R₁, R₁', R₂ und R₂' ihre vorstehende Bedeutung aufweisen, Z eine geschützte oder latente Aminogruppe Zₒ wiedergibt und R₄ Wasserstoff oder eine Hydroxyschutzgruppe R₅ bedeutet, und worin Amino als ein Bestandteil von R und/oder Hydroxy R₂' oder Oxo R₂ + R₂' in einer zeitweilig geschützten Form vorliegen können, Gruppen R₅ oder R₆ durch Wasserstoff ersetzt werden und/oder eine Gruppe Zₒ in -NH₂ umgewandelt wird; oder
b) in einer Verbindung der Formel III 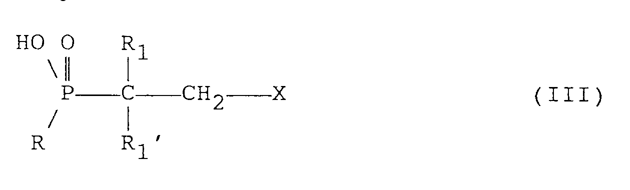 worin R₁ und R₁' ihre vorstehende Bedeutung aufweisen und X eine Gruppe ist, die in eine Gruppe der Formel -CH₂NH₂ (Ia) umgewandelt werden kann, die Gruppe X in eine Gruppe der Formel Ia umgewandelt wird; oder
c) eine Verbindung der Formel IV 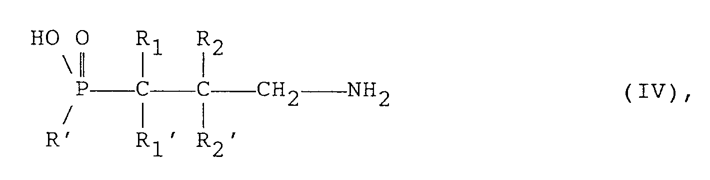 worin R' ausgewählt sein kann aus Niederalkenyl, Niederalkadienyl oder Niederalkinyl zur Herstellung einer Verbindung der Formel I, worin R Niederalkyl bedeutet, oder aus Phenyl zur Herstellung einer Verbindung der Formel I, worin R Cyclohexyl darstellt, reduziert wird und, falls gewünscht, ein sich ergebendes Salz, erhalten in diesem Verfahren, in die freie Verbindung oder in ein anderes Salz umgewandelt werden kann und/oder, falls gewünscht, eine sich ergebende freie Verbindung in ein Salz, das der vorstehenden Definition genügt, umgewandelt wird und/oder, falls gewünscht, ein sich ergebendes Isomerengemisch in die einzelnen Isomeren aufgetrennt wird.

2. Verfahren nach Anspruch 1 zur Herstellung von Verbindungen der Formel I 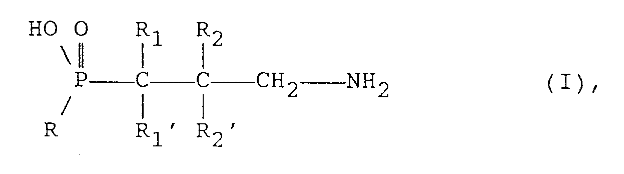 worin entweder R₁ Halogen bedeutet, R₁' Halogen oder Wasserstoff darstellt und R₂ und R₂' Wasserstoff bedeuten oder R₁ und R₁' Wasserstoff darstellen, R₂ einen aromatischen Rest bedeutet und R₂' Hydroxy darstellt oder R₂ und R₂' zusammen Oxo bedeuten oder worin R einen aliphatischen, cycloaliphatischen, cycloaliphatisch-aliphatischen oder araliphatischen Rest mit 2 oder mehr Kohlenstoffatomen bedeutet und deren Salzen dadurch gekennzeichnet, daß
a) in einer Verbindung der Formel II 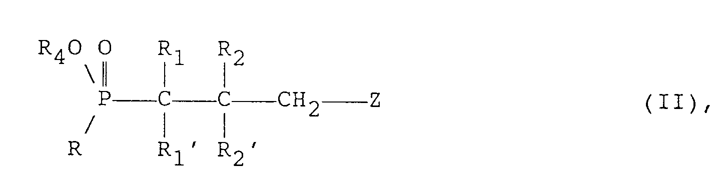 worin R, R₁, R₁', R₂ und R₂' ihre vorstehenden Bedeutungen aufweisen, Z eine geschützte oder latente Aminogruppe Zₒ wiedergibt und R₄ Wasserstoff oder eine Hydroxyschutzgruppe R₅ bedeutet, und worin Amino als ein Bestandteil von R und/oder Hydroxy R₂' oder Oxo R₂ + R₂' in einer zeitweilig geschützten Form vorliegen können, eine Gruppe R₅ durch Wasserstoff ersetzt wird und/oder eine Gruppe Zₒ in -NH₂ umgewandelt wird; oder
b) in eine Verbindung der Formel III 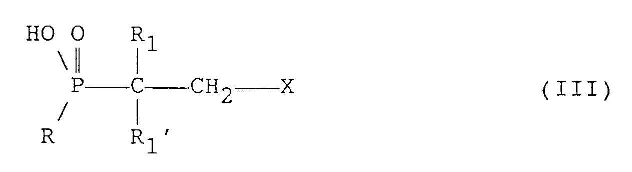 worin R₁ und R₁' ihre vorstehende Bedeutung aufweisen und X eine Gruppe, die in eine Gruppe der Formel -CH₂NH₂ (Ia) umgewandelt werden kann, bedeutet, die Gruppe X in eine Gruppe der Formel Ia umgewandelt wird; oder
c) eine Verbindung der Formel IV 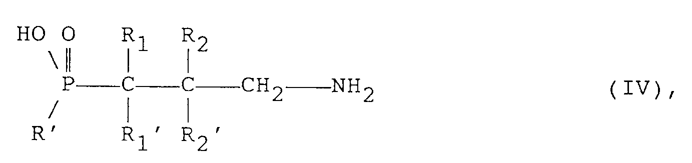 worin R' ausgewählt sein kann aus Niederalkenyl, Niederalkadienyl oder Niederalkinyl zur Herstellung einer Verbindung der Formel I, worin R Niederalkyl bedeutet, oder aus Phenyl zur Herstellung einer Verbindung der Formel I, worin R Cyclohexyl darstellt, reduziert wird und, falls gewünscht, ein sich ergebendes Salz, erhalten in diesem Verfahren, in die freie Verbindung oder in ein anderes Salz umgewandelt werden kann und/oder, falls gewünscht, eine sich ergebende freie Verbindung in ein Salz, das der vorstehenden Definition genügt, umgewandelt wird und/oder, falls gewünscht, ein sich ergebendes Isomerengemisch in die einzelnen Isomeren aufgetrennt wird.

3. Verfahren nach Anspruch 1 zur Herstellung von Verbindungen der Formel I, worin R₁ Halogen bedeutet, R₁' Halogen oder Wasserstoff darstellt und R₂ und R₂' Wasserstoff sind oder R₁ und R₁' Wasserstoff sind, R₂ Niederalkyl, Phenyl, Phenyl, mono- oder disubstituiert mit Halogen, Niederalkyl, Niederalkoxy und/oder Trifluormethyl oder Pyridyl bedeutet und R₂' Hydroxy darstellt oder R₂ und R₂' zusammen Oxo wiedergeben und worin R Niederalkyl mit 2 oder mehreren Kohlenstoffatomen, Niederalkenyl, Niederalkinyl, eine Cycloalkyl-, Hydroxycycloalkyl-, Cycloalkylniederalkyl-, Cycloalkyl(hydroxy)niederalkyl- oder Niederalkylthiocycloalkyl-(hydroxy)niederalkylgruppe mit 3 bis 6 Ringkohlenstoffatomen, Oxoniederalkyl, Aminoniederalkyl, Niederalkanoylaminoniederalkyl, Phthalimidoniederalkyl, Mono- oder Dihydroxyniederalkyl, Hydroxyniederalkenyl, Amino-(hydroxy)niederalkyl, Niederalkanoylamino-(hydroxy)niederalkyl, Phthalimido-(hydroxy)niederalkyl, Mono-, Di- oder Polyhalogenniederalkyl, Mono-, Di- oder Polyhalogenniederalkenyl, Mono-, Di- oder Polyhalogen-(hydroxy)niederalkyl, Mono-, Di- oder Polyhalogen(hydroxy)niederalkenyl, Aminoniederalkoxyniederalkyl, Niederalkylthioniederalkyl, Niederalkansulfinylniederalkyl, Niederalkansulfonylniederalkyl, Diniederalkoxyniederalkyl, Diniederalkylthioniederalkyl, Niederalkoxy-(hydroxy)niederalkyl, Niederalkoxy-(halogen)niederalkyl, Phenylniederalkyl, Phenylniederalkyl, mono- oder disubstituiert in dem Phenylteil mit Halogen, Niederalkyl, Niederalkoxy und/oder Trifluormethyl, Naphthylniederalkyl, Oxa- oder Thiacycloalkyl mit 2 bis 6 Ringkohlenstoffatomen, oder Dioxa-, Oxathia- oder Dithiacycloalkyl mit 3 bis 5 Ringkohlenstoffatomen bedeutet oder, falls R₁ und R₁' Wasserstoff bedeuten, R₂ Phenyl, Phenyl, mono- oder disubstituiert mit Halogen, Niederalkyl, Niederalkoxy und/oder Trifluormethyl bedeutet oder Pyridyl bedeutet und R₂' Hydroxy, R Methyl wiedergibt oder einem Salz davon.

4. Verfahren nach Anspruch 2 zur Herstellung von Verbindungen der Formel I, worin R₁ Halogen bedeutet, R₁' Halogen oder Wasserstoff darstellt und R₂ und R₂' Wasserstoff bedeuten oder R₁ und R₁' Wasserstoff darstellen, R₂ Phenyl oder Phenyl, mono- oder disubstituiert mit Halogen, Niederalkyl, Niederalkoxy und/oder Trifluormethyl bedeutet und R₂' Hydroxy darstellt oder R₂ und R₂' zusammen Oxo wiedergeben und worin R Niederalkyl mit 2 oder mehreren Kohlenstoffatomen, Niederalkenyl, Niederalkinyl, eine Cycloalkyl-, Hydroxycycloalkyl-, Cycloalkylniederalkyl, Cycloalkyl-(hydroxy)niederalkyl- oder Niederalkylthiocycloalkyl-(hydroxy)niederalkylgruppe mit 3 bis 6 Ringkohlenstoffatomen, Oxoniederalkyl, Aminoniederalkyl, Niederalkanoylaminoniederalkyl, Phthalimidoniederalkyl, Mono- oder Dihydroxyniederalkyl, Hydroxyniederalkenyl, Amino-(hydroxy)niederalkyl, Niederalkanoylamino-(hydroxy)niederalkyl, Phthalimido-(hydroxy)niederalkyl, Mono-, Di- oder Polyhalogenniederalkyl, Mono-, Di- oder Polyhalogenniederalkenyl, Mono-, Di- oder Polyhalogen(hydroxy)niederalkyl, Mono-, Di- oder Polyhalogen-(hydroxy)niederalkenyl, Aminoniederalkoxyniederalkyl, Niederalkylthioniederalkyl, Niederalkansulfinylniederalkyl, Niederalkansulfonylniederalkyl, Diniederalkoxyniederalkyl, Diniederalkylthioniederalkyl, Niederalkoxy-(hydroxy)niederalkyl, Niederalkoxy-(halogen)niederalkyl, Phenylniederalkyl, Phenylniederalkyl, mono- oder disubstituiert in dem Phenylteil mit Halogen, Niederalkyl, Niederalkoxy und/oder Trifluormethyl, Naphthylniederalkyl, Oxa- oder Thiacycloalkyl mit 2 bis 6 Ringkohlenstoffatomen oder Dioxa-, Oxathia- oder Dithiacycloalkyl mit 3 bis 5 Ringkohlenstoffatomen wiedergibt oder einem Salz davon.

5. Verfahren nach Anspruch 2 zur Herstellung von Verbindungen der Formel I, worin entweder R₁ und R₁' Fluor bedeuten und R₂ und R₂' Wasserstoff wiedergeben oder R₁ und R₁' Wasserstoff darstellen, R₂ Phenyl, Phenyl, substituiert mit Halogen, C₁-C₄-Alkyl, C₁-C₄-Alkoxy und/oder Trifluormethyl bedeutet und R₂' Hydroxy bedeutet oder R₁ und R₁' Wasserstoff darstellen und R₂ und R₂' zusammen Oxo wiedergeben, und worin R C₂-C₁₂-Alkyl, C₂-C₇-Alkenyl, C₂-C₇-Alkinyl, Mono- oder Dihydroxy-C₂-C₇-alkyl, Oxo-C₃-C₇-alkyl, Amino-C₃-C₆-alkyl, Phthalimido-C₃-C₆-alkyl oder Phthalimido-C₃-C₇(hydroxy)alkyl bedeutet, oder einem Salz davon.

6. Verfahren nach Anspruch 1 zur Herstellung von Verbindungen der Formel I, worin R₁ und R₁' Fluor bedeuten und R₂ und R₂' Wasserstoff darstellen, oder R₁ und R₁' Wasserstoff darstellen und R₂ und R₂' zusammen Oxo wiedergeben, und worin R C₂-C₇-Alkyl, α-gesättigtes C₃-C₇-Alkenyl, α-gesättigtes C₃-C₇-Alkinyl, α-, β-, γ- oder δ-Hydroxy-C₂-C₇-alkyl, α,α-Difluor-C₂-C₄-alkyl, Mono-, Di- oder Trifluor-α-hydroxy-C₃-C₇-alkyl, Mono-, Di- oder Trihalogen-α-hydroxy-C₃-C₇-alkenyl, C₁-C₄-Alkoxy-C₁-C₄-alkyl, Di-C₁-C₄-alkoxy-C₁-C₄-alkyl, C₃-C₆-Cycloalkyl-C₁-C₄-alkyl, α-Hydroxy-C₃-C₆-cycloalkyl oder C₃-C₆-Cycloalkyl-α-hydroxy-C₁-C₄-alkyl bedeutet, oder einem Salz davon.

7. Verfahren nach Anspruch 1 zur Herstellung von Verbindungen der Formel I, worin R₁ und R₁' Wasserstoff bedeuten, R₂ Phenyl, Phenyl, substituiert mit Halogen, C₁-C₄-Alkyl, C₁-C₄-Alkoxy und/oder Trifluormethyl bedeutet und R₂' Hydroxy darstellt, und worin R C₁-C₇-Alkyl wiedergibt oder einem Salz davon.

8. Verfahren nach einem der Ansprüche 1 bis 7 zur Herstellung von Verbindungen der Formel I, worin R C₃-C₄-Alkyl oder, falls anwendbar, Methyl, wiedergibt oder einem Salz davon.

9. Verfahren nach Anspruch 2 zur Herstellung von Verbindungen der Formel I, worin R C₂-C₇-Alkyl bedeutet und worin R₁ und R₁' Fluor darstellen und R₂ und R₂' Wasserstoff bedeuten oder R₁ und R₁' Wasserstoff darstellen und R₂ und R₂' zusammen Oxo wiedergeben oder einem Salz davon.

10. Verfahren nach Anspruch 2 zur Herstellung von 3-Amino-2-oxo-propyl(n-butyl)phosphinsäure oder einem Salz davon.

11. Verfahren nach Anspruch 2 zur Herstellung von 3-Amino-2-(4-chlorophenyl )-2-hydroxypropyl(n-butyl )phosphinsäure oder einem Salz davon.

12. Verfahren nach Anspruch 2 zur Herstellung von 3-Amino-1,1-difluor-propyl(n-butyl)phosphinsäure oder einem Salz davon.

13. Verfahren nach Anspruch 2 zur Herstellung von 3-Amino-2-oxo-propyl(cyclopropylmethyl)phosphinsäure oder einem Salz davon.

14. Verfahren nach Anspruch 2 zur Herstellung von 3-Amino-2-oxo-propyl(cyclohexylmethyl)phosphinsäure oder einem Salz davon.

15. Verfahren nach Anspruch 2 zur Herstellung von 3-Amino-2-hydroxy-2-methyl-propyl(n-butyl)phosphinsäure oder einem Salz davon.

16. Verfahren nach Anspruch 2 zur Herstellung von 3-Amino-1-fluor-propyl(n-butyl)phosphinsäure oder einem Salz davon.

17. Verfahren nach Anspruch 2 zur Herstellung von 3-Amino-2- (4-chlorphenyl)-2-hydroxy-propyl-(cyclohexylmethyl)phosphinsäure oder einem Salz davon.

18. Verfahren nach Anspruch 2 zur Herstellung von 3-Amino-2-(4-chlorphenyl)-2-hydroxy-propyl(benzyl)phosphinsäure oder einem Salz davon.

19. Verfahren nach Anspruch 2 zur Herstellung von 3-Amino-2-(4-chlorphenyl) -2-hydroxy-propyl(cyclopropylmethyl)phosphinsäure oder einem Salz davon.

20. Verfahren nach Anspruch 2 zur Herstellung von 3-Amino-2-(4-chlorphenyl) -2-hydroxypropyl-(cyclopropylmethyl)phosphinsäure oder einem Salz davon.

21. Verfahren nach Anspruch 2 zur Herstellung von 3-Amino-2-(4-chlorphenyl)-2-oxo-propyl(diethoxymethyl)phosphinsäure oder einem Salz davon.

22. Verfahren nach Anspruch 2 zur Herstellung von 3-Amino-2-(4-chlorphenyl)-2-hydroxy-propyl(diethoxymethyl)phosphinsäure oder einem Salz davon.

23. Verfahren nach Anspruch 2 zur Herstellung von 3-Amino-2-(4-chlorphenyl)-2-hydroxy-propyl(methyl)phosphinsäure oder einem Salz davon.

## Revendications (Revendications pour l'(les) Etat(s) contractant(s) suivant(s): AT, BE, CH, DE, DK, FR, GB, IT, LI, LU, NL, SE)

1. Composé de formule I 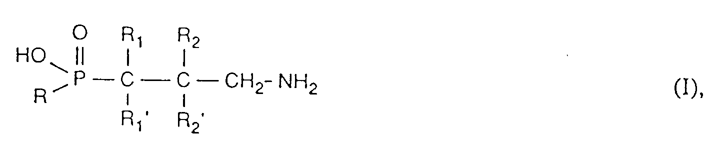 dans laquelle ou bien R₁ est un halogène, R_{1'} est un halogène ou un hydrogène et R₂ et R_{2'} dénotent un hydrogène, ou bien R₁ et R_{1'} représentent un hydrogène, R₂ est un radical aliphatique ou aromatique et R_{2'} est un hydroxy, ou bien R₂ et R_{2'} représentent ensemble un oxo, et où R dénote un radical aliphatique, cycloaliphatique, cycloaliphatique-aliphatique ou araliphatique ayant 2 atomes de carbone ou plus ou, si R₁ et R_{1'} dénotent un hydrogène, R₂ représente un radical aromatique et R_{2'} est un hydroxy, R représente un méthyle, ou un de ses sels.

2. Composé selon la revendication 1, de formule I, ou bien R₁ est un halogène, R_{1'} est un halogène ou un hydrogène et R₂ et R_{2'} dénotent un hydrogène, ou bien R₁ et R_{1'} représentent un hydrogène, R₂ est un radical aromatique et R_{2'} est un hydroxy ou bien R₂ et R₂' représentent ensemble un oxo, ou bien R dénote un radical aliphatique, cycloaliphatique, cycloaliphatique-alipha-tique, ayant de 2 atomes de carbone ou plus, ou un de ses sels.

3. Composé selon la revendication 1 de formule I, dans laquelle R₁ est un halogène, R_{1'} est un halogène ou un hydrogène et R₂ et R_{2'} représentent un hydrogène ou bien R₁ et R_{1'} représentent un hydrogène, R₂ dénote un alkyle inférieur, un phényle, un phényl mono- ou disubstitué par un halogène, un alkyle inférieur, un alcoxy inférieur et/ou un trifluorométhyle ou un pyridyle et R_{2'} est un hydroxy ou bien R₂ et R_{2'} représentent ensemble un oxo, et où R dénote un alkyle inférieur ayant 2 atomes de carbone ou plus, un alcényle inférieur, un alcynyle inférieur, un cycloalkyle, un hydroxycycloalkyle, un cycloalkyl-alkyle inférieur, un cycloalkyle(hydroxy) alkyle inférieur, ou un alkyl inférieur thio-cycloalkyle- (hydroxy)alkyle inférieur ayant de 3 à 6 atomes de carbone dans le cyle, un oxo-alkyle inférieur, un amino alkyle inférieur, un alcanoyl inférieur amino-alkyle inférieur, un phtalimido-alkyle inférieur, un mono- ou di-hydroxy alkyle inférieur, un hydroxy-alcényle inférieur, un amino-(hydroxy)alkyle inférieur, un alcanoyl inférieur amino-(hydroxy)alkyle inférieur, un phtalimido-(hydroxy)alkyle inférieur, un mono-, di- ou polyhalogéno-alkyle inférieur, un mono-, di- ou polyhalogéno-alcényle inférieur, un mono-, di- ou polyhalogéno-(hydroxy)alkyle inférieur, un mono-, di- ou polyhalogéno(hydroxy)alcényle inférieur, un amino alcoxy inférieur-alkyle inférieur, un alkyl inférieur thio alkyle inférieur, un alcane inférieur sulfinyl-alkyle inférieur, un alcane inférieur sulfonyl-alkyle inférieur, un di-alcoxy inférieur-alkyle inférieur, un di-alkyl-inférieur thio-alkyle inférieur, un alcoxy inférieur-(hydroxy)alkyle inférieur, un alcoxy inférieur(halogéno) alkyle inférieur, un phényl alkyle inférieur, un phényl-alkyl inférieur mono- ou disubstitué, dans la partie phényle, par un halogène, un alkyle inférieur, un alcoxy inférieur et ou un trifluorométhyle, un naphtyl-alkyle inférieur, un oxa- ou thiacycloalkyle ayant de 2 à 6 atomes de carbone dans le cycle ou un dioxa-, oxathia- ou dithiacycloalkyle ayant de 3 à 5 atomes de carbone dans le cycle ou, si R₁ et R_{1'} dénotent un hydrogène, R₂ représente un phényle, un phényl mono- ou disubstitué par un halogène, un alkyle inférieur, un alcoxy inférieur et/ou un trifluorométhyle ou un pyridyle et R_{2'} est un hydroxy, R représente un méthyle ou un de ses sels.

4. Composé selon la revendication 2, de formule I dans laquelle R₁ est un halogène, R_{1'} est un halogène ou un hydrogène et R₂ et R_{2'} représentent un hydrogène ou R₁ et R_{1'} représentent un hydrogène, R₂ dénote un phényle ou un phényl mono- ou disubstitué par un halogène, alkyle inférieur, alcoxy inférieur et/ou trifluorométhyle et R_{2'} est un hydroxy ou R₂ et R_{2'} représentent ensemble un oxo, et où R dénote un alkyle inférieur ayant 2 atomes de carbone ou plus, un alcényle inférieur, alcynyle inférieur, un groupe cycloalkyle, hydroxycycloalkyle, cycloalkyl-alkyle inférieur, cycloalkyl-(hydroxy)alkyle inférieur, ou un alkyl inférieur thiocycloalkyl-(hydroxy)-alkyle inférieur ayant de 3 à 6 atomes de carbone dans le cycle, un oxo-alkyle inférieur, amino-alkyle inférieur, alcanoyl inférieur amino-alkyle inférieur, phtalimido-alkyle inférieur, mono- ou dihydroxy alkyle inférieur, hydroxy-alcényle inférieur, amino(hydroxy)alkyle inférieur, alcanoyl inférieur amino-(hydroxy)alkyle inférieur, phtalimido(hydroxy) alkyle inférieur, mono-, di- ou polyhalogéno-alkyle inférieur, mono-, di- ou polyhalogéno-alcényle inférieur, mono-, di- ou polyhalogéno(hydroxy)alkyle inférieur, mono-, di- ou polyhalogéno-(hydroxy)alcényle inférieur, amino alcoxy inférieur alkyle inférieur, alkyl inférieur thio-alkyle inférieur, alcane inférieur sulfinyl-alkyle inférieur, alcane inférieur sulfonyl-alkyle inférieur, di-alcoxy inférieur-alkyle inférieur, di-alkyl inférieur-thio-alkyle inférieur, alcoxy inférieur-(hydroxy)alkyle inférieur, alcoxy inférieur-(halogéno)alkyle inférieur, phényl-alkyle inférieur, phényl-alkyl inférieur mono-ou disubstitué, dans la partie phényle, par un halogène, alkyle inférieur, alcoxy inférieur et/ou trifluoro-méthyle, un naphtyl-alkyle inférieur, oxa- ou thiacycloalkyle ayant de à 6 atomes de carbone dans le cycle, ou un dioxa-, oxathia-ou dithiacycloalkyle ayant de 3 à 5 atomes de carbone dans le cycle et leurs sels.

5. Composé selon la revendication 2, de formule I dans laquelle ou bien R₁ et R_{1'} représentent un fluoro et R₂ et R_{2'} représentent un hydrogène ou bien R₁ et R_{1'} représentent un hydrogène, R₂ est un phényle, un phényle substitué par un halogène comme fluoro, alkyle en C₁ à C₄ alcoxy en C₁ à C₄ et/ou trifluorométhyle et R_{2'} est un hydroxy, ou R₁ et R_{1'} représentent un hydrogène et R₂ et R_{2'} représentent ensemble un oxo, et où R est un alkyle en C₂ à C₁₂, un alcényle en C₂ à C₇, un alcynyle en C₂ à C₇, un mono- ou dihydroxy-alkyle en C₂ à C₇, un oxo-alkyle en C₃ à C₇, un amino-alkyle en C₃ à C₆, un phtalimido-alkyle en C₃ à C₆, ou un phtalimido-(hydroxy) alkyle en C₃ à C₇, ou un de ses sels.

6. Composé selon la revendication 2, de formule I dans laquelle R₁ et R_{1'} représentent un fluoro et R₂ et R_{2'} représentent un hydrogène ou R₁ et R_{1'} représentent un hydrogène et R₂ et R_{2'} représentent ensemble un oxo, et où R représente un alkyle en C₂ à C₇, un alcényle en C₂ à C₇ saturé en α, un alcynyle en C₃ à C₇ saturé en α, un α, β γ ou δ-hydroxy-alkyle en C₂ à C₇, un α,α-difluoro-alkyle en C₂ à C₄, un mono-, di- ou trihalogéno-hydroxy - α-alcényle en C₃ à C₇, un mono-, di- ou trifluoro- α-hydroxy-alkyle en C₃ à C₇, un mono-, di- ou trihalogéno-αhydroxy-alcényle en C₃ à C₇, et un alcoxy en C₁ à C₄-alkyle en C₁ à C₄, un di-alcoxy en C₁ à C₄-alkyle en C₁ à C₄, un cycloalkyle en C₃ à C₆-alkyle en C₁ à C₄, un α-hydroxy en C₃ à C₆ ou un cycloalkyle en C₃ à C₆-α-hydroxy-alkyle en C₁ à C₄, ou un de ses sels.

7. Composé selon la revendication 1, de formule I, dans laquelle R₁ et R_{1'} représentent un hydrogène, R₂ est un phényle, un phényle substitué par un halogène, un alkyle en C₁ à C₄, un alcoxy en C₁ à C₄, et/ou un trifluorométhyle et R_{2'} est un hydroxy, et où R représente un alkyle en C₁ à C₇, ou un de ses sels.

8. Composé selon l'une quelconque des revendications 1 à 7, où R représente un alkyle en C₃ à C₄, ou, si cela est applicable, un méthyle ou un de ses sels.

9. Composé selon la revendication 2, où R est un alkyle en C₂ à C₇, et où R₁ et R_{1'} représentent un fluoro et R₂ et R_{2'} représentent un hydrogène, ou bien R₁ et R_{1'} représentent un hyrogène et R₂ et R_{2'} représentent ensemble un oxo du un de ses sels.

10. Acide 3-amino-2-oxo-propyl(n-butyl)phosphinique ou un de ses sels.

11. Acide 3-amino-2-(4-chlorophényl)-2-hydroxy-propyl-(n-butyl)-phosphinique ou un de ses sels.

12. Acide 3-amino-1,1-difluoro-propyl(n-butyl)-phosphinique ou un de ses sels.

13. Acide 3-amino-2-oxo-propyl(cyclopropylméthyl)-phosphinique ou un de ses sels.

14. Acide 3-amino-2-oxo-propyl(cyclohexylméthyl)-phosphinique ou un de ses sels.

15. Acide 3-amino-2-hydroxy-2-méthyl-propyl(n-butyl)-phosphinique ou un de ses sels.

16. Acide 3-amino-1-fluoro-propyl(n-butyl)phosphinique ou un de ses sels.

17. Acide 3-amino-2-(4-chlorophényl)-2-hydroxy-propyl(cyclohexylméthyl)phosphinique ou un de ses sels.

18. Acide 3-amino-2-(4-chlorophényl)-2-hydroxy-propyl (benzyl)phosphinique ou un de ses sels.

19. Acide 3-amino-2-(4-chlorophényl)-2-hydroxy-propyl(cyclo propylméthyl)phosphinique ou un de ses sels.

20. Acide 3-amino-2-(4-chlorophényl)-2-hydroxy-propyl(cyclopropyl-méthyl)phosphinique ou un de ses sels.

21. Acide 3-amino-2-(4-chlorophényl)-2-oxo-propyl(diéthoxyméthyl)phosphinique ou un de ses sels.

22. Acide 3-amino-2-(4-chlorophényl)-2-hydroxy-propyldiéthoxyméthyl)-phosphinique ou un de ses sels.

23. Acide 3-amino-2-(4-chlorophényl)-2-hydroxy-propyl(méthyl)-phosphinique ou un de ses sels.

24. Composé selon l'une quelconque des revendications 2, 4 à 6 et 9 à 13 pour application dans un procédé de traitement thérapeutique du corps humain ou animal.

25. Composé selon l'une quelconque des revendications 1, 3, 7, 8 et 14 à 23 pour application dans un procédé pour le traitement thérapeutique du corps humain ou animal.

26. Composition pharmaceutique qui contient au moins un composé selon l'une quelconque des revendications 2, 4 à 6, 9 à 13 et 24 mélangée à des excipients pharmaceutiques classiques.

27. Composition pharmaceutique qui contient au moins un composé selon l'une quelconque des revendications 1, 3, 7, 8, 14 à 23 et 25 mélangé à des excipients pharmaceutiques classiques.

28. Procédé de préparation de composés de formule I 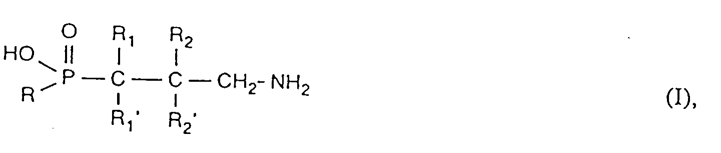 dans laquelle ou bien R₁ est un halogène, R_{1'} est un halogène ou un hydrogène et R₂ et R_{2'} dénotent un hydrogène, ou bien R₁ et R_{1'} représentent un hydrogène, R₂ est un radical aliphatique ou aromatique et R_{2'} est un hydroxy ou bien R₂ et R_{2'} représentent ensemble un oxo, et où R dénote un radical aliphatique, cycloaliphatique, cycloaliphatique-aliphatique ou araliphatique ayant 2 atomes de carbone ou plus, ou si R₁ et R_{1'} dénotent un hydrogène, R₂ représente un radical aromatique, et R_{2'} est un hydroxy, R représente un méthyle, et de leurs sels caractérisé en ce que
a) dans un composé de formule II 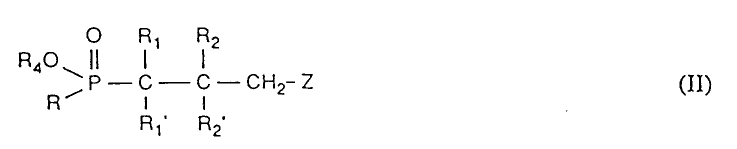 dans laquelle R, R₁, R_{1',} R₂ et R_{2'} ont les significations données ci-dessus, Z représente un groupe amino protégé ou datent Zₒ et R₄ dénotent un hydrogène ou un groupe protecteur d'hydroxy-R₅ et où un amino comme constituant de R et/ou un hydroxy R_{2'} ou oxo R₂ + R_{2'} peut être présent sous une forme temporairement protégée, tout groupe R₅ ou R₆ est remplacé par un hydrogène et/ou tout groupe Zₒ est transformé en -NH₂; ou
b) dans un composé de formule III 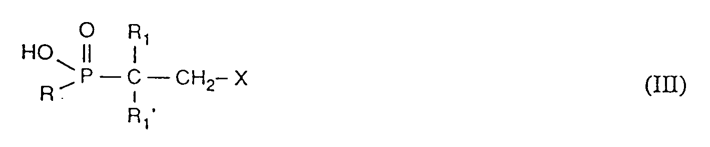 dans laquelle R₁ et R_{1'} ont les significations données ci-dessus et X est un groupe capable d'être transformé en un groupe de formule -CH₂NH₂ (Ia), on transforme le groupe X en un composé de formule Ia; ou
c) on réduit un composé de formule IV 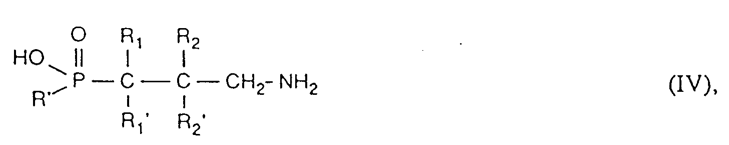 dans laquelle R' peut être choisi parmi un alcényle inférieur, alcanediényle inférieur ou alcynyle inférieur, pour produire un composé de formule I, dans laquelle R est un alkyle inférieur, ou à partir d'un phényl pour produire un composé de formule I, dans laquelle R est un cyclohexyle, et, si on le désire, on peut transformer un sel obtenu dans ce procédé en un composé libre ou en un autre sel et/ou, si on le désire, on transforme un compose libre résultant en un sel pour se conformer à la définition ci-dessus et/ou si on le désire, on sépare un mélange d'isomères résultants en les isomères individuels.

29. Application d'un composé selon l'une quelconque des revendications 1 à 25 à la préparation d'un médicament nootropique, anti-dépresseur ou anxiolytique.

## Revendications (Revendications pour l'(les) Etat(s) contractant(s) suivant(s): ES, GR)

1. Procédé de préparation de composés de formule I 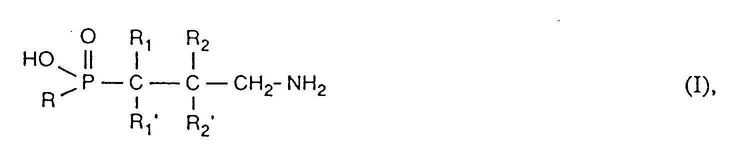 dans laquelle ou bien R₁ est un halogène, R_{1'} est un halogène ou un hydrogène et R₂ et R_{2'} dénotent un hydrogène, ou bien R₁ et R_{1'} représentent un hydrogène, R₂ est un radical aliphatique ou aromatique et R_{2'} est un hydroxy, ou bien R₂ et R_{2'} représentent ensemble un oxo, et où R dénote un radical aliphatique, cycloaliphatique, cycloaliphatique-aliphatique ou araliphatique ayant 2 atomes de carbone ou plus ou, si R₁ et R_{1'} dénotent un hydrogène, R₂ représente un radical aromatique et R_{2'} est un hydroxy, R représente un méthyle, et de leurs sels, caractérisé en ce que
a) dans un composé de formule II 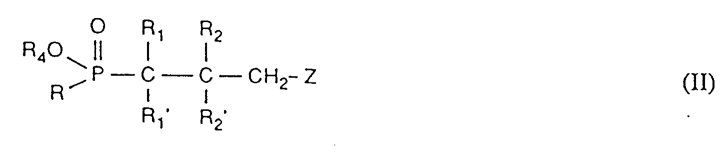 dans laquelle R, R₁, R_{1'}, R₂ et R_{2'} ont les significations données ci-dessus, Z représente un groupe amino protégé ou latent Zₒ et R₄ dénotent un hydrogène ou un groupe protecteur d'hydroxy-R₅ et où un amino comme constituant de R et/ou un hydroxy R_{2'} ou oxo R₂ + R_{2'} peut être présent sous une forme temporairement protégée, tout groupe R₅ ou R₆ est remplacé par un hydrogène et/ou tout groupe Zₒ est transformé en -NH₂; ou
b) dans un composé de formule III 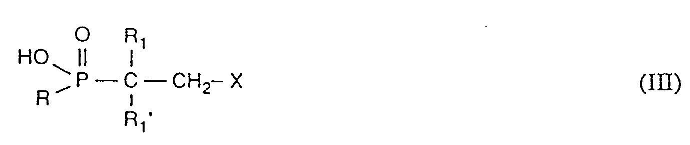 dans laquelle R₁ et R_{1'} ont les significations données ci-dessus et X est un groupe capable d'être transformé en un groupe de formule -CH₂NH₂ (Ia), on transforme le groupe X en un composé de formule Ia; ou
c) on réduit un composé de formule IV 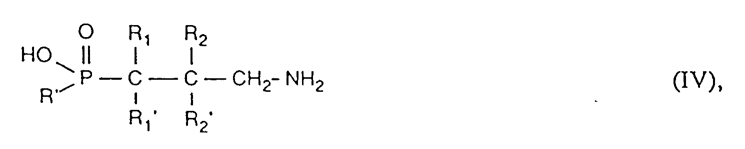 dans laquelle R' peut être choisi parmi un alcényle inférieur, alcanediényle inférieur ou alcynyle inférieur, pour produire un composé de formule I, dans laquelle R est un alkyle inférieur, ou à partir d'un phényl pour produire un composé de formule I, dans laquelle R est un cyclohexyle, et, si on le désire, on peut transformer un sel obtenu dans ce procédé en un composé libre ou en un autre sel et/ou, si on le désire, on transforme un compose libre résultant en un sel pour se conformer à la définition ci-dessus et/ou si on le désire, on sépare un mélange d'isomères résultants en les isomères individuels.

2. Procédé selon la revendication 1 de préparation de composés de formule I 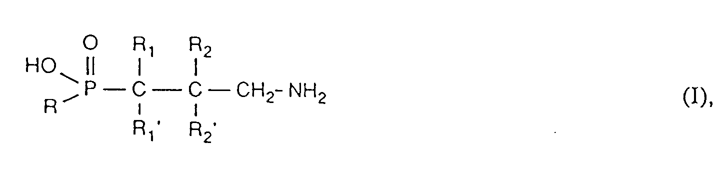 dans laquelle ou bien R₁ est un halogène, R_{1'} est un halogène ou un hydrogène et R₂ et R_{2'} dénotent un hydrogène, ou bien R₁ et R_{1'} représentent un hydrogène, R₂ est un radical aliphatique ou aromatique et R_{2'} est un hydroxy, ou bien R₂ et R_{2'} représentent ensemble un oxo, et où R dénote un radical aliphatique, cycloaliphatique, cycloaliphatique-aliphatique ou araliphatique ayant 2 atomes de carbone ou plus ou, si R₁ et R_{1'} dénotent un hydrogène, R₂ représente un radical aromatique et R_{2'} est un hydroxy, R représente un méthyle, ou un de leurs sels, caractérisé en ce que
a) dans un composé de formule II 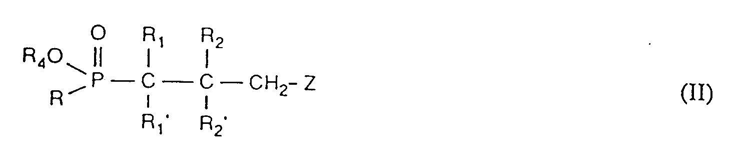 dans laquelle R, R₁, R_{1'}, R₂ et R_{2'} ont les significations données ci-dessus, Z représente un groupe amino protégé ou latent Zₒ et R₄ dénotent un hydrogène ou un groupe protecteur d'hydroxy-R₅ et où un amino comme constituant de R et/ou un hydroxy R_{2'} ou oxo R₂ + R_{2'} peut être présent sous une forme temporairement protégée, tout groupe R₅ ou R₆ est remplacé par un hydrogène et/ou tout groupe Zₒ est transformé en -NH₂; ou
b) dans un composé de formule III 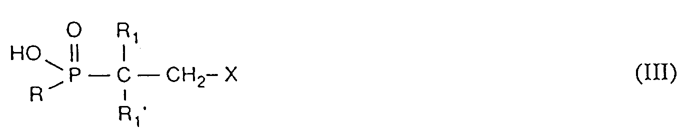 dans laquelle R₁ et R_{1'} ont les significations données ci-dessus et X est un groupe capable d'être transformé en un groupe de formule -CH₂NH₂ (Ia), on transforme le groupe X en un composé de formule Ia; ou
c) on réduit un composé de formule IV 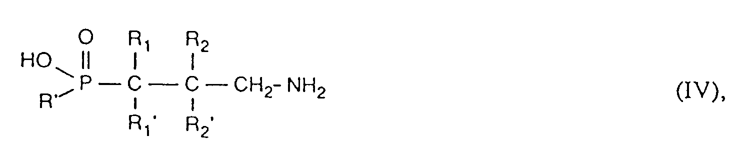 dans laquelle R' peut être choisi parmi un alcényle inférieur, alcanediényle inférieur ou alcynyle inférieur, pour produire un composé de formule I, dans laquelle R est un alkyle inférieur, ou à partir d'un phényl pour produire un composé de formule I, dans laquelle R est un cyclohexyle, et, si on le désire, on peut transformer un sel obtenu dans ce procédé en un composé libre ou en un autre sel et/ou, si on le désire, on transforme un composé libre résultant en un sel pour se conformer à la définition ci-dessus et/ou si on le désire, on sépare un mélange d'isomères résultants en les isomères individuels.

3. Procédé selon la revendication 1 de préparation de composés de formule L, dans laquelle R₁ est un halogène, R_{1'} est un halogène ou un hydrogène et R₂ et R_{2'} représentent un hydrogène ou bien R₁ et R_{1'} représentent un hydrogène, R₂ dénote un alkyle inférieur, un phényle, un phényl mono- ou disubstitué par un halogène, un alkyle inférieur, un alcoxy inférieur et/ou un trifluorométhyle ou un pyridyle et R_{2'} est un hydroxy ou bien R₂ et R_{2'} représentent ensemble un oxo, et où R dénote un alkyle inférieur ayant 2 atomes de carbone ou plus, un alcényle inférieur, un alcynyle inférieur, un cycloalkyle, un hydroxycycloalkyle, un cycloalkyl-alkyle inférieur, un cycloalkyle(hydroxy) alkyle inférieur, ou un alkyl inférieur thio-cycloalkyle- (hydroxy)alkyle inférieur ayant de 3 à 6 atomes de carbone dans le cyle, un oxo-alkyle inférieur, un amino alkyle inférieur, un alcanoyl inférieur amino-alkyle inférieur, un phtalimido-alkyle inférieur, un mono- ou di-hydroxy alkyle inférieur, un hydroxy-alcényle inférieur, un amino-(hydroxy)alkyle inférieur, un alcanoyl inférieur amino-(hydroxy)alkyle inférieur, un phtalimido(hydroxy)alkyle inférieur, un mono-, di- ou polyhalogénoalkyle inférieur, un mono-, di- ou polyhalogéno-alcényle inférieur, un mono-, di- ou polyhalogéno-(hydroxy)alkyle inférieur, un mono-, di- ou polyhalogéno(hydroxy)alcényle inférieur, un amino alcoxy inférieur-alkyle inférieur, un alkyl inférieur thio alkyle inférieur, un alcane inférieur sulfinyl-alkyle inférieur, un alcane inférieur sulfonyl-alkyle inférieur, un di-alcoxy inférieur-alkyle inférieur, un di-alkyl-inférieur thio-alkyle inférieur, un alcoxy inférieur-(hydroxy)alkyle inférieur, un alcoxy inférieur(halogéno) alkyle inférieur, un phényl alkyle inférieur, un phényl-alkyl inférieur mono- ou disubstitué, dans la partie phényle, par un halogène, un alkyle inférieur, un alcoxy inférieur et ou un trifluorométhyle, un naphtyl-alkyle inférieur, un oxa- ou thiacycloalkyle ayant de 2 à 6 atomes de carbone dans le cycle ou un dioxa-, oxathia- ou dithiacycloalkyle ayant de 3 à 5 atomes de carbone dans le cycle ou, si R₁ et R_{1'} dénotent un hydrogène, R₂ représente un phényle, un phényl mono- ou disubstitué par un halogène, un alkyle inférieur, un alcoxy inférieur et/ou un trifluorométhyle ou un pyridyle et R_{2'} est un hydroxy, R représente un méthyle ou un de ses sels.

4. Procédé selon la revendication 2, de préparation de composés de formule I dans laquelle R₁ est un halogène, R_{1'} est un halogène ou un hydrogène et R₂ et R_{2'} représentent un hydrogène ou R₁ et R_{1'} représentent un hydrogène, R₂ dénote un phényle ou un phényl mono- ou disubstitué par un halogène, alkyle inférieur, alcoxy inférieur et/ou trifluorométhyle et R_{2'} est un hydroxy ou R₂ et R_{2'} représentent ensemble un oxo, et où R dénote un alkyle inférieur ayant 2 atomes de carbone ou plus, un alcényle inférieur, alcynyle inférieur, un groupe cycloalkyle, hydroxycycloalkyle, cycloalkyl-alkyle inférieur, cycloalkyl-(hydroxy)alkyle inférieur, ou un alkyl inférieur thiocycloalkyl-(hydroxy)-alkyle inférieur ayant de 3 à 6 atomes de carbone dans le cycle, un oxo-alkyle inférieur, amino-alkyle inférieur, alcanoyl inférieur amino-alkyle inférieur, phtalimido-alkyle inférieur, mono- ou dihydroxy alkyle inférieur, hydroxy-alcényle inférieur, amino-(hydroxy)alkyle inférieur, alcanoyl inférieur amino-(hydroxy)alkyle inférieur, phtalimido(hydroxy) alkyle inférieur, mono-, di-ou polyhalogéno-alkyle inférieur, mono-, di- ou polyhalogénoalcényle inférieur, mono-, di- ou polyhalogéno-(hydroxy)alkyle inférieur, mono-, di- ou polyhalogéno-(hydroxy)alcényle inférieur, amino alcoxy inférieur alkyle inférieur, alkyl inférieur thio-alkyle inférieur, alcane inférieur sulfinyl-alkyle inférieur, alcane inférieur sulfonyl-alkyle inférieur, di-alcoxy inférieur-alkyle inférieur, di-alkyl inférieur-thio-alkyle inférieur, alcoxy inférieur-(hydroxy)alkyle inférieur, alcoxy inférieur-(halogéno)alkyle inférieur, phényl-alkyle inférieur, phényl-alkyl inférieur mono-ou disubstitué, dans la partie phényle, par un halogène, alkyle inférieur, alcoxy inférieur et/ou trifluoro-méthyle, un naphtyl-alkyle inférieur, oxa- ou thiacycloalkyle ayant de 2 à 6 atomes de carbone dans le cycle, ou un dioxa-, oxathia-ou dithiacycloalkyle ayant de 3 à 5 atomes de carbone dans le cycle et leurs sels.

5. Procédé selon la revendication 2, de préparation de composés de formule I dans laquelle ou bien R₁ et R_{1'} représentent un fluoro et R₂ et R_{2'} représentent un hydrogène ou bien R₁ et R_{1'} représentent un hydrogène, R₂ est un phényle, un phényle substitué par un halogène comme fluoro, alkyle en C₁ à C₄ alcoxy en C₁ à C₄ et/ou trifluorométhyle et R_{2'} est un hydroxy, ou R₁ et R_{1'} représentent un hydrogène et R₂ et R_{2'} représentent ensemble un oxo, et où R est un alkyle en C₂ à C₁₂, un alcényle en C₂ à C₇, un alcynyle en C₂ à C₇, un mono- ou dihydroxy-alkyle en C₂ à C₇, un oxo-alkyle en C₃ à C₇, un amino-alkyle en C₃ à C₆, un phtalimido-alkyle en C₃ à C₆, ou un phtalimido-(hydroxy) alkyle en C₃ à C₇, ou un de ses sels.

6. Procédé selon la revendication 1 de préparation de composés de formule I dans laquelle R₁ et R_{1'} représentent un fluoro et R₂ et R_{2'} représentent un hydrogène ou R₁ et R_{1'} représentent un hydrogène et R₂ et R_{2'} représentent ensemble un oxo, et où R représente un alkyle en C₂ à C₇, un alcényle en C₂ à C₇ saturé en α, un alcynyle en C₃ à C₇ saturé en α, un α, β γ ou δ-hydroxy-alkyle en C₂ à C₇, un α,α-difluoro-alkyle en C₂ à C₄ un mono-, di- ou trihalogénohydroxy - α-alcényle en C₃ à C₇, un mono-, di- ou trifluoro-α-hydroxy-alkyle en C₃ à C₇, un mono-, di- ou trihalogéno-α-hydroxy-alcényle en C₃ à C₇ et un alcoxy en C₁ à C₄-alkyle en C₁ à C₄, un di-alcoxy en C₁ à C₄-alkyle en C₁ à C₄, un cycloalkyle en C₃ à C₆-alkyle en C₁ à C₄ un α-hydroxy en C₃ à C₆ ou un cycloalkyle en C₃ à C₆- α-hydroxy-alkyle en C₁ à C₄, ou un de ses sels.

7. Procédé selon la revendication 1 de préparation de composés de formule I, dans laquelle R₁ et R_{1'} représentent un hydrogène, R₂ est un phényle, un phényle substitué par un halogène, un alkyle en C₁ à C₄, un alcoxy en C₁ à C₄, et/ou un trifluorométhyle et R_{2'} est un hydroxy, et où R représente un alkyle en C₁ à C₇, ou un de leurs sels.

8. Procédé selon l'une quelconque des revendications 1 à 7 de préparation de composés de formule I dans laquelle R représente un alkyle en C₃ à C₄, ou, si cela est applicable, un méthyle ou un de ses sels.

9. Procédé selon la revendication 2 de préparation de composés de formule I, où R est un alkyle en C₂ à C₇, et où R₁ et R_{1'} représentent un fluoro et R₂ et R_{2'} représentent un hydrogène, ou bien R₁ et R_{1'} représentent un hyrogène et R₂ et R_{2'} représentent ensemble un oxo ou un de leurs sels.

10. Procédé selon la revendication 2 de préparation de l'acide 3-amino-2-oxo-propyl(n-butyl)phosphinique ou d'un de ses sels.

11. Procédé selon la revendication 2 de préparation de l'acide 3-amino-2-(4-chlorophényl)-2-hydroxy-propyl(n-butyl)phosphinique ou d'un de ses sels.

12. Procédé selon la revendication 2 de préparation de l'acide 3-amino-1,1-difluoro-propyl(n-butyl)phosphinique ou d'un de ses sels.

13. Procédé selon la revendication 2 de préparation de l'acide 3-amino-2-oxo-propyl(cyclopropylméthyl) phosphinique ou d'un de ses sels.

14. Procédé selon la revendication 2 de préparation de l'acide 3-amino-2-oxo-propyl(cyclohexylméthyl) phosphinique ou d'un de ses sels.

15. Procédé selon la revendication 2 de préparation de l' acide 3 -amino-2-hydroxy-2-méthyl-propyl(n-butyl) phosphinique ou d'un de ses sels.

16. Procédé selon la revendication 2 de préparation de l'acide 3-amino-1-fluoro-propyl(n-butyl)phosphinique ou d'un de ses sels.

17. Procédé selon la revendication 2 de préparation de l'acide 3-amino-2-(4-chlorophényl)-2-hydroxy-propyl-(cyclohexylméthyl)phosphinique ou d'un de ses sels.

18. Procédé selon la revendication 2 de préparation de l'acide 3-amino-2-(4-chlorophényl)-2-hydroxy-propyl (benzyl)phosphinique ou d'un de ses sels.

19. Procédé selon la revendication 2 de préparation de l'acide 3-amino-2-(4-chlorophényl)-2-hydroxy-propyl(cyclopropylméthyl)phosphinique ou d'un de ses sels.

20. Procédé selon la revendication 2 de préparation de l'acide 3-amino-2-(4-chlorophényl)-2-hydroxy-propyl-(cyclopropylméthyl)phosphinique ou d'un de ses sels

21. Procédé selon la revendication 2 de préparation de l'acide 3-amino-2-(4-chlorophényl)-2-oxo-propyl(diéthoxyméthyl)phosphinique ou d'un de ses sels.

22. Procédé selon la revendication 2 de préparation de l'acide 3-amino-2-(4-chlorophényl)-2-hydroxy-propyl- diéthoxyméthyl)phosphinique ou d'un de ses sels.

23. Procédé selon la revendication 2 de préparation de l'acide 3-amino-2-(4-chlorophényl)-2-hydroxy-propyl (méthyl)phosphinique ou d'un de ses sels.
